(19) Europäisches Patentamt
European Patent Office
Office européen des brevets

(11) **EP 2 136 443 A1**

(12) **EUROPEAN PATENT APPLICATION**
published in accordance with Art. 153(4) EPC

(43) Date of publication:
**23.12.2009 Bulletin 2009/52**

(51) Int Cl.:
*H01S 3/106* (2006.01)          *A61B 10/00* (2006.01)
*G01N 21/17* (2006.01)          *G02F 2/02* (2006.01)
*H01S 3/06* (2006.01)

(21) Application number: **07806624.8**

(22) Date of filing: **03.09.2007**

(86) International application number:
**PCT/JP2007/067156**

(87) International publication number:
**WO 2008/093448 (07.08.2008 Gazette 2008/32)**

(84) Designated Contracting States:
**AT BE BG CH CY CZ DE DK EE ES FI FR GB GR HU IE IS IT LI LT LU LV MC MT NL PL PT RO SE SI SK TR**

(30) Priority: **29.01.2007   JP 2007018168**
**29.06.2007   JP 2007173347**
**29.06.2007   JP 2007173348**

(71) Applicant: **Optical Comb, Inc.**
**Kanagawa 226-8510 (JP)**

(72) Inventors:
• **KOUROGI, Motonobu**
**Yokohama-shi**
**Kanagawa 241-0801 (JP)**
• **IMAI, Kazuhiro**
**Yokohama-shi**
**Kanagawa 226-0028 (JP)**

(74) Representative: **Müller - Hoffmann & Partner**
**Patentanwälte**
**Innere Wiener Strasse 17**
**81667 München (DE)**

(54) **WAVELENGTH SCANNING LIGHT SOURCE AND OPTICAL COHERENCE TOMOGRAPHY DEVICE**

(57)    An optical coherence tomography device includes a wavelength scanning laser light source (10) provided with two Fabry-Perot resonators (13A, 13B) provided in a light path for laser oscillation. The values of FSR (free spectral range) of the Fabry-Perot resonators are set so as to be proximate to each other. The resonator length of at least one of the two Fabry-Perot resonators is periodically varied within a preset range to cause the two Fabry-Perot resonators (13A, 13B) to operate as a wavelength length varying filter of a narrow pass band capable of varying the selection wavelength by the vernier effect to output laser light that has wavelength temporally scanned. The optical coherence tomography device also includes an interference optical system (20) that causes the laser light output from the wavelength scanning laser light source (10) to be branched into light for reference and light for observation to be illuminated on an object for observation (60) and that generates interference light of reflected light of the light for observation illuminated on the object for observation (60) and the light for reference. The optical coherence tomography device further includes a signal processing means (50) that receives the interference light obtained from the interference optical system (20) for transforming the received interference light into an electrical signal to calculate the optical tomographic image information of the object for observation (60).

FIG.3

**Description**

Technical Field

This invention relates to a wavelength scanning light source for periodically scanning the emission wavelength, and to an optical tomography device having a wavelength scanning light source.
The present application claims priority rights based on Japanese Patent Application No. 2007-18168 filed in Japan on January 29, 2007, and on Japanese Patent Application Nos. 2007-173347 and 2007-173448, both filed on June 29, 2007. These patent applications of the senior filing dates are incorporated by reference herein.

Background Art

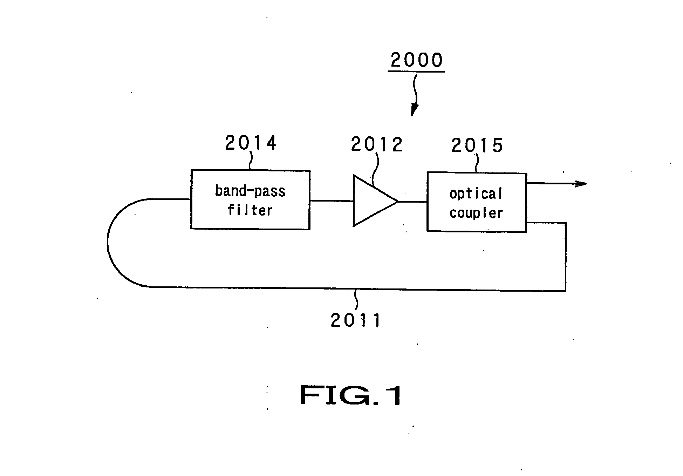

Up to now, a broadband light source has been used as a light source for an analysis system that illuminates light on an object for measurement in order to perform an analysis. In spectroscopic analysis, such a technique consisting in projecting light of a broad bandwidth to an object for measurement, and spatially decomposing the reflected or transmitted light into respective wavelength components by e.g. a diffraction grating, or Fourier transforming the light by an interferometer for analysis, is widely used. The light source may be exemplified by, for example, a white light source or an ASE light source that uses an erbium-doped fiber (EDF). With the spectroscopic analysis, the light level that may be used in spectroscopy is small because of low light output intensity with respect to the wavelength. As a result, a detected optical signal obtained on Fourier transform analysis is buried in the noise to render the analysis difficult. As a light source for the analysis device, there is also such a method which uses a light source of the variable wavelength type in which the wavelength of a sole spectral light beam of high intensity is varied at a desired frequency band. With this technique, the strongly monochromatic light is varied in wavelength and illuminated on an object for measurement, and light transmitted through or reflected by the object for measurement is directly received by a light receiving device. With this technique, the light intensity density with respect to the wavelength of the light source is high so that the level of detected light and the signal-to-noise ratio are sufficiently high with the result that sufficiently high detection accuracy may be realized. Among variable wavelength light sources of the related art, there are an external resonator laser, a fiber ring laser and a laser device a wavelength variation unit provided therein. The external resonator laser uses a gain medium such as a semiconductor laser, and an external resonator is formed between one end face of the semiconductor laser and an external mirror. A light source of the wavelength variation type may be obtained by providing a filter for wavelength variation by e.g. a diffraction grating within the external resonator to vary the oscillation wavelength.
With the external resonator laser light source, the length of the external resonator is small as e.g. 50 mm, with the longitudinal mode spacing being e.g. 3 GHz. Thus, if simply the wavelength of the variable wavelength filter is varied, the operation is destabilized between longitudinal modes. For example, non-continuous mode hop or multi-mode oscillations may arise between the longitudinal modes. Thus, to continuously vary the wavelength with the single mode and to provide a stabilized output, the length of the external resonator needs to be delicately controlled using e.g. a piezo device. Moreover, mechanical operations are also in play so that the wavelength and the length of the external resonator need to be controlled in synchronism with each other, with the result that it is difficult to vary the wavelength at a high speed. As a wavelength scanning fiber laser light source capable of continuously scanning the wavelength of a light source having a narrow band spectrum over a wide bandwidth at a high speed, there has been proposed a wavelength scanning fiber laser light source provided with a gain medium having a gain bandwidth at an oscillation bandwidth and endowed with an optical circulator on an optical fiber loop. The light taken out by the optical circulator is enlarged by a collimator lens, and a polygonal mirror provided on the optical axis is driven in rotation. A diffraction grating is provided at a position of receiving light reflected by the polygonal mirror to reflect the light back in the same direction as the incident light by a retrogressive configuration. With this wavelength scanning fiber laser light source, the selection wavelength is varied in dependence upon the angle of incidence on the diffraction grating, and the degree of selectivity is increased by light incidence that occurs twice, so that, if the polygonal mirror is rotated at a high speed to vary the selection wavelength, it is again possible to vary the oscillation wavelength with the narrow bandwidth (see Japanese Patent Application Laid-Open No. 2006-237359, for instance).
There has also been proposed a variable wavelength light source by a ring laser employing an erbium doped fiber. Referring for example to Fig.1, this variable wavelength light source 2000 uses a fiber amplifier 2012 having an erbium doped fiber (EDF) as a gain medium. A variable wavelength band-pass filter 2014 is provided on its optical fiber loop 2011. The wavelength of this band-pass filter 2014 is varied to vary the wavelength of laser light taken outside via an optical coupler 2015 connected to this optical fiber loop 2011. Since the resonator length of the optical fiber loop 2011 may be made long and equal to, for example, 30m, the longitudinal mode spacing may be reduced, thus eliminating the effect of mode hop without changing the resonator length. Thus, even though the oscillation is not strictly the single mode oscillation, the wavelength may be varied pseudo-continuously by simply varying the selection wavelength of the band-pass filter 2014. See YAMASHITA ET AL., IEEE JOURNAL ON SELECTED TOPICS IN QUANTUM ELECTRONICS, VOL.7, NO.1 JANUARY/FEBRUARY 2001, pp.41 to 43, for instance).

In connection with a type in which a variable wavelength mechanism is provided within a laser device, there is proposed DBR-LD (Distributed Bragg reflector laser diode) in which an active region responsible for gain and a DBR region responsible for reflection by a diffraction grating are provided in the same laser device. The wavelength variation range of this DBR-LD is on the order of 10 nm at the maximum. A DBR-LD employing a non-uniform diffraction grating, in which an active region responsible for the gain and DBR regions disposed ahead and in rear of the active region are provided in the same laser device, has also been proposed. In the forward and rear DBR regions, a large number of reflection peaks are generated by the non-uniform diffraction grating with the separations between the reflection peaks on the forward side being slightly offset from those on the rear side. Since the so-called vernier effect may be obtained with this configuration, wavelength variations over an extremely wide range may be realized. With the DBR-LD employing this non-uniform diffraction grating, the operation of wavelength variation exceeding 100 nm and pseudo-continuous wavelength variation of 40 nm has been achieved. See Japanese Patent Application Laid-Open No.2006-278770, for example.

With recent progress in therapeutic techniques, such as procedures with an endoscope, a method for non-intrusive real-time diagnosis of pathologic tissues has become a desideratum. An electronic endoscope employing a CCD or imaging with CT, MRI or ultrasonic waves has been used as the diagnostic method. An electronic endoscope is usable only for observing the body surface of a living body, whereas, with the latter method, technical constraint is imposed if observation with micron-order resolution is desired.

As one of the techniques of non-destructive tomographic measurements used in, for example, medical treatment, an optical coherence tomography (OCT) employing temporally low-coherence light as a probe has become known. The OCT, employing light as a probe for measurement, has a merit that measurements may be made of, for example, the refractive index distribution, spectral information or the polarized light information, e.g. double refractive index distribution, of an object for measurement. See Japanese Patent Application Laid-Open No.2007-101365, for example.

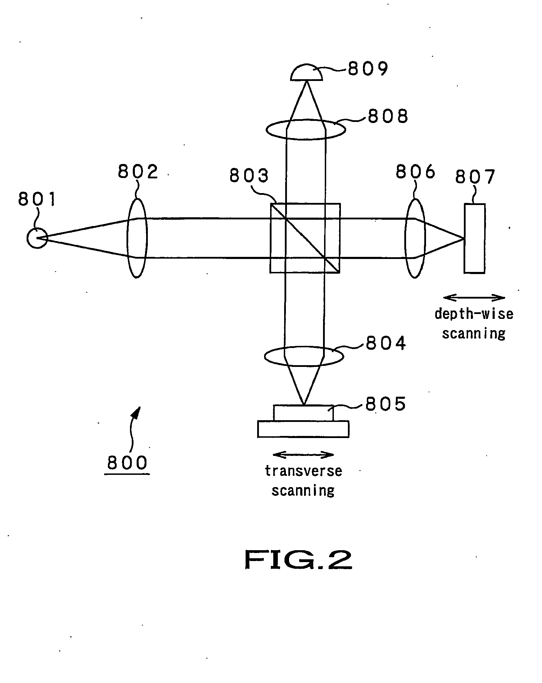

An OCT 800 is based on a Michelson interferometer. Its principle is shown in Fig.2.

The light radiated from a light source 801 is collimated by a collimator lens 802, and split by a beam splitter 803 into light for reference and object light. The object light is condensed in an object for measurement 805 by an objective lens 804 in an object arm and is returned to the objective lens 804 and to the beam splitter 803 after scattering and reflection. On the other hand, the light for reference is reflected by a mirror for reference 807 after passing through an objective lens 806 in a reference arm, and is again returned via the objective lens 806 to the beam splitter 803. The object light and the light for reference thus returned to the beam splitter 803, again fall on a condenser lens 808 so as to be condensed on a photodetector 809 (photodiode).

As a light source 801 of the OCT 800, a light source of light of temporally low coherence is used. By the temporally low coherence light is meant such light in which light rays exiting a light source at different time points hardly interfere with one another. Since the Michelson interferometer uses a light source for light of low temporal coherence, an interference signal presents itself only in case of the length of the reference arm being approximately equal to that of the object arm. Hence, if measurement is made of the intensity of the interference signal by the photodetector 809 as the optical path difference ($\tau$) between the object arm and the reference arm is varied, there is obtained an interference signal (interferogram) with respect to the optical path difference.

The profile of the interferogram indicates the refractive index distribution along the depth-wise direction of an object for measurement 805. The structure along the depth-wise direction of the object for measurement 805 may be obtained by one-dimensional axial scanning. Thus, with the OCT 800, the structure along the depth-wise direction of the object for measurement 805 may be measured by optical path scanning.

In addition to the scanning along the axial direction, transverse mechanical scanning may also be used by way of performing two-dimensional scanning. This yields a two-dimensional tomographic image of the object for measurement. For transverse scanning, the object for measurement is directly moved, or alternatively, the object is fixed and the objective lens is shifted. Still alternatively, a galvanometer mirror, placed in the vicinity of a pupil plane of an objective lens is angularly rotated, with the object for measurement and the objective lens both being fixed.

As further technical developments of the fundamental OCT, there are a wavelength scanning OCT that sweeps the wavelength of the light source to generate a spectral interference signal, and a spectral domain OCT that generates a spectral signal using a spectroscope. An example of the latter is Fourier domain OCT.

The wavelength scanning OCT is such a system in which, as stated in Handbook of Optical Coherence Tomography, p.41 to p.43, Mercel Dekker Inc. 2002, light is illuminated into a living body, and the wavelength of the illuminated light is continuously varied. The light for reference and reflected light returned from different depths of the living body are made to interfere with each other by an interferometer, and frequency components of the interference signal are analyzed to produce a tomographic image. This technique that allows constructing a tomographic image of high resolution from frequency analysis of a signal from within the object is felt to be promising as an advanced system for diagnosis. The wavelength scanning OCT, high in measurement sensitivity and sturdy against dynamic noise, lends itself to practical utilization on e.g. an endoscope. As broader the bandwidth of the wavelength scanning of the light illuminated, higher the band of the frequency analysis, and hence the resolution in the depth-wise direction becomes high.

The Fourier domain OCT acquires the wavelength spectrum of light reflected from the object for measurement by a spectrometer (spectroscope). The resulting spectral intensity distribution is Fourier-transformed to take out a signal in a real space (OCT signal space). With the Fourier domain OCT, there is no necessity of depth-wise scanning, and scanning along the x-axis enables measurement of a tomographic structure of an object for measurement.

Polarized light sensitive OCT acquires the wavelength spectrum of reflected light from an object for measurement by a spectroscope as in Fourier domain OCT. The incident light and the light for reference are passed through a half wave plate or a quarter wave plate so as to be turned into horizontal linear polarized light, vertical linear polarized light, 45° linear polarized light and circular polarized light. The light for reference and the reflected light from the object for measurement are superposed one on another and passed through the half wave plate or the quarter wave plate. Only the horizontal polarized light component is made to be incident on the spectroscope for interference, and only a component of the object light having a specified light polarized state is taken out and Fourier transformed. With this polarized light sensitive OCT, it is again unnecessary to perform depth-wise scanning.

Disclosure of the Invention

Problem to be solved by the Invention

If the variable wavelength light source is used as a light source for an analysis device, it is necessary to carry out wavelength variation at a high speed and to narrow the width of the oscillation spectrum, and a corresponding characteristic is required of a band-pass filter. For example, in optical coherence tomography device (OCT), if a high-speed wavelength scanning is available, high-speed image processing and blood stream observation as well as dynamic analysis of changes in oxygen saturation become possible. Hence, there arises the demand for a corresponding device. As a product that uses the technique disclosed in the above Japanese Patent Application Laid-Open No.2006-237359, a wavelength scanning laser light source HSL-2000 capable of repeatedly scanning the wavelength at the maximum scan rate of 20 kHz, is presented e.g. by Santec Corporation. However, at present, only 20 kHz is available as the period of wavelength scanning.

Thus, a stereo image may be obtained by optical coherence tomography (OCT) only after several seconds.

On the other hand, the Fourier transform of a time signal in a wavelength scanning OCT yields the distribution of scattering of the object for measurement in the depth-wise direction, that is, an optical tomographic image. To obtain an accurate optical tomographic image, the original signal needs to be of an equal spacing with respect to the wavenumber of light ($2\pi$ / wavelength).

In general, a wavelength scanning light source or a spectroscope is designed to be linear with respect to the wavelength. On the other hand, the linearity is not optimum and is difficult to calibrate. If calibration or conversion to the wavenumber is not performed, the resulting optical tomographic image is severely deteriorated in resolution, while its linearity in the depth-wise direction is lost. It is therefore indispensable to calibrate the output of the scanning characteristic of the light source to the wavenumber.

To calibrate the wavelength scanning light source, the time characteristic of the wavelength scanning light source is measured with an oscilloscope or an interference filter or using the technique disclosed in Patent publication 1. Based on the time characteristic thus measured, the interference signal obtained is re-arrayed so as to be of an equal spacing with respect to the wavenumber of light ($2\pi$ / wavelength) before proceeding to signal processing. An image may be output after a time corresponding to the time for signal processing.

Moreover, wavelength scanning by the wavelength scanning light source is that by a mechanical wavelength selection device. Hence, there is a limit to the operating speed, which is 20 kHz at most. Thus, several seconds are needed to obtain a 3D OCT signal.

Moreover, if the variable wavelength light source is used as a light source for an analysis device, it is necessary to vary the wavelength at a high speed and to narrow down the width of the oscillation spectrum, and hence a corresponding characteristic is required of the band-pass filter. In optical coherence tomography (OCT), if high-speed wavelength scanning is made available, high-speed image processing, observation of the blood stream and dynamic analysis of changes in the oxygen saturation become a reality. Hence, there is a demand for such devices that may satisfy these needs.

In the DBR-LD, carriers are implanted into a DBR region to vary the refractive index in this region to implement the operation of wavelength variation. See Japanese Patent Application Laid-Open No.2006-278770. Thus, if crystal defects are increased due to current delivery, the rate of changes in the refractive index with respect to current delivery is appreciably varied to render it difficult to maintain laser oscillation at stable wavelength for prolonged time. In the technique of the current compound semiconductor process, it is difficult to increase the size by more than two inches. Thus, with a laser device increased in complexity and size, it is difficult to decrease the cost to less than the current cost. Notwithstanding, the longitudinal mode spacing is large as e.g. 100 GHz with a small-sized device less than 1 mm. If simply the wavelength of the variable wavelength filter is varied as when the wavelength is varied at a high speed, the operation of wavelength variation is a skipped operation from one longitudinal mode spacing to the next. This skipped variable wavelength operation with large skip distance is not desirable to be applied to optical coherence tomography. If simply the wavelength of the variable wavelength filter is changed, the operation is destabilized in a region between neighboring longitudinal modes. For example, multi-mode oscillations or non-continuous mode hops between the modes may be produced.

The technique disclosed in the Japanese Patent Application Laid-Open No.2006-278770, an optical ring resonator uses a plurality of optical resonators as a wavelength filter. Even though the wavelength may be varied under a vernier effect, wavelength variation is by a heater. Hence, the resonator does not lend itself to high speed scanning. It is difficult to adjust the resonator lengths of a plurality of optical ring resonators provided on the same substrate. Since the length of the entire laser including the optical ring resonators is short, the operation is destabilized between the longitudinal modes if simply the wavelength of the variable wavelength filter is varied. For example, multi-mode oscillations or non-continuous mode hops may be produced between the modes.

It is therefore an embodiment of the present invention is desirable to overcome these defects of the related art, and to provide a wavelength scanning laser light source in which the wavelength of a light source having a narrow range spectrum may be continuously scanned at a high speed over a broad range.

An embodiment of the present invention is further desirable to provide an optical coherence tomography device capable of continuously scanning the wavelength of a light source having a narrow range spectrum over a broad range at a high speed.

A wavelength scanning light source according to an embodiment of the present invention includes an optical amplifier provided in a light path of laser oscillation and having a gain bandwidth at a wavelength of oscillation, two Fabry-Perot resonators arranged in the light path of laser oscillation and having values of FSR (free spectral range) proximate to each other, an optical device that takes part of light propagated on the light path of laser oscillation, and a resonator length controller that periodically varies the resonator length of one of the two Fabry-Perot resonators having FSR values proximate to each other.

Another wavelength scanning light source according to an embodiment of the present invention includes a broadband light source, and a variable wavelength filter unit that takes out light of a desired wavelength range from broadband light outgoing from the broadband light source. The variable wavelength filter unit includes two Fabry-Perot electro-optic modulators each including a spatial Fabry-Perot resonator, and a resonator length controller. The Fabry-Perot electro-optic modulators are arranged in an outgoing path of light outgoing from the broadband light source and are each provided with an electro-optic crystal arranged therein. The values of FSR (free spectral range) of the Fabry-Perot electro-optic modulators are proximate to each other. The resonator length controller causes the resonator length of at least one of the two Fabry-Perot resonators having the FSR values proximate to each other to be periodically varied within a preset range. The light being propagated is optically modulated in at least one the two Fabry-Perot resonators by a periodic scanning signal afforded by the resonator length controller.

With the wavelength scanning light source, each of the two Fabry-Perot electro-optic modulators may be a ring resonation Fabry-Perot electro-optic modulator in which ring resonation is produced by a Fabry-Perot resonator having the electro-optic crystal arranged therein. The wavelength scanning light source may also include an optical amplifier provided on the outgoing light path of the variable wavelength filter unit, and a reflection mirror that reflects amplified spontaneous emission (ASE) radiated from the optical amplifier to cause the resulting light to be incident on the variable wavelength filter unit. The optical amplifier radiating the amplified spontaneous emission (ASE) may be used as the broadband light source.

An optical coherence tomography device according to an embodiment of the present invention may include a wavelength scanning light source including two Fabry-Perot resonators provided in a light path for laser oscillation. The values of FSR (free spectral range) of the Fabry-Perot resonators are proximate to each other. The resonator length of at least one of the two Fabry-Perot resonators is periodically varied to cause the two Fabry-Perot resonators to operate as a wavelength varying filter of a narrow bandwidth capable of varying the selection wavelength by the vernier effect to output temporally wavelength scanned laser light. The optical coherence tomography device may also include an interference optical system that causes the laser light output from the wavelength scanning light source to be branched into light for reference and light for observation and that generates interference light of reflected light of the light for observation illuminated on an object for observation and the light for reference. The optical coherence tomography device may further include a signal processing means for receiving the interference light obtained from the interference optical system for transforming the interference light received into an electrical signal to calculate the optical tomographic image information of the object for observation.

With the optical coherence tomography device according to an embodiment of the present invention, the wavelength scanning light source may be calibrated so that the wavenumber of laser light being scanned will be linear with respect to time.

With the optical coherence tomography device according to an embodiment of the present invention, the wavelength scanning light source may include an optical fiber loop that is to be a light path for laser oscillation, and an optical amplifier provided within the optical fiber loop and having a gain bandwidth at a wavelength of oscillation. The wavelength scanning light source may also include two Fabry-Perot resonators provided within the optical fiber loop and having values of FSR proximate to each other, and an optical device connected to the optical fiber loop to take out part of light propagated through the optical fiber loop. The wavelength scanning light source further includes a resonator length controller that periodically varies the resonator length of at least one of the two Fabry-Perot resonators having the values of FSR proximate to each other within a preset range.

With the optical coherence tomography device according to an embodiment of the present invention, the Fabry-Perot resonator whose resonator length is periodically varied within a preset range by the resonator length controller may be a Fabry-Perot electro-optic modulator provided with a pair electrode, and may modulate the propagated light by a periodic scanning signal afforded by the resonator length controller.

With the optical coherence tomography device according to an embodiment of the present invention, the two Fabry-Perot resonators having values of FSR proximate to each other may each be a Fabry-Perot electro-optic modulator and may each be adjusted by temperature control to the proximate FSR values.

With the optical coherence tomography device according to an embodiment of the present invention, the resonator length controller may afford reciprocally reversed scanning signals to the two Fabry-Perot resonators having proximate FSR values to vary the resonator lengths of the Fabry-Perot resonators in respective opposite directions.

With the optical coherence tomography device according to an embodiment of the present invention, the resonator length controller may superpose a control voltage on a scanning signal to control the center wavelength of light propagated through the two Fabry-Perot resonators having values of FSR proximate to each other.

With the optical coherence tomography device according to an embodiment of the present invention, the optical amplifiers may be arranged on the trailing sides of the two Fabry-Perot resonators.

With the optical coherence tomography device according to an embodiment of the present invention, the light may be separated by polarized light or the direction of light propagated through the optical fiber loop, and light may be amplified by the sole optical amplifier on the trailing sides of the two Fabry-Perot resonators.

With the optical coherence tomography device according to an embodiment of the present invention, the resonator length controller may adjust the waveform of the periodic scanning signal afforded to the Fabry-Perot resonator to calibrate the wavelength scanning light source so that the wavenumber of laser light taken outside by the optical device will be linear with respect to time.

With the optical coherence tomography device according to an embodiment of the present invention, the light path for laser oscillation may interconnect two Fabry-Perot electro-optic modulators in which spatial Fabry-Perot resonators with proximate values of FSR each having an electro-optic crystal arranged therein, an optical amplifier having a gain bandwidth at a wavelength of oscillation, and an optical device that takes output light to outside, over an optical fiber.

With the optical coherence tomography device according to an embodiment of the present invention, the two Fabry-Perot electro-optic modulators may each be a ring resonation Fabry-Perot electro-optic modulator designed to produce ring resonation by a Fabry-Perot resonator having an electro-optic crystal arranged therein.

With the optical coherence tomography device according to an embodiment of the present invention, the two Fabry-Perot resonators may each be a V-shaped resonation Fabry-Perot electro-optic modulator designed to produce V-shaped resonation by a Fabry-Perot resonator having an electro-optic crystal arranged therein.

With the optical coherence tomography device according to an embodiment of the present invention, the two Fabry-Perot electro-optic modulators may each be a confocal Fabry-Perot electro-optic modulator in which curvatures of respective concave mirrors are set so that the Fabry-Perot resonator having an electro-optic crystal arranged therein will prove to be a confocal resonator.

With the optical coherence tomography device according to an embodiment of the present invention, the Fabry-Perot electro-optic modulator may include two electro-optic crystals arranged with the C-axes at right angles to each other between two concave mirrors of the Fabry-Perot resonator.

With the optical coherence tomography device according to an embodiment of the present invention, the resonator length controller may adjust the waveform of the periodic scanning signal afforded to the Fabry-Perot resonator to calibrate the wavelength scanning light source so that the wavenumber of laser light taken outside by the optical device will be linear with respect to time.

With the optical coherence tomography device according to an embodiment of the present invention, the light path for laser oscillation may interconnect a Fabry-Perot electro-optic modulator in which a spatial Fabry-Perot resonators having an electro-optic crystal arranged therein, an optical amplifier having a gain bandwidth at a wavelength of oscillation, a polarized light converter that causes 90° rotation of the direction of polarized light outgoing from the Fabry-Perot electro-optic modulator, and an optical device that takes output light to outside, via an optical fiber. The Fabry-Perot electro-optic modulator may operate as two Fabry-Perot resonators having proximate values of FSR with respect to polarized light components that are perpendicular to each other. The polarized light components perpendicular to each other may be modulated by a periodic scanning signal afforded by the resonator length controller.

With the optical coherence tomography device according to an embodiment of the present invention, the Fabry-Perot electro-optic modulator may be a ring resonation Fabry-Perot electro-optic modulator adapted to produce ring-shaped resonation by a Fabry-Perot resonator having the electro-optic crystal arranged therein.

With the optical coherence tomography device according to an embodiment of the present invention, the Fabry-Perot resonator may be a V-shaped resonation Fabry-Perot electro-optic modulator adapted to produce V-shaped resonation by a Fabry-Perot resonator having the electro-optic crystal arranged therein.

With the optical coherence tomography device according to an embodiment of the present invention, the two Fabry-Perot electro-optic modulators may each be a confocal Fabry-Perot electro-optic modulator in which curvatures of respective concave mirrors are set so that the Fabry-Perot resonator having an electro-optic crystal arranged therein will prove to be a confocal resonator.

With the optical coherence tomography device according to an embodiment of the present invention, the Fabry-Perot electro-optic modulator may include two electro-optic crystals arranged between the concave mirrors of the Fabry-Perot resonator with the C-axes at right angles to each other.

With the optical coherence tomography device according to an embodiment of the present invention, the resonator length controller may adjust the waveform of the periodic scanning signal afforded to the Fabry-Perot resonator to calibrate the wavelength scanning light source so that the wavenumber of laser light taken outside by the optical device will be linear with respect to time.

With the optical coherence tomography device according to an embodiment of the present invention, the two Fabry-Perot electro-optic modulators may each be a ring resonation Fabry-Perot electro-optic modulator designed to produce ring-shaped resonation by a Fabry-Perot resonator having an electro-optic crystal arranged therein.

With the optical coherence tomography device according to an embodiment of the present invention, the two Fabry-Perot electro-optic modulators may each be a polarized wave non-dependent ring resonation Fabry-Perot electro-optic modulator. The wavelength scanning light source may include a polarized wave non-dependent optical amplifier having a gain bandwidth at a wavelength of oscillation, and which is arranged in a light path for laser oscillation.

Yet another optical coherence tomography device according to an embodiment of the present invention includes a broadband light source and a wavelength scanning light source. The wavelength scanning light source includes two Fabry-Perot resonators provided in a light path for laser oscillation of light outgoing from the broadband light source. The values of FSR (free spectral range) of the Fabry-Perot resonators are proximate to each other. The resonator length of at least one of the two Fabry-Perot resonators is periodically varied within a preset range to cause the two Fabry-Perot resonators to operate as a narrow bandwidth variable wavelength filter capable of periodically varying the selection wavelength by the vernier effect to output laser light that has temporally scanned the wavelength. The optical coherence tomography device also includes an interference optical system that causes the laser light output from the wavelength scanning light source to be branched into light for reference and light for observation and that generates interference light of reflected light of the light for observation illuminated on an object for observation and the light for reference. The optical coherence tomography device further includes a signal processing means for receiving the interference light obtained from the interference optical system for transforming the interference light received into an electrical signal to calculate the optical tomographic image information of the object for observation.

According to an embodiment of the present invention, the two Fabry-Perot resonators operate as a narrow bandwidth variable wavelength filter capable of varying the selection wavelength by a vernier effect to provide a wavelength scanning light source that allows for outputting temporally wavelength scanned light and for high-speed wavelength scanning. An OCT signal may thus be acquired at a high speed. By adjusting the waveform of the periodic scanning signal afforded by the resonator length controller to the Fabry-Perot resonator, the wavenumber ($2\pi$ / wavelength) of the laser light taken outside by the optical device is calibrated so that the wavenumber will be linear with respect to time. The produced interference light is re-arrayed so as to provide for an equal spacing with respect to the wavenumber ($2\pi$ / wavelength) of the laser light, such that there is no necessity for performing signal processing. It is thus possible to provide an optical coherence tomography device capable of performing continuous high-speed scanning of the wavelength of a light source of a narrow bandwidth spectrum over a wide range without the necessity of performing signal processing for calibration on the OCT signal.

Other objects and specified advantages of an embodiment of the present invention will become more apparent from the following detailed explanation of preferred exemplary embodiments of the invention.

Brief Description of the Drawings

Fig.1 is a block diagram for illustrating the principle of an optical coherence tomography device which is based on a Michelson interferometer.
Fig.2 is a block diagram showing a formulation of a variable wavelength light source by a ring laser using an erbium doped fiber.

Fig.3 is a block diagram showing a basic arrangement of an optical coherence tomography device according to an embodiment of the present invention.

Fig.4 is a block diagram showing a basic arrangement of a wavelength scanning laser light source used in the optical coherence tomography device.

Fig.5A is a graph showing the transmission spectrum of a commonplace optical resonator.

Fig.5B is a more detailed graph showing the transmission spectrum of a commonplace optical resonator.

Figs.6A to 6G are graphs each showing the transmission spectrum of two tandem-connected optical resonators different in FSR by 1%.

Fig.7 is a graph that has partially enlarged portion of the transmission spectrum shown in Fig.6.

Fig.8 is a block diagram showing an actual arrangement of a wavelength scanning laser light source in which the two Fabry-Perot resonators are each a Fabry-Perot electro-optic modulator.

Fig.9 is a block diagram showing an actual example formulation of a fiber ring laser for a wavelength band of 1.5 μm employing an optical fiber amplifier, two com generator modules and an optical coupler.

Fig.10 is a graph showing measured results of the fiber ring laser.

Fig.11 is a block diagram showing an actual example formulation of a wavelength scanning laser light source including two optical amplifiers arranged on the trailing ends of the two Fabry-Perot electro-optic modulators.

Fig. 12 is a block diagram showing an example formulation of a wavelength scanning laser light source in which a sole optical amplifier is arranged in rear of two Fabry-Perot electro-optic modulators to amplify light.

Fig.13 is a block diagram showing another example formulation of a wavelength scanning laser light source in which a sole optical amplifier is arranged in rear of two Fabry-Perot electro-optic modulators to amplify light.

Fig.14 is a block diagram showing an arrangement of a system for measuring the relationship between a scanning signal voltage and the laser frequency in a wavelength scanning laser light source.

Figs.15A and 15B are graphs showing measured results of the relationship between the scanning signal voltage and the laser frequency.

Figs.16A to 16C are graphs showing results observed of the waveform of the scanning signal afforded by a resonator length controller to the Fabry-Perot resonators and the waveform of the interference signal obtained by an interferometer.

Fig.17 is a block diagram showing an example formulation of a wavelength scanning laser light source with which linear laser light having a wavenumber linear with respect to time may be obtained via an optical coupler.

Fig.18 is a block diagram showing a basic formulation of a wavelength scanning laser light source having two confocal Fabry-Perot electro-optic modulators.

Fig.19 is a cross-sectional view schematically showing the structure of a confocal ring-shaped resonation Fabry-Perot electro-optic modulator used as the aforementioned wavelength scanning laser light source.

Fig.20 is a cross-sectional view schematically showing an example formulation of two confocal Fabry-Perot electro-optic modulators in which two electro-optic crystals are arranged between two concave mirrors with their C-axes at right angles to each other.

Fig.21 is a block diagram showing an example formulation of the resonator length controller configured for controlling the center wavelength of a bandpass filter composed of two Fabry-Perot resonators.

Fig.22 schematically shows a connection structure for transmitting a modulated signal of two confocal Fabry-Perot electro-optic modulators in which two electro-optic crystals are arranged between two concave mirrors so that their C-axes will be at right angles to each other.

Fig.23 is a schematic view showing an example fabrication of a monolithic Fabry-Perot electro-optic modulator.

Fig.24A is a schematic view similarly showing a process for fabrication of the monolithic Fabry-Perot electro-optic modulator.

Fig.24B is a schematic view similarly showing a process for fabrication of the monolithic Fabry-Perot electro-optic modulator.

Fig.24C is a schematic view similarly showing a process for fabrication of the monolithic Fabry-Perot electro-optic modulator.

Fig.24D is a schematic view similarly showing a process for fabrication of the monolithic Fabry-Perot electro-optic modulator.

Fig.24E is a schematic view similarly showing a process for fabrication of the monolithic Fabry-Perot electro-optic modulator.

Fig.24F is a schematic view similarly showing a process for fabrication of the monolithic Fabry-Perot electro-optic modulator.

Fig.25 is a schematic view showing a monolithic Fabry-Perot electro-optic modulator having a light incident/ light outgoing optical system including a prism-shaped mirror on an input plane.

Fig.26 is a schematic view showing a monolithic Fabry-Perot electro-optic modulator having a light incident/ light outgoing optical system in which a ring-shaped resonator and a fiber loaded on a two-core ferule by a prism are linked together by the prism.

Fig.27 is a block diagram showing another example formulation of a wavelength scanning laser light source provided with two confocal Fabry-Perot electro-optic modulators

Fig.28 is a block diagram showing a basic formulation of a wavelength scanning laser light source having a sole confocal Fabry-Perot electro-optic modulator.

Fig.29 is a block diagram showing another formulation of a wavelength scanning laser light source having a sole confocal Fabry-Perot electro-optic modulator.

Fig.30 is a block diagram showing a further arrangement of a wavelength scanning laser light source having a sole confocal Fabry-Perot electro-optic modulator.

Fig.31 is a block diagram showing a further arrangement of a wavelength scanning laser light source having a sole confocal Fabry-Perot electro-optic modulator.

Fig.32 is a block diagram showing yet another arrangement of a wavelength scanning laser light source having a sole confocal Fabry-Perot electro-optic modulator.

Fig.33 is a cross-sectional view schematically showing the structure of a confocal V-shaped resonation Fabry-Perot electro-optic modulator used for a wavelength scanning laser light source.

Fig.34 is a block diagram showing an arrangement of a linear resonator wavelength scanning laser light source formed by two confocal V-shaped resonation Fabry-Perot electro-optic modulators.

Fig.35 is a block diagram showing an arrangement of a linear resonator wavelength scanning laser light source formed by a sole confocal V-shaped resonation Fabry-Perot electro-optic modulator.

Fig.36 is a block diagram showing an arrangement of a wavelength scanning laser light source including a polarized wave non-dependent ring resonation Fabry-Perot electro-optic modulator and a polarized wave non-dependent optical amplifier in a laser oscillation light path.

Fig.37 is a block diagram showing an arrangement of a wavelength scanning laser light source including a sole ring Fabry-Perot electro-optic modulator remodeled from the wavelength scanning laser light source shown in Fig.36.

Fig.38 is a block diagram showing the arrangement of a non-laser wavelength scanning light source provided in the optical coherence tomography device.

Figs.39A and 39B are graphs schematically showing characteristics of light of a narrow wavelength obtained with the non-laser wavelength scanning light source.

Fig.40 is a block diagram showing another example formulation of the non-laser wavelength scanning light source provided in the optical coherence tomography device.

Preferred Mode for carrying out the Invention

The preferred mode for carrying out an embodiment of the present invention will now be described in detail with reference to the drawings.

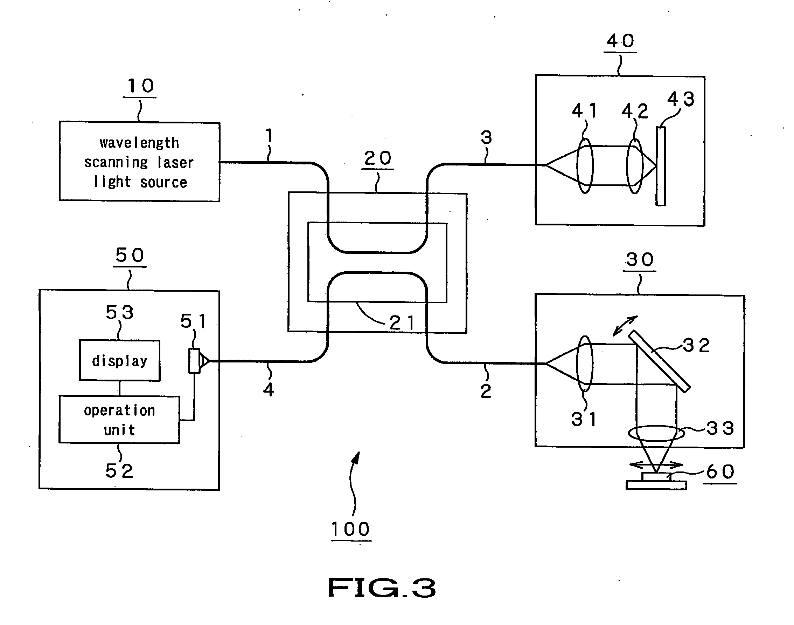

An embodiment of the present invention is applied to an optical coherence tomography device 100 configured as shown for example in Fig.3.

The optical coherence tomography device 100 is made up of a wavelength scanning laser light source 10, an interference optical system 20, a scanning optical system 30, a reference optical system 40 and a signal processor 50. The interference optical system 20 is connected to the wavelength scanning laser light source 10 via an optical fiber 1, and the scanning optical system 30 is connected to the interference optical system 20 via an optical fiber 2. The reference optical system 40 is connected to the interference optical system 20 via an optical fiber 3, and the signal processor 50 is connected to the interference optical system 20 via an optical fiber 4.

With the wavelength scanning laser light source 10 in this optical coherence tomography device 100, two Fabry-Perot resonators are caused to operate as a narrow-band variable wavelength filter capable of varying the selection wavelength by a vernier effect, thereby outputting temporally wavelength scanned laser light, as will be set forth subsequently in detail. The interference optical system 20 branches the laser light output from the wavelength scanning laser light source 10 into light for reference and light for observation to be illuminated on an object for observation 60. The interference optical system generates interference light of reflected light of the light for observation illuminated on the object for observation 60 and light which is return light of the light for reference. For example, the interference optical system includes a fiber coupler 21 on which the laser light output from the wavelength scanning laser light source 10 is incident via the optical fiber 1.

The fiber coupler 21 in the interference optical system 20 branches the laser light, incident thereon from the wavelength scanning laser light source 10 via the optical fiber 1 into the light for observation transmitted to the scanning optical system 30 via the optical fiber 2, and into the light for reference transmitted to the reference optical system 40 via the optical fiber 3. The fiber coupler 21 transmits the light for observation via the optical fiber 2 to the scanning optical system 30, while transmitting the light for reference via the optical fiber 3 to the reference optical system 40. The fiber coupler 21 also generates interference light of the reflected light of the light of observation illuminated on the object for observation 60 and returned back from the scanning optical system 30 via the optical fiber 2, and the light for reference returned back from the reference optical system 40 via the optical fiber 3 and transmits the so generated interference light via the optical fiber 4 to the signal processor 50.

The scanning optical system 30 spatially sweeps the light for observation branched by the interference optical system 20, to illuminate the so scanned light for observation on the object for observation 60. The scanning optical system also causes the reflected light from the object for observation 60 to be returned to the interference optical system 20. Specifically, the scanning optical system is made up by a lens 31, a scanning mirror 32 with a variable scanning angle, and a lens 33.

The light for observation branched by the fiber coupler 21 of the interference optical system 20 and transmitted via the optical fiber 2 to the scanning optical system 30 is illuminated via the lens 31, scanning mirror 32 with a variable scanning angle and the lens 33 on the object for observation 60. The light reflected from the object for observation 60 is returned back to the fiber coupler 21 via a reversed route.

The reference optical system 40 returns the light for reference branched at the interference optical system 20 back to the interference optical system 20 after reflection on a fixed reference mirror 43, and is made up of lenses 41, 42 and the fixed reference mirror 43.

The light for reference branched by the fiber coupler 21 of the interference optical system 20 and transmitted to the reference optical system 40 via the optical fiber 3 is illuminated on the fixed reference mirror 43 via the lenses 41 and 42. The light for reference is reflected by this fixed reference mirror 43 and returned to the fiber coupler 21 via the reverse route.

The fiber coupler 21 of the interference optical system 20 generates the interference light of the reflected light of the light for observation illuminated on the object for observation 60, and the light for reference. The so generated interference light is transmitted via the optical fiber 4 to the signal processor 50.

The signal processor 50 receives the interference light obtained by the interference optical system 20, and transforms the so received interference light into an electrical signal to calculate the optical tomographic image information of the object for observation 60. Specifically, the signal processor 50 is made up by a photodetector 51, an operation unit 52 and a display 53.

The signal processor 50 receives the interference light transmitted via the optical fiber 4 from the fiber coupler 21 of the interference optical system 20 by the photodetector 51, and transforms the light into an electrical signal, which is detected as a spectral interference signal. The operation unit 52 captures the spectral interference signal detected by the photodetector 51, and performs Fourier transform on the spectral interference signal at an equal frequency spacing to calculate the optical tomographic image information along the depth-wise direction of the object for observation 60 and along the scanning direction of the scanning mirror 32. The operation unit demonstrates the tomographic image on the display 53.

With the optical coherence tomography device 100, the wavelength scanning laser light source 10 is a scanning light source with a temporally changing wavelength, that is, a light source of light whose wavelength exhibits time dependency. That is, the reflectance distribution along the depth-wise direction of the object for observation 60 may be obtained to get the structure along the depth-wise direction without the necessity of scanning the reference mirror 43. It is thus possible to form a two-dimensional tomographic image simply by performing the scanning along the one-dimensional direction.

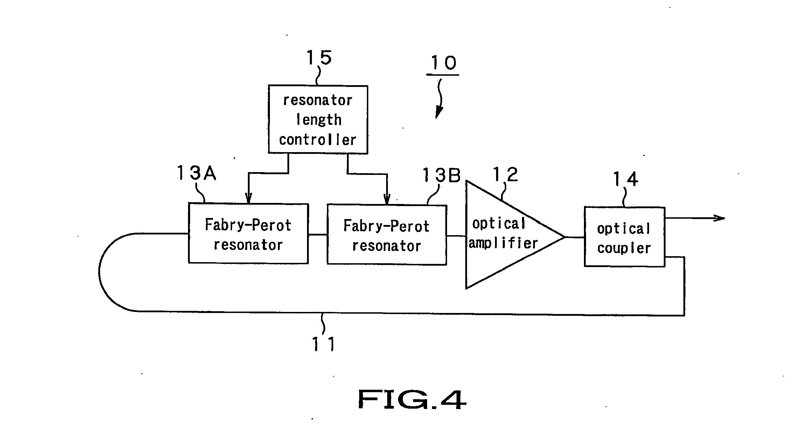

With the wavelength scanning laser light source 10 of the optical coherence tomography device 100, the resonator length of at least one of two Fabry-Perot resonators, provided on the light path of laser oscillation and which has values of FSR (Free spectral range) proximate to each other, is periodically changed within a preset range. By so doing, the two Fabry-Perot resonators may be in operation as a variable wavelength filter with a narrow bandwidth based on a vernier effect and may thus output laser light having temporally scanned wavelengths. The basic formulation of the wavelength scanning laser light source is shown in Fig.4, and includes an optical fiber loop 11 providing a light path for laser oscillation, and an optical amplifier 12 provided on the optical fiber loop 11 and having a gain bandwidth at a wavelength of oscillation. The wavelength scanning laser light source also includes two Fabry-Perot resonators 13A, 13B provided within the optical fiber loop 11 and having values of FSR (fiber spectral range) proximate to each other. The wavelength scanning laser light source also includes an optical device, such as an optical coupler 14, which is connected to the optical fiber loop 11 and which takes out part of light passing on the optical fiber loop 11. The wavelength scanning laser light source further includes a resonator length controller 15 that periodically changes the resonator length of one of the two Fabry-Perot resonators 13A, 13B having values of FSR proximate to each other, here the Fabry-Perot resonator 13A, within a preset range.

In this wavelength scanning laser light source 10, the two Fabry-Perot resonators 13A, 13B having values of FSR proximate to each other operate as respective wavelength selection filters. At least one of the two Fabry-Perot resonators 13A, 13B, here the Fabry-Perot resonator 13A, may be varied in resonator length to vary its selection wavelength.

The two Fabry-Perot resonators 13A, 13B having values of FSR proximate to each other operate as a bandpass filter with a narrow bandwidth wavelength selection characteristic based on a vernier effect. This is made possible by varying the resonator length of one 13A of the two Fabry-Perot resonators 13A, 13B having the values of FSR proximate to each other.

With this wavelength scanning laser light source 10, the light propagated through the bandpass filter formed by the two Fabry-Perot resonators 13A, 13B provided on the optical fiber loop 11 and having values of FSR proximate to each other is amplified by the optical amplifier 12 and fed back via the optical fiber loop 11. This sets the laser light into oscillation. With this wavelength scanning laser light source 10, the resonator length of the Fabry-Perot resonator 13A is periodically varied by the resonator length controller 15 within a preset range. This causes the oscillation wavelength to be periodically varied to cause periodic change in the wavelength of the laser light taken out via the optical coupler 14.

The optical coupler 14 that takes out laser light from the optical fiber loop 11 is provided in rear of the optical amplifier 12. However, the optical coupler may also be provided ahead of the optical amplifier 12 or intermediate between the two Fabry-Perot resonators 13A, 13B.

With the wavelength scanning laser light source 10 set forth above, it is possible to elongate the resonator length of the optical fiber loop 11. For example, the resonator length of the optical fiber loop 11 may be set to 1000m, in which case the longitudinal mode spacing of the laser in its entirety may be made as narrow as ca. 200 kHz. By so doing, the longitudinal mode spacing of the laser in its entirety may be made sufficiently smaller than the bandwidth of each mode of the Fabry-Perot resonators 13A, 13B (FSR / finesse, for example, 2.5GHz / 50 = 50 MHz). It is thus possible to eliminate the effect of mode hop without changing the resonator length. Hence, by simply changing the selection wavelength of the bandpass filter, it is possible to effect pseudo-continuous wavelength variations, even granting that the mode of oscillation is not the single mode oscillation in the strict sense of the term.

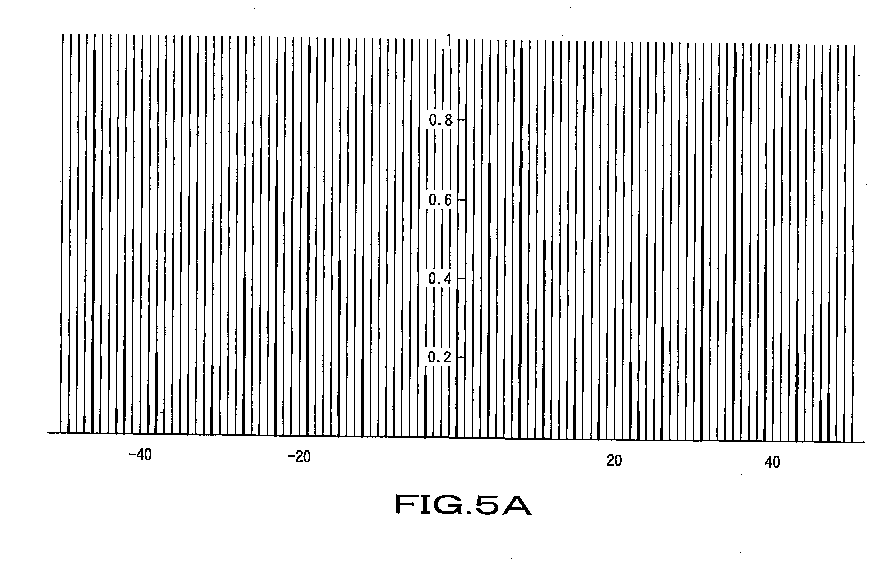

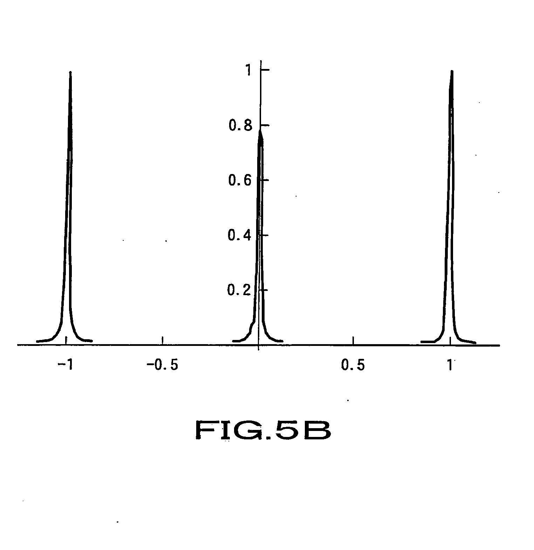

Figs.5A, 5B depict transmission spectra of commonplace optical resonators. The abscissa stands for the light frequency which is normalized by setting FSR = 1 and shows a range of 100×FSR. 100 modes, each one of which is for each FSR, may be seen. Fig.5B is an enlarged view of Fig.5A.

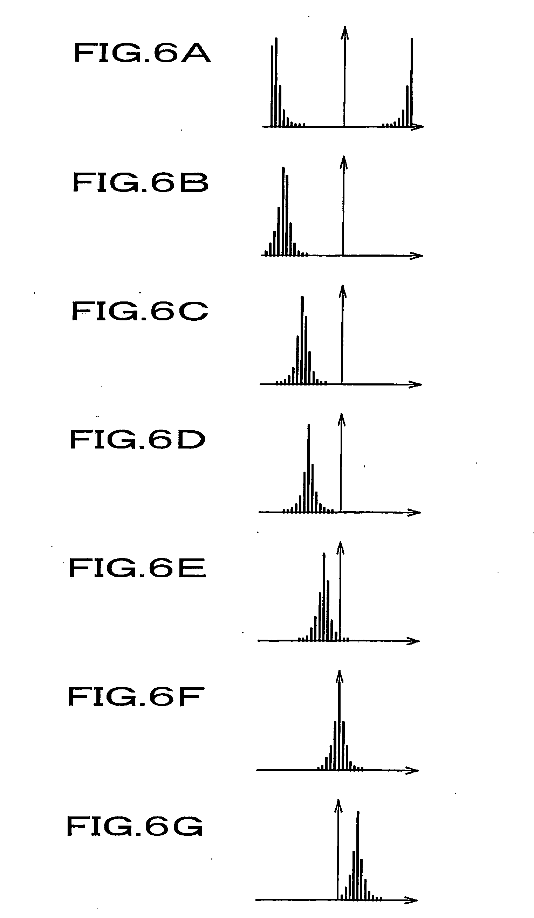

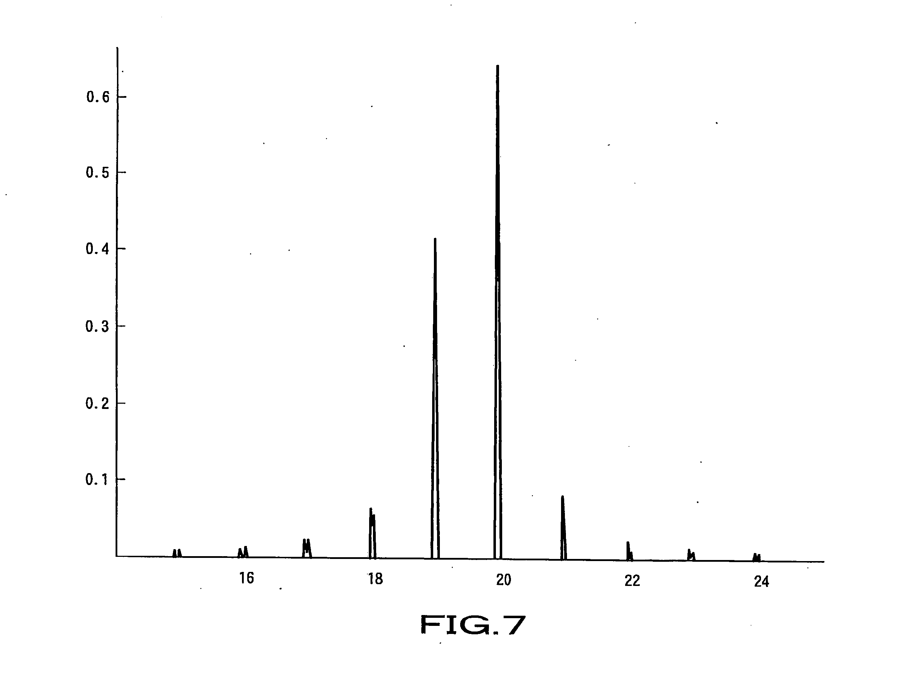

Figs.6A to 6G show transmission spectra in case two optical resonators whose values of FSR differ by 1% are arranged in tandem. Although the abscissas of these figures are the same as that of Fig.5A, the mode separations of the two optical resonators differ from each other. Hence, the envelope of the spectrum constituted by the mode of the small FSR becomes equivalent to that of the optical resonator having the large FSR due to the vernier effect. The difference of 1% of the FSR increases the FSR of the envelope of the moiré streak by a factor of 100. On the other hand, if the resonator length is varied by ca. one wavelength, the peak is varied by FSR of the envelope of the moiré streak. Fig.7 shows part of Fig.6 to an enlarged scale.

Thus, even in case an optical resonator of a small FSR is used, but the vernier effect of the spectra of two optical resonators is exploited, the FSR of the envelope of the moiré streak increases in inverse proportion to the difference in the values of FSR of the two optical resonators.

That is, even if an optical resonator of small FSR is used, a variable wavelength laser light source may be constituted by exploiting the vernier effect of the spectra of two optical resonators.

In this case, the laser wavelength is skipped by a distance equal to the FSR, for example, 2.5 GHz, for a spacing equivalent to the FSR of two optical resonators. However, in case of application to optical CT, wavelength variations may be regarded as pseudo-continuous if the range of measurement along the depth-wise direction is sufficiently narrower than c/FSR (ca. 10cm).

Also, wavelength variations may be achieved under an electro-optic effect by using a modulator termed a Fabry-Perot electro-optic modulator or a light com generator. This modulator is composed of a Fabry-Perot resonator formed of a material whose refractive index is variable, such as LN(LiNbO$_3$), as an optical resonator, and a pair electrode. Since modulation with this modulator is electrical modulation, it is superior in linearity and reproducibility.

Hence, the Fabry-Perot electro-optic modulator is used as the Fabry-Perot resonator 13A in the wavelength scanning laser light source 10 whose resonator length is variable. The resonator length controller 15 applies a periodic signal such as serrated signal to the Fabry-Perot electro-optic modulator to effect optical modulation to scan the oscillation wavelength of the wavelength scanning laser light source 10 to high accuracy at an elevated speed.

Moreover, the Fabry-Perot electro-optic modulator may have its length adjusted by polishing and may have a resonator length accurately controlled under temperature control. Hence, subject to temperature control, the resonator length may be controlled within 1 ppm in a desired range. Hence, a Fabry-Perot electro-optic modulator with an FSR equal to 2.5 GHz and another Fabry-Perot electro-optic modulator different in FSR by 1/4000, with a total of two Fabry-Perot electro-optic modulators may be provided with ease. This will readily yield a wavelength selection device of an FSR of 10 THz, higher by a factor of 4,000, under the vernier effect.

For each of the two Fabry-Perot resonators 13A, 13B in the wavelength scanning laser light source 10, having the values of FSR proximate to each other, a Fabry-Perot electro-optic modulator whose length has been adjusted on polishing is used. The absolute value of the resonator length of each Fabry-Perot resonator is adjusted under temperature control. Meanwhile, if two optical resonators can be manufactured with a length variation by an accurately necessary quantity, the difference in FSR may be made constant by thermally contacting the resonators so that these resonators will be at the same temperature, thereby assuring a constant difference in the FSR. Hence, there is no necessity of conducting temperature control. For example, the difference in FSR may be realized by adjustment of a waveguide path process. In the case of a waveguide path by Ti diffused waveguide in LN, a waveguide Fabry-Perot electro-optic modulator with an FSR difference of 1/4000 may be manufactured by adjusting the Ti doping quantity so that the two optical resonators will differ in refractive index by 1/4000.

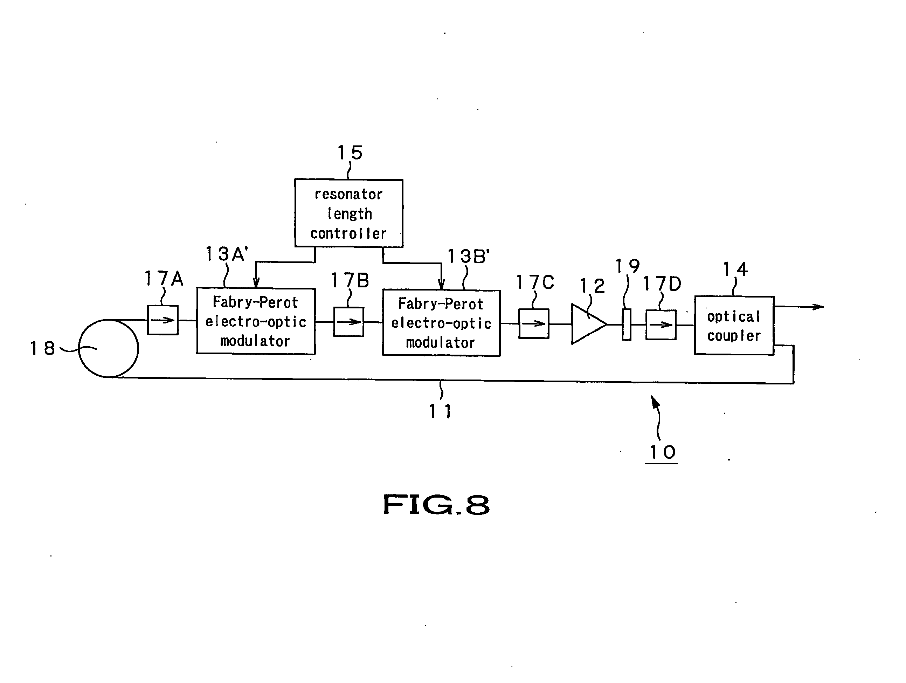

Fig.8 shows an example formulation of a wavelength scanning laser light source 10 in which Fabry-Perot electro-optic modulators 13A', 13B' are used as the two Fabry-Perot resonators 13A, 13B.

The Fabry-Perot electro-optic modulator now available suffers from reflection. Hence, optical isolators 17A to 17D are inserted in the wavelength scanning laser light source 10 shown in Fig.8. There is also inserted a delay line 18 which is a fiber of 1 km, for example. Thus, the time for light to make a round trip on the optical fiber loop 11 is 5 μs. Hence, the mode spacing is of the period of 200 kHz. The entire system of the wavelength scanning laser light source 10 is constructed in such a manner as to save the state of the polarized wave. Thus, a polarizer 19 is interposed between the optical amplifier 12 and the optical isolator 17C for determining the state of polarization of light propagated through the optical fiber loop 11 as the light path of laser oscillation. For delay, a Faraday mirror, an SM fiber and a PBS coupler may be used. The scanning frequency may be an integer number of times of 200 kHz referred to above.

It is observed that the polarizer 19 performs the role of determining the state of polarization of light propagated through the optical fiber loop 11 as the light path of laser oscillation, and basically, the polarizer may be inserted at any location desired in the loop.

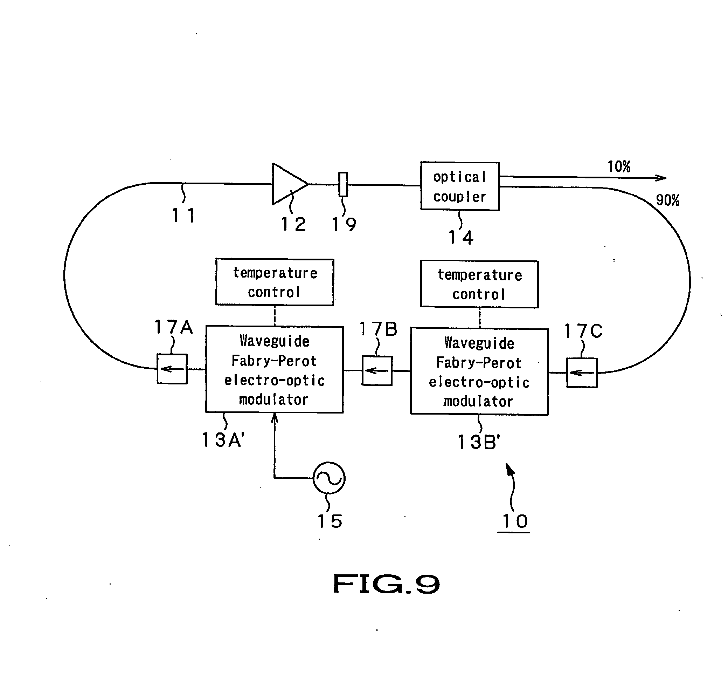

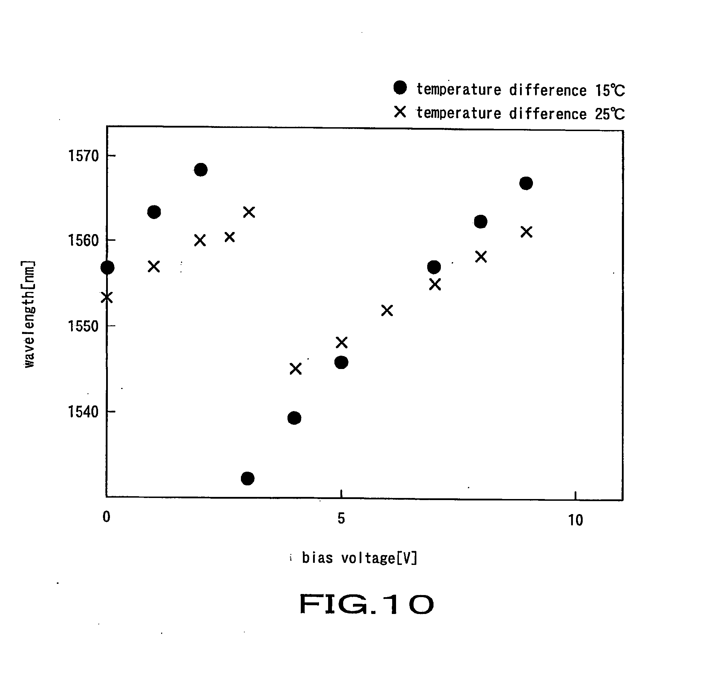

A fiber ring laser shown in Fig.9 was constructed using an optical amplifier 12, Fabry-Perot resonators 13A, 13B and an optical coupler 14 in the wavelength scanning laser light source 10. As this optical coupler 14, an optical fiber amplifier EFDA, two com generator modules OFCG1, OFCG2 and an optical coupler PC for the wavelength of 1.5 μm bandwidth were used. Measurements were made of the wavelength variation characteristic by a bias voltage applied to the com generator module OFCG1. It has been found that the so constructed fiber ring laser operates as a scanning laser light source capable of high speed wavelength scanning. Fig.10 shows measured results.

With a greater temperature difference, a marked difference in the values of FSR is produced, with the result that wavelength changes are moderate and the range of wavelength variation is reduced. As the temperature difference becomes smaller, wavelength changes become sensitive to the bias voltage and the range of wavelength variation is increased. For the temperature difference of 15°C and 25°C, the ranges of wavelength variation are ca. 33 nm and 15 nm, respectively.

As the optical amplifier 12, an optical fiber amplifier PM EDFA, a trade name of a product of IPG Photonics Corporation, was used with Pout = +22.6 dBm. As the Fabry-Perot resonators 13A, 13B, two waveguide FP-EO modulators WR-250-03, a trade name of a product manufactured by Optical Comb, Inc., with an FSR at ambient temperature of 2.5 GHz, were used. The temperature of the first waveguide Fabry-Perot electro-optic modulator 13A' was shifted with respect to that of the second waveguide Fabry-Perot electro-optic modulator 13B' to shift the values of FSR to construct a variable wavelength filter. As the waveguide Fabry-Perot electro-optic modulators 13A', 13B', those with FSR changing ca. 0.08 MHz per °C were used. By temperature control, with the use of e.g. a Peltier device, the temperature of the first waveform Fabry-Perot electro-optic modulator 13A' was set at a fixed value of 20°C, while that of the second waveguide Fabry-Perot electro-optic modulator 13B' was set at a variable value between 33°C and 45°C.

As the optical coupler 14, a 10:90 coupler, that is, a coupler outputting 10% was used.

The optical isolators 17A, 17B and 17C were used one at an input and one at an output of each of the waveguide Fabry-Perot electro-optic modulators 13A', 13B'.

The waveguide Fabry-Perot electro-optic modulators 13A', 13B' each suffer from the loss of ca. 7 dB, so that the two Fabry-Perot electro-optic modulators 13A', 13B' suffer from the total loss of 14 dB. Since these losses lead to increased ASE of the laser light, two optical amplifiers 12A, 12B are arranged in rear of the waveguide Fabry-Perot electro-optic modulators 13A', 13B'. By so doing, light amplification may occur downstream of the respective waveguide Fabry-Perot electro-optic modulators 13A', 13B' to render it possible to suppress the loss of the ASE from increasing due to loss in the waveguide Fabry-Perot electro-optic modulators 13A', 13B'.

Or, referring to Figs,12 and 13, polarized light components may be separated by polarized light converters 19A, 19B and PBS couplers 20A, 20B to set the light path. The polarized light converters 19A, 19B cause rotation by 90° of the direction of polarization of light being propagated through the optical fiber loop 11. A sole optical amplifier 12 is used as two optical amplifiers 12A, 12B to amplify light downstream of the waveguide Fabry-Perot electro-optic modulators 13A', 13B'. By so doing, it is similarly possible to suppress the loss of the ASE from increasing due to loss in the waveguide Fabry-Perot electro-optic modulators 13A', 13B'.

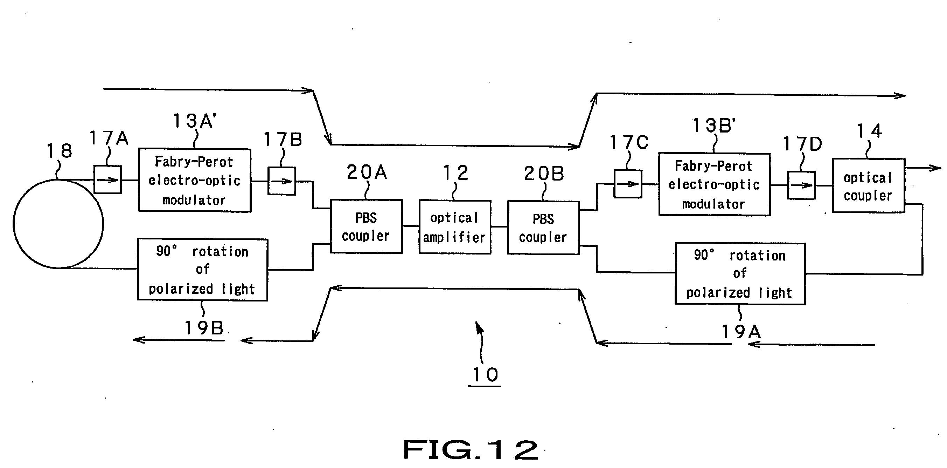

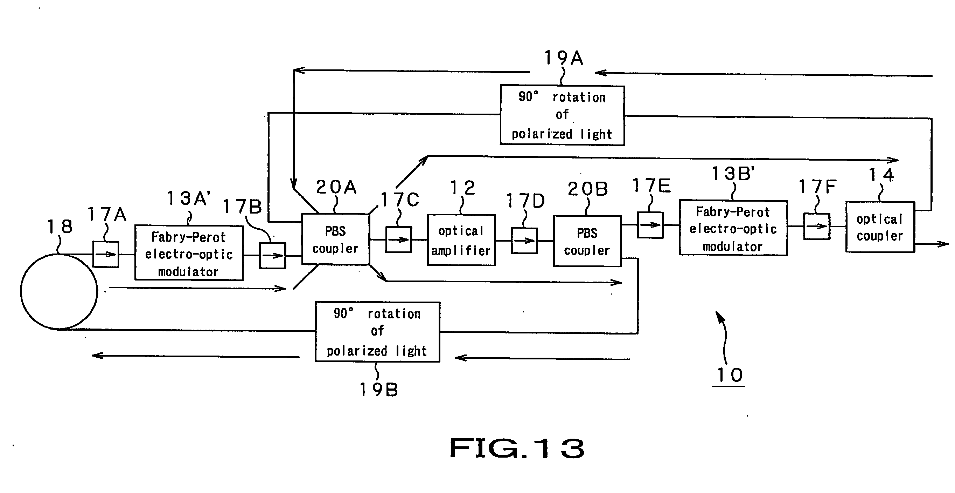

It is observed that an optical amplifier such as SOA (semiconductor optical amplifier) is able to amplify polarized light without regard to the polarized light component or its direction. Thus, by using the SOA, light may be amplified twice during the time the light completes one round trip. With the wavelength scanning laser light source 10 shown in Fig.12, light is separated based on the direction of light and on the sorts of the polarized light, and light is amplified using the sole optical amplifier 12 formed by SOA. The PBS couplers 20A, 20B may be replaced by a circulator. With the wavelength scanning laser light source 10 shown in Fig.13, light is propagated through the optical amplifier 12 in the same direction and light separation is by the difference in the polarized light components.

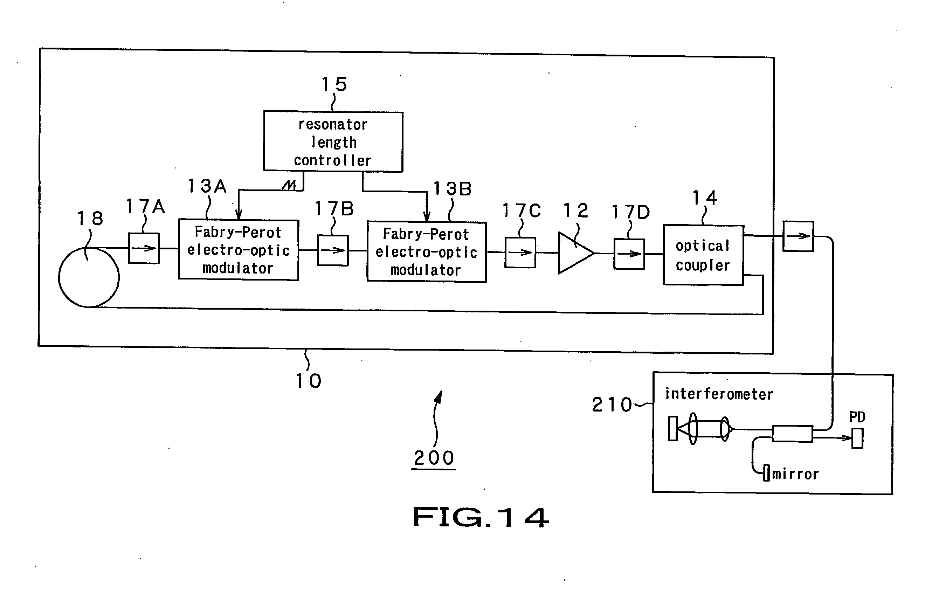

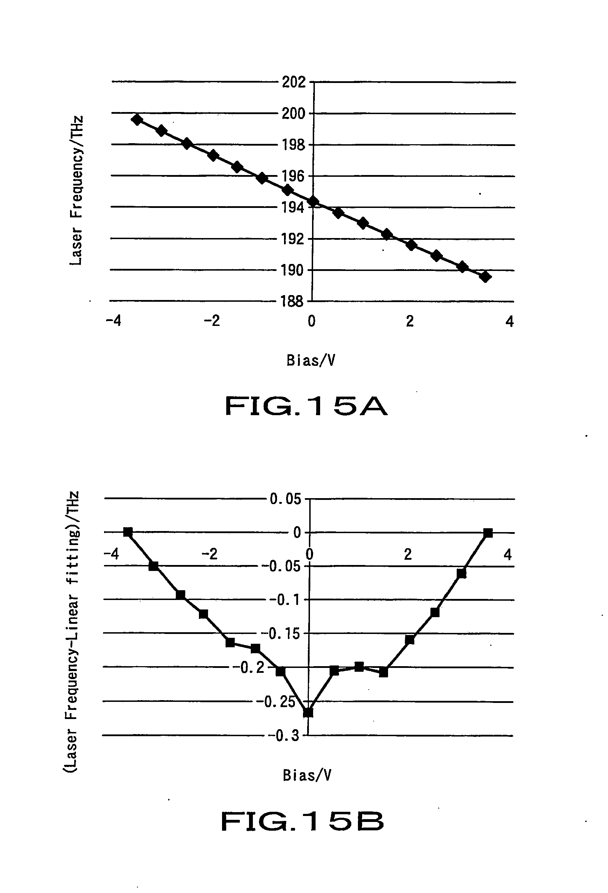

A measurement system 200 shown in Fig.14 was constructed, and the wavelength of the laser light, output from the vernier effect based wavelength scanning laser light source 10, was measured by an interferometer 210. The relationship between the laser frequency and the scanning signal voltage is shown in Fig.15A. The scanning signal voltage applied by the resonator length controller 15 causes the length of one 13A of the two Fabry-Perot electro-optic modulators 13A, 13B to be changed. The two Fabry-Perot electro-optic modulators compose the band-pass filter as set forth above. Fig. 15B shows deviations of the measured values from the linear approximate values. In Figs.15A and 15B, the abscissas stand for the scanning signal voltages. The ordinate of Fig.15A stands for the laser frequency for the wavelength values obtained by the measurements, and that of Fig.15B stands for deviations from the linear approximation of measured values of the laser frequency.

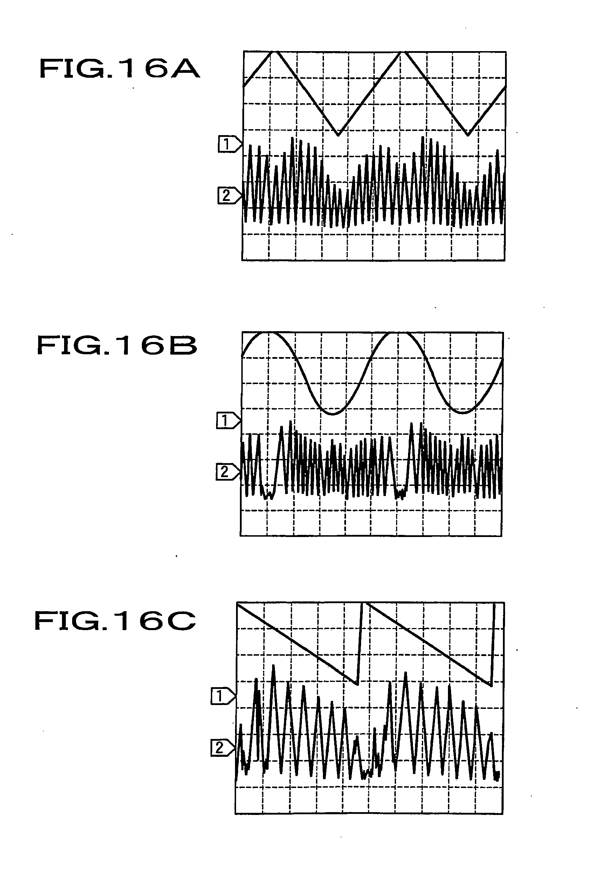

Measurements were made of the waveform of the scanning signal afforded by the resonator length controller 15 to the Fabry-Perot resonator 13A and that of the interference signal obtained with the interferometer 210. The waveforms of the interference signal that follow the waveforms of the scanning signal (triangular, sinusoidal and serrated waveforms) were obtained as shown in Figs.16A to C.

With the measurement system 200 shown in Fig.14, an SOA (semiconductor optical amplifier) was used as the optical amplifier 12 of the wavelength scanning laser light source 10, and a delay line formed by an SM fiber 1 km long, a Faraday mirror and a PBS coupler, was used as a delay line 18. In addition, two channels of function generators synchronized with each other were used as the resonator length controller 15, in order that the light for each of the two Fabry-Perot resonators 13A, 13B will be modulated with an optional waveform.

With this measurement system 200, the range of wavelength variation is enlarged through the use of SOA as the optical amplifier 12. Referring to Fig.15A, there are shown measured results of changes in the frequency (wavelength) caused on application of a bias voltage to one 13A of the two Fabry-Perot resonators. It is seen from the data shown that the wavelength variation has reached 10 THz. The frequency of 10 THz corresponds to 80 nm which is larger than experimental data of 33 nm of Fig.10 obtained with the system of Fig.9. As the temperature-related condition, the difference between the temperatures of the two Fabry-Perot resonators 13A, 13B is set to 8°C.

Moreover, with the present measurement system 200, modulation has been made possible with an integer number times 100 kHz by inserting the delay line 18 in the wavelength scanning laser light source 10. The delay line composed of 1 km of an SM fiber, a Faraday mirror and a PBS coupler is equivalent to a delay line of 2 km because light is propagated back and forth on the fiber. The data of Fig.16 is data with the scanning frequency of 200 kHz, and the temperature condition is 8°C which is the same as above. The scanning range is 10THz.

By using the synchronized 2-channel function generators as the resonator length controller 15, and by applying inverted scanning signals to the Fabry-Perot resonators 13A, 13B, optimum modulation could be obtained without dependency on the wavelength change directions, as shown in Fig.16. If the scanning signal exceeding 100 kHz is applied to only the Fabry-Perot resonator 13A, there occurred malfunctions such as light intensity differing with the wavelength change directions or cessation of laser oscillations. However, these malfunctions were appreciably moderated by applying inverted scanning signals to the respective Fabry-Perot resonators 13A, 13B. The scanning frequency up to at least 1 MHz could be confirmed.

If the reversed scanning signals are applied to the respective Fabry-Perot resonators 13A, 13B, the distance between the two Fabry-Perot resonators 13A, 13B is not negligible in particular at a high speed scanning frequency on the order of 1 MHz. It is therefore necessary to adjust the phase difference between the reversed scanning signals applied to the respective Fabry-Perot resonators 13A, 13B.

As may be seen from the above measured results, the laser frequency at the wavelength scanning laser light source 10 by the vernier result is roughly proportionate to the scanning signal voltage by the resonator length controller 15.

That is, with the vernier effect based wavelength scanning laser light source 10, the wavelength of the light is proportionate to the input voltage signal. Thus, if the linear input electrical signal is modulated with e.g. a serrated signal, serrated modulation becomes possible. In particular, since the wavelength may be operated by an electrical effect, that is, EO effect, there is no risk of hysteresis otherwise produced on mechanical modulation.

In case of using the wavelength scanning laser light source 10 as the optical coherence tomography device, if the maximum FSR (FSRmax) of the Fabry-Perot electro-optic modulator is given, from the sampling theorem, by the following equation (1):

$$FSRmax = c / \delta L \tag{1}$$

where $\delta L$ is the coherence length needed and c is the speed of light.

Since the two Fabry-Perot electro-optic modulators each suffer the loss of 7dB, the total loss is 14dB or more. The output power falls short of the saturation limit and is sensitive to an optical response characteristic of the Fabry-Perot electro-optic modulator.

Hence, it may be presumed that the cut-off (Srate-cut) of the frequency scanned by the laser light per unit time, that is, the frequency scanning rate (Srate) is given as the function of the maximum scanning range (FSRv), by the following equation (2):

$$\text{Srate-cut} = \text{FSRv} / \text{F}\delta t \tag{2}$$

where $\delta t$ (= F / FSR) is a life of a photon in the Fabry-Perot electro-optic modulator.

The cut-off (Srate-cut) of the frequency scanning rate (Srate) obtained as empirical value was 12 THz / $\mu$s.

On the other hand, the limit ($\delta$Llimit) of the coherence length ($\delta$L) is presumably given by the following equation (3):

$$\delta\text{Llimit} = c / \text{Srate } \delta t \tag{3}$$

From the above results, the coherence length ($\delta$L) and the frequency scanning rate (Srate) are presumably in a relationship of trade-off to each other. The limit ($\delta$Llimit-cutoff) of the coherence length ($\delta$L) in the cut-off (Srate-cut) of the frequency scanning rate (Srate) is given by the following equation (4):

$$\delta\text{Llimit-cutoff} = c\text{F} / \text{FSRv} \tag{4.}$$

Thus, if the coherence length ($\delta$L), frequency scanning rate (Srate) and the maximum scanning range (FSRv) are given by system requirements, the condition of the resonator, that is, the condition for the finesse (F) and the free spectral range (FSR) may be determined as follows:

That is, if the range that can be assumed by the finesse (F) is generally found from the above equation (3),

$$c\text{FSR} / \text{Srate } \delta L > F$$

and the range that can be assumed by the free spectral range (FSR) is

$$c / \delta L > \text{FSR}$$

In an actual device, the range that can be assumed by the finesse (F) is

$$c / \delta L > \text{FSR} > \text{SrateF}2 / \text{FSRv} / 2$$

It has been confirmed on the actual device that a satisfactory operation occurs in a range of the frequency scanning rate (Srate) of 1/2 to 2 times the cut-off (Srate-cut).

With the present wavelength scanning laser light source 10, if the scanning rate is slow, in particular if the frequency scanning rate (Srate) is lower than cut-off (Srate-cut) / 2, intensity noise was produced. This phenomenon is thought to be due to the mode competition noise. This mode competition noise may be decreased by high frequency superposition on the SOA to improve the characteristic in case of the slow scanning rate. In case the frequency scanning rate (Srate) is slower than cut-off (Srate-cut) / 2, the characteristic may be improved by enlarging the loop gain by a method shown in Figs.10 to 12, for example.

Also, with the vernier-effect based wavelength scanning laser light source 10, the laser wavelength is proportionate to the voltage of the scanning signal as a principle. Hence, the laser frequency is not perfectly proportionate to the scanning signal voltage. Thus, with the linear scanning signal, scanning with a temporally equal interval cannot be achieved with respect to the wavenumber (laser frequency $\times$ 2π ö speed of light). However, scanning with a temporally equal interval may be made with respect to the wavenumber by adjusting the waveform of the scanning signal.

That is, if the electrical signal is adjusted to a non-linear serrated shape, the vernier effect based wavelength scanning laser light source 10 is able to perform serrated modulation at an equal time interval with respect to the wavenumber. That is, if α is a constant, the wavelength for the voltage V is

$$\lambda = \alpha \cdot V + \lambda 0$$

On the other hand, the wavenumber of light K is given by

$$K = 2\pi / \lambda = 2\pi / (\alpha \cdot V + \lambda 0)$$

so that, to realize the wavenumber which is a function K(t) with respect to optional time, it is sufficient to set the function V(t) of the voltage with respect to time such that

$$V(t) = (2\pi / K(t) - \lambda 0) / \alpha$$

With the above-described wavelength scanning laser light source 10, the resonator length of at least one 13A of the two Fabry-Perot electro-optic modulators 13A, 13B that make up the band-pass filter is periodically varied within a preset range by the resonator length controller 15. This should result in periodically scanning the wavelength of the laser light taken outside by the optical coupler 14. It is noted that the waveform of the scanning signal which the resonator length controller affords to the Fabry-Perot resonator may be adjusted beforehand to calibrate the laser light source so that the wavenumber of the laser light taken outside by the optical coupler 14 will be linear with respect to time. By so doing, the laser light whose wavenumber is linear with respect to time may be obtained via the optical coupler 14.

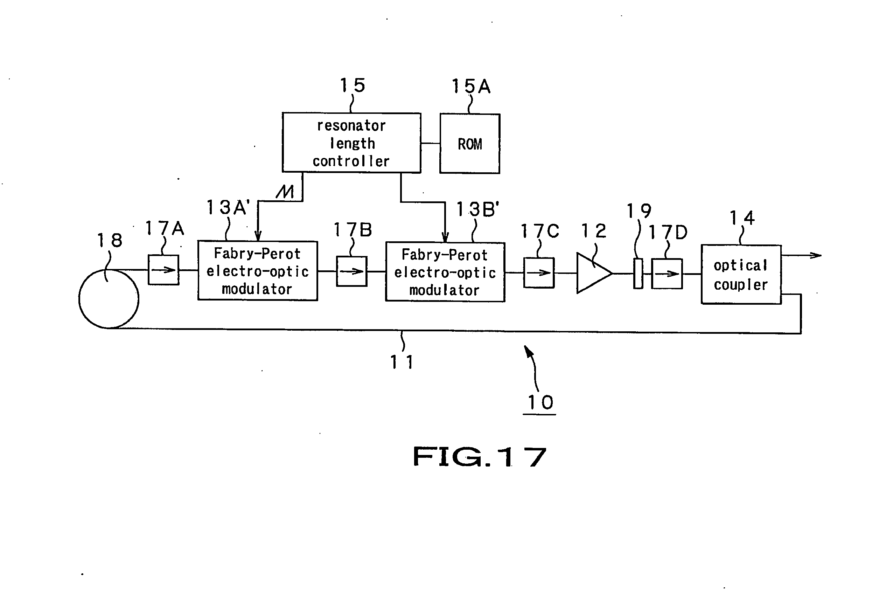

With the wavelength scanning laser light source 10 shown in Fig.17, calibration data for producing laser light, the wavenumber of which is linear with respect to time, is read out by the resonator length controller 15 from a ROM 15A to generate a waveform of the scanning signal. This arrangement enables the laser light, the wavenumber of which is linear with respect to time, to be obtained via the optical coupler 14.

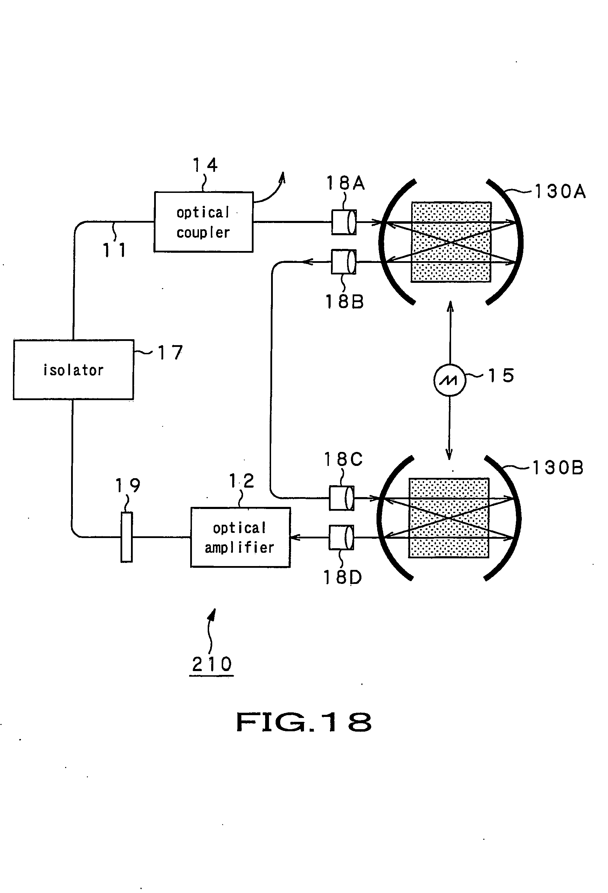

A wavelength scanning laser light source 210 remodeled from the wavelength scanning laser light source 10 is shown in Fig.18.

This wavelength scanning laser light source 210 includes two Fabry-Perot resonators 130A, 130B having the values of FSR (free spectral ranges) proximate to each other, a optical amplifier 12, an optical coupler 14 and a resonator length controller 15. The optical amplifier 12 has a gain bandwidth at its oscillation wavelength, and the optical coupler 14 is coupled to the optical amplifier 12 by an optical fiber via a polarizer 19 and an optical isolator 17. The resonator length controller 15 causes the resonator length of one of the two Fabry-Perot resonators 130A, 130B, here the Fabry-Perot resonator 130A, to be changed periodically within a preset range. The optical coupler 14 is fiber-coupled to the Fabry-Perot resonator 130A via a light incident side optical system 18A composed of a collimator and a convex lens. This light incident side optical system is designed to match to the resonator mode of the optical resonator. The Fabry-Perot resonator 130A is fiber-coupled to the Fabry-Perot resonator 130B via a light outgoing side optical system 18B composed of a collimator and a convex lens and via a light incident side optical system 18C composed of another collimator and another convex lens. The light outgoing side optical system and the light incident side optical system 18C are designed to match to the resonator mode of the optical resonator. The Fabry-Perot resonator 130B is fiber-coupled to the optical amplifier 12 via a light outgoing side optical system 18D having a collimator function. The wavelength scanning laser light source thus constitutes a fiber ring laser having an optical fiber loop that is to form a light path for laser oscillation. The function of the polarizer 19 is to determine the state of polarization of light propagated through the fiber loop that is to form the light path for laser oscillation. This polarizer basically may be inserted at any desired position on the loop.

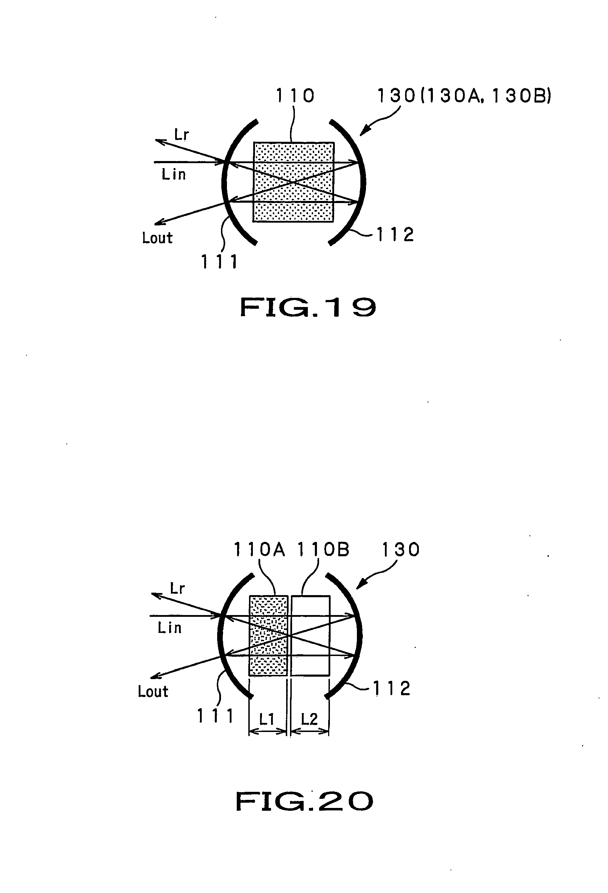

With this wavelength scanning laser light source 210, a ring resonation Fabry-Perot electro-optic modulator 130 is used for each of the two Fabry-Perot resonators 130A, 130B. With this ring resonation Fabry-Perot electro-optic modulator 130, ring-shaped resonation may be realized by setting the curvatures of concave mirrors 111, 112 so that the Fabry-Perot resonator having an electro-optic crystal 110 arranged therein will prove to be a confocal resonator as shown for example in Fig.19.

With the ring resonation Fabry-Perot electro-optic modulator 130, in which the curvatures of concave mirrors 111, 112 are set so that the Fabry-Perot resonator having an electro-optic crystal 110 will prove to be the confocal resonator, the direct reflected light Lr of the incident light Lin is not returned to the light incident side. Hence, the optical isolator may be dispensed with.

With the wavelength scanning laser light source 210, the ring resonation Fabry-Perot electro-optic modulator 130, in which the direct reflected light Lr of the incident light Lin is not returned to the light incident side, is used as each of the two Fabry-Perot resonators 130A, 130B having the proximate values of FSR arranged therein, as set forth above. Hence, the optical isolators at the input and output sides of the Fabry-Perot resonators 130A, 130B may be dispensed with. Thus, the optical isolator 17 is provided only at the input side of the optical amplifier 12.

That is, with the present wavelength scanning laser light source 210, the effect of reflection is only low because the confocal ring resonation Fabry-Perot electro-optic resonator, in which the direct reflected light Lr of the incident light Lin is not returned to the light incident side, is used as each of the two Fabry-Perot resonators 130A, 130B having the proximate values of FSR, as set forth above. However, the optical isolator 17 is inserted only on an input side of the optical amplifier 12 to determine the direction of oscillation of the ring laser, that is, to determine whether the ring laser oscillates clockwise or counterclockwise. This optical isolator 17 is not aimed to reduce the effect of reflection from the Fabry-Perot electro-optic modulator that is prone to reflection, and hence is only required to afford the gain difference as to whether the direction of laser oscillation is clockwise or counterclockwise. In addition, the optical isolator 17 may be inserted at any desired position within the loop.

With this wavelength scanning laser light source 210, the two Fabry-Perot resonators 130A, 130B having the proximate values of FSR, operate as wavelength selection filters. At least one of the two Fabry-Perot resonators 130A, 130B, here the Fabry-Perot resonator 130A is configured for varying the selection wavelength by varying its resonator length by the resonator length controller 15.

The two Fabry-Perot resonators 130A, 130B, having the proximate values of FSR, operate as a bandpass filter having a narrow band wavelength selection characteristic which is capable of varying the selection waveform by the vernier effect by varying the resonator length of one 130A of the two Fabry-Perot resonators 130A, 130B having the proximate values of FSR.

With the wavelength scanning laser light source 210, light propagated through the bandpass filter and amplified by the optical amplifier 12 is returned via the optical fiber loop 11 and is thereby set into oscillations. The bandpass filter is constituted by the two Fabry-Perot resonators 130A, 130B provided within the optical fiber loop 11 and having the proximate FSRs. With this wavelength scanning laser light source 210, the oscillation wavelength is periodically changed by the resonator length of the Fabry-Perot resonator 130A periodically changed within a preset range by the resonator length controller 15 to cause periodic changes in the wavelength of laser light taken outside via the optical coupler 14. Although the optical coupler 14 adapted for taking out the laser light from the optical fiber loop 11 is provided in rear of the optical amplifier 12, it may also be provided ahead of the optical amplifier 12 or intermediate between the two Fabry-Perot resonators 130A, 130B.

With the wavelength scanning laser light source 210 set forth above, it is possible to enlarge the resonator length of the optical fiber loop 11. If the resonator length of the optical fiber loop 11 is 1000m, for example, the longitudinal mode spacing of the laser in its entirety may be narrowed to, for example, ca. 200 kHz. Hence, the longitudinal mode spacing of the entire laser may be made sufficiently narrower than the bandwidth of each individual mode of each of the Fabry-Perot resonators 130A, 130B (FSR / finesse, for example, 2.5 GHz / 50 = 50 MHz). The effect of mode hop may thus be removed without changing the resonator length. Hence, even granting that the oscillation is not strictly a single-mode oscillation, pseudo-continuous wavelength variations may be achieved by solely changing the selection wavelength of the band-pass filter.

With the present wavelength scanning laser light source 210, the wavelength of the light source having a narrow-band spectrum may be continuously scanned over a wide bandwidth at a high speed. Moreover, since the confocal ring resonation Fabry-Perot electro-optic modulator 130 is used as each of the two Fabry-Perot resonators 130A, 130B, the optical isolators 17 at the input and the output of each of the two Fabry-Perot resonators 130A, 130B may be dispensed with, thus suppressing the noise from increasing due to light loss at the optical isolator.

It is observed that length adjustment may be made by polishing, and the resonator length may be accurately controlled by temperature control with the waveguide Fabry-Perot electro-optic modulator. Specifically, the absolute value of the resonator length may be controlled within 1 ppm in a desired range subject to temperature control. Hence, with the use of a Fabry-Perot electro-optic modulator with FSR = 2.5 GHz and another Fabry-Perot electro-optic modulator different in FSR only by 1/4000, it is possible to readily implement a wavelength selection device with an FSR increased by a factor of 4000, or 10 THz, by the vernier effect. However, with the spatial Fabry-Perot electro-optic modulator employing a set of concave mirrors, it is difficult to adjust the length to high accuracy by polishing. It is therefore necessary to adjust the concave mirror position to an accuracy of a few $\delta$ms and to effect temperature adjustment of the two optical resonators to high accuracy.

It is observed that the FSR (free spectral range) of the confocal ring resonation Fabry-Perot electro-optic modulator 130 is

$$FSR = c/ (4ng \cdot L)$$

where ng is a group refractive index, c is speed of light in vacuum and L a resonator length.

With lithium niobate LN (LiNbO$_3$) preferentially used for electro-optic modulation, the group refractive index nog of ordinary light and that neg of extraordinary light are both of the order of 2. However, the two differ from each other by 4%. Hence, the FSR of the confocal ring resonation Fabry-Perot electro-optic modulator 130 adapted for modulating ordinary light by an electro-optic crystal (LN) 110 is

FSRI = c / (4nog·L), while that of the confocal ring resonation Fabry-Perot electro-optic modulator 130 adapted for modulating extraordinary light by an electro-optic crystal (LN) 110 is

$$FSR2 = c / (4neg \cdot L).$$

Referring to Fig.20, there is shown a confocal ring resonation Fabry-Perot electro-optic modulator 130, in which an electro-optic crystal (LN) 110A with a length L1 and an electro-optic crystal (LN) 110B with a length L2 are introduced and arranged so that their C-axes are at right angles to each other. The electro-optic crystal (LN) 110A with the length L1 modulates the ordinary light and the electro-optic crystal (LN) 110B with the length L2 modulates the extraordinary light. The FSR of the confocal ring resonation Fabry-Perot electro-optic modulator 130 is given by

$$FSR1 = c / (4nog \cdot L1 + 4neg \cdot L2).$$

The FSR of the confocal ring resonation Fabry-Perot electro-optic modulator 130, in which the electro-optic crystal (LN) 110A with the length L1 modulates the extraordinary light and the electro-optic crystal (LN) 110B with the length L2 modulates the ordinary light, is given by

$$FSR2 = c / (4neg \cdot L1 + 4nog \cdot L2).$$

If L1 = L2, FSR1 = FSR2, and the difference between FSR1 and FSR2 may be finely set by the difference between the lengths L1 of the electro-optic crystal (LN) 110A and the length L2 of the electro-optic crystal (LN) 110B.

In calculating the FSR of the ring resonation Fabry-Perot electro-optic modulator 130, the spatial part is discounted and the crystal length is assumed to be equal to the resonator length for illustrating the principle.

In case two crystals are inserted into the sole Fabry-Perot resonator, the entire resonator length is co-owned. As regards the vernier effect, the ratio of the difference between the two values of FSR and the FSR is crucial. Hence, the absolute value of the length is intrinsically not crucial and the difference between the lengths of two crystals is crucial. The difference between the lengths of two crystals may be adjusted by polishing. It is possible to discount slight deviations in the externally mounted mirrors.

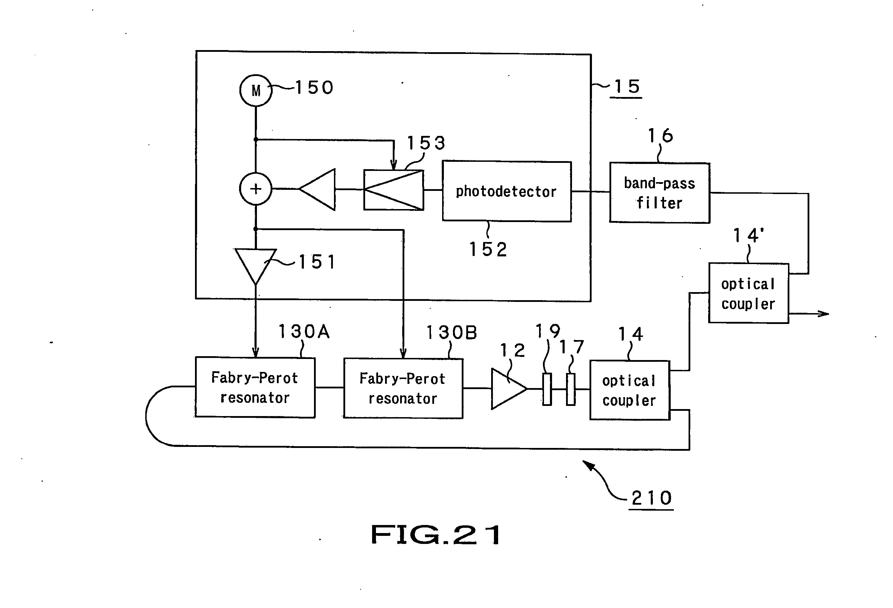

With the wavelength scanning laser light source 210, the resonator length of one 130A of the two Fabry-Perot resonators 130A, 130B that make up the band-pass filter is periodically changed within a preset range by the resonator length controller 15 in order to periodically scan the wavelength of the laser light taken outside by the optical coupler 14. Alternatively, a serrated wave signal generated by a serrated save generator 150 as a periodic scanning signal may directly be delivered to the Fabry-Perot resonator 130B, while the same signal is directly supplied to the Fabry-Perot resonator 11A via an inverting amplifier 151, as with the resonator length controller 15 of the wavelength scanning laser light source 210 shown for example in Fig.21. A scanning signal reversed from a scanning signal afforded to one of the two Fabry-Perot electro-optic modulators 130A, 130B may be afforded to the other so that the resonator lengths of the respective Fabry-Perot resonators are varied in anti-phase. By so doing, the modulation voltages may be halved. In addition, phase changes produced on applying the voltage to the electro-optic modulator within the Fabry-Perot resonator may be canceled out to enable wavelength scanning in stability to high accuracy.

In the wavelength scanning laser light source 210, the center wavelength is highly sensitive to temperature difference of the respective Fabry-Perot electro-optic modulators. Thus, in the resonator length controller 15 of the wavelength scanning laser light source 210 shown in Fig.21, the laser light taken outside via the optical coupler 14, that is, part of the light propagated through the band filter and which has a center wavelength as a passband, is routed from an optical coupler 14' via a band-pass filter 16 to a photodetector 152. After the light is detected by the photodetector 152, the phase difference between the detection timing and a periodic scanning signal is detected by a lock-in amplifier 153. A control signal is superposed on the periodic signal to provide for a constant value of the phase difference and the so generated signal is fed back to control the center wavelength. This provides for modulation at a constant wavelength at all times.

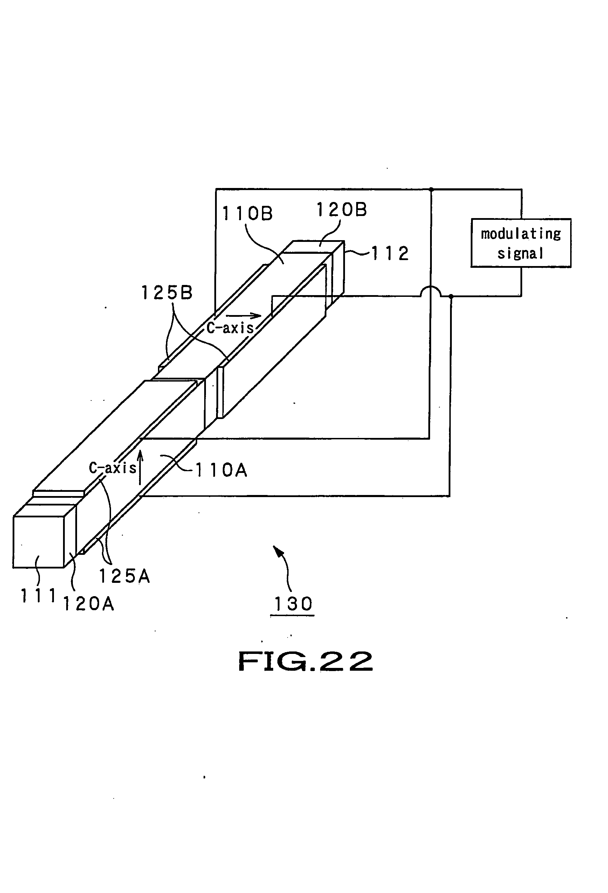

It is assumed that an electro-optic crystal (LN) 110A with the length L1 and an electro-optic crystal (LN) 110B with the length L2 are inserted in the confocal ring resonation Fabry-Perot electro-optic modulator 130, and these crystals are arranged so that their C-axes will be at right angles to each other as shown in Fig.20. It is also assumed that electrodes 125A, 125B of the two electro-optic crystals (LN) 110A, 110B are connected in parallel with one another as shown in Fig.22, and the modulation signal is afforded for modulation. If a voltage is applied in opposite directions with respect to the C-axes of the crystals, the optical distance is varied relative to the direction of light polarization.

Thus, with electro-optic constant of ν33, ν31, crystal lengths of L1, L2 and the crystal thickness of d, the change in the optical distance produced for each pass through the crystal is (ν31·L1 - ν33·L2)·V/d and (ν33·L1 -ν31·L2)·V/d for the polarized light on the FSR1 side and for that on the FSR2 side, respectively. Hence, the optical distance is modulated by the difference between the two, or by (ν31-ν33)·(L1 + L2)·V/d.

That is, if two Fabry-Perot electro-optic modulators are used, there is no necessity of providing the inverting amplifier 151 in case the electrodes may be interchanged.

In selecting the crystal, such a crystal having a larger difference between ν33 and ν31 is to be selected. In case of LN or LT, congruent LN/LT is preferred to stoichiometric LN/LT in view of ease in modulation or fabrication.

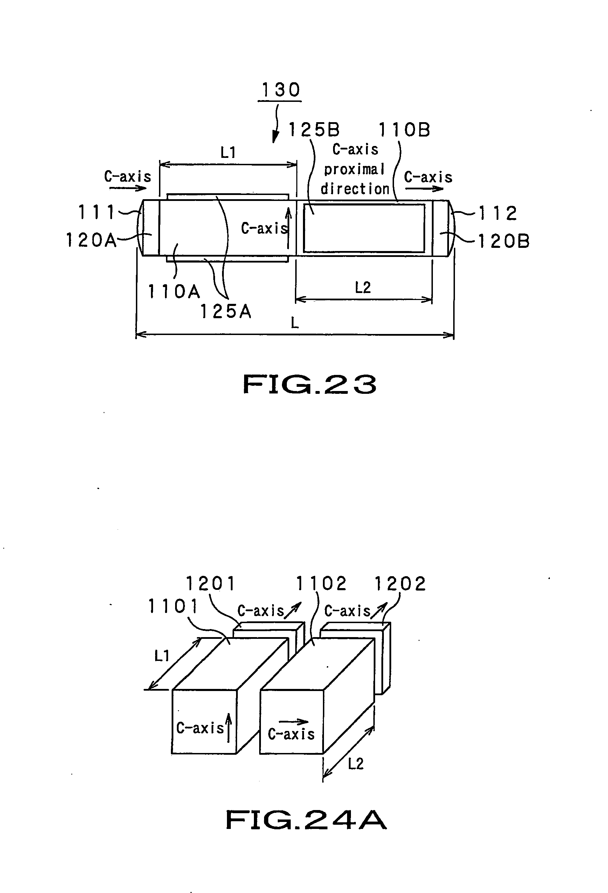

A confocal ring resonation Fabry-Perot electro-optic modulator 11, composed of a sole Fabry-Perot resonator and two electro-optic crystals mounted therein, may be fabricated as a monolithic Fabry-Perot electro-optic modulator configured as shown for example in Fig.23.

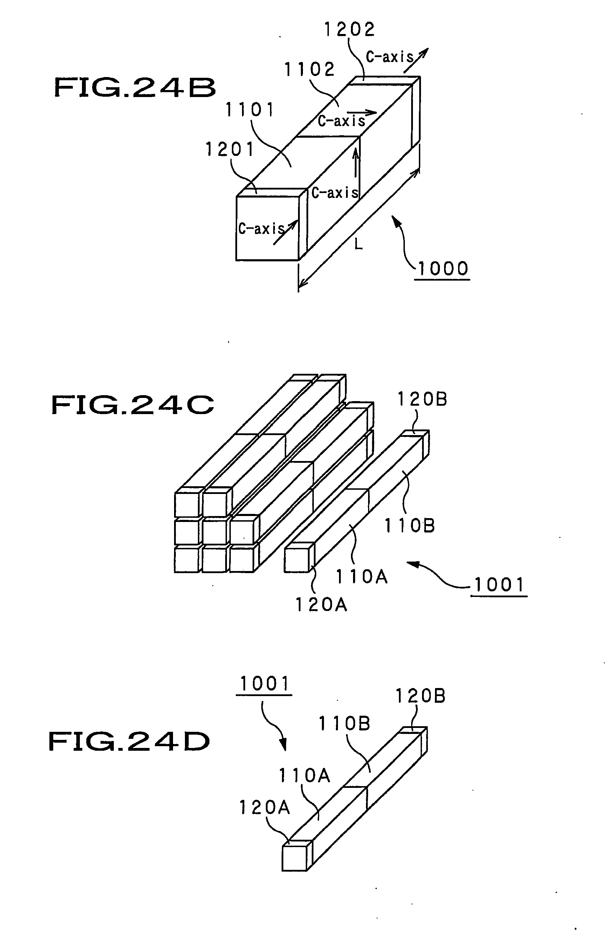

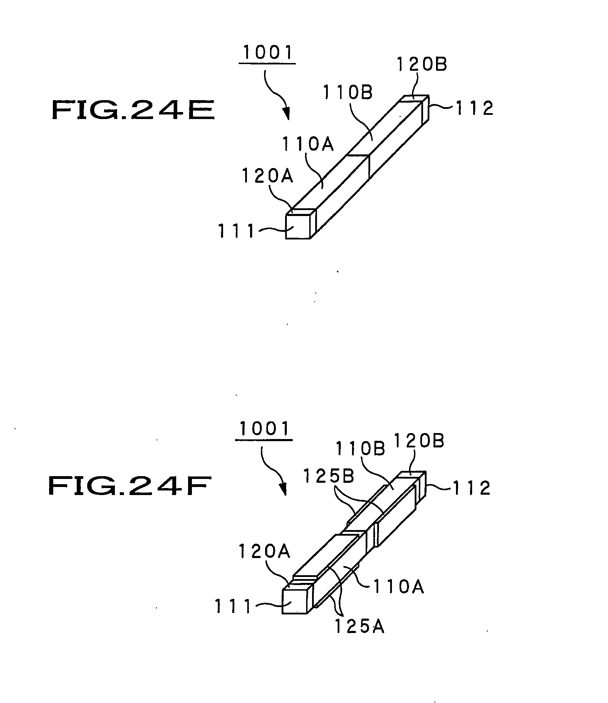

First, referring to Fig.24A, elongated crystals (LN) 1101, 1102 having C-axes at right angles to the direction of light and to each other, and thin crystals 1201, 1202 having their C-axes extending in the direction of light, were fabricated. End faces of these crystals running at right angles to the direction of light, were polished and the length of the electro-optic crystal (LN) 1101 was adjusted to L1, while that of the electro-optic crystal (LN) 1102 was adjusted to L2.

Then, referring to Fig.24B, the electro-optic crystal (LN1) 1101 with the length L1 and the electro-optic crystal (LN1) 1102 with the length L2 were bonded together with the respective C-axes at right angles to each other, using an optical contact or adhesive the refractive index of which is matched to that of the crystals. Further, thin crystals 1201, 1202, the C-axes of which are along the direction of light, were bonded to both ends of the crystals. In this manner, a bonded product 1000 with a total length equal to L was prepared. It is observed that the crystals 1201, 1202 are media isotropic with respect to light.

The bonded product 1000 was diced to form a substrate 1001 of a monolithic Fabry-Perot electro-optic modulator 130 as shown in Fig.24C.

With the substrate 1001 formed by the electro-optic crystals (LN) 110A, 110B, and both end crystals 120A, 120B thus prepared, the end crystals 120A, 120B of the substrate 1001 were polished to form convex surfaces at a curvature R to yield a confocal resonator.

Then, as shown in Fig.24E, confocal concave mirrors 111, 112 on opposite sides of a Fabry-Perot resonator were then formed by coating on evaporation on both end faces of the substrate 1001 which were already polished to the convex surfaces of the curvature R as described above. The end face of the crystal 120A was coated by less than 100%, while that of the opposite side crystal 120B was coated by 100% to form concave mirrors 111, 112. With the overall length of L, the curvature R is given by

$$R = (no / ne-1)·(L1 + L2)/2 + L$$

Then, electrodes 125A, 125B are formed by evaporation on the lateral sides extending at right angles to the C-axes of the electro-optic crystals (LN) 110A, 110B of the substrate 1001, on the end faces of which have been formed the concave mirrors 111, 112. This completes a monolithic Fabry-Perot electro-optic modulator 130 shown in Fig.23.

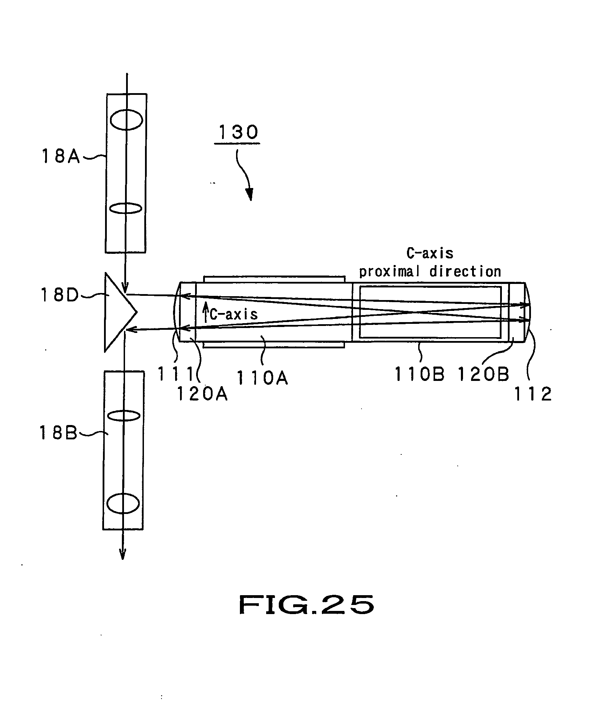

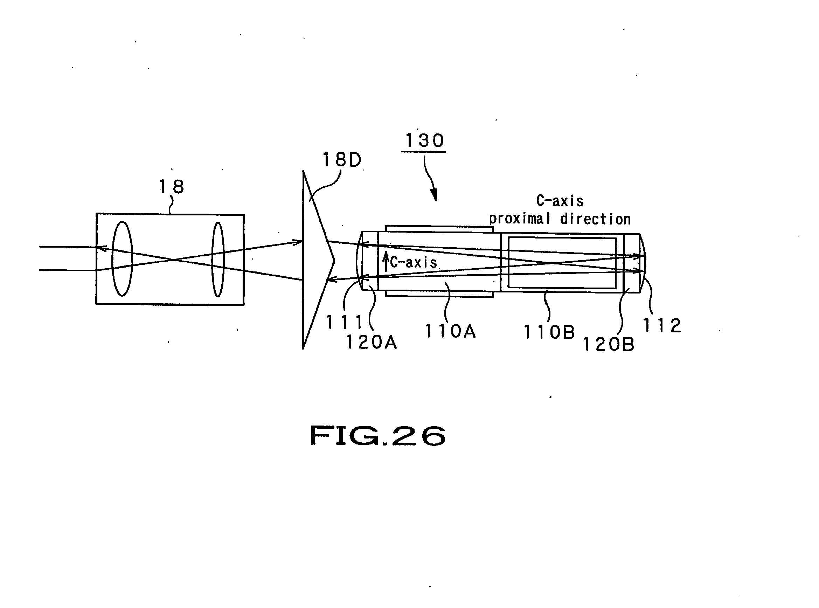

It is observed that, since the crystal size is smaller with the monolithic Fabry-Perot electro-optic modulator 130 than with the collimator, it is difficult to array two collimators side-by-side as incident/outgoing optical systems 18A, 18B. Thus, a prism-shaped mirror 18C is arranged on an input side as shown in Fig.25 to provide an incident/outgoing optical system 16A, 16B, or a fiber is loaded on a two-core ferule and a prism 18D is used to couple the fiber loaded on the ferule to the ring resonator to provide an incident/outgoing optical system 18 as shown in Fig.26.

It is also observed that the confocal ring resonation Fabry-Perot electro-optic modulator 130 may be used as a bidirectional electro-optic modulator. Hence, a bidirectional optical amplifier 12' such as SOA, capable of light amplification in both directions without regard to the polarized light component or its direction such as a wavelength scanning laser light source 210', is used as the optical amplifier 12 of the wavelength scanning laser light source 210 shown in Fig.18. A total reflection mirror14A and a half mirror 14B are provided in place of the optical coupler 14. In addition, the two Fabry-Perot resonators 130A, 130B having proximate values of FSR are constituted by the confocal ring resonation Fabry-Perot electro-optic modulators 130 operating as bidirectional electro-optic modulators. Hence, light may be amplified twice in the course of a round trip on the optical fiber loop.

With the wavelength scanning laser light source 210', the light radiated from the Fabry-Perot resonator 130A via a light incident/outgoing optical system 18A' is totally reflected by a total reflection mirror 14A so as to fall on the Fabry-Perot resonator 130A via the light incident/outgoing optical system 18A'. The light outgoing from the Fabry-Perot resonator 130A via a light incident/outgoing optical system 18B' is incident on the other Fabry-Perot resonator 130B via the light incident/outgoing optical system 18C'. The light outgoing from the Fabry-Perot resonator 130B via a light incident/ outgoing optical system 18D' is amplified by a bidirectional optical amplifier 12'. The preset polarized light component taken out via a polarizer 19 from the light amplified by the bidirectional optical amplifier 12' is reflected by the half mirror 14B so as to be incident on the bidirectional optical amplifier 12' via the polarizer 19. The preset polarized light component propagated through the polarizer 19 is amplified by the bidirectional optical amplifier 12' to fall on the Fabry-Perot resonator 130B via the light incident/outgoing optical system 18B'. The light radiated from the Fabry-Perot resonator 130B via the incident/outgoing optical system falls on the Fabry-Perot resonator 130A via the light incident/outgoing optical system. The laser light is taken outside by the half mirror 14B.

With the wavelength scanning laser light source 210', light has two passes through the Fabry-Perot resonator 130A of FSR1 and two passes through the Fabry-Perot resonator 130B of FSR2 each composed of a confocal ring resonator Fabry-Perot electro-optic modulator 130, during each round trip of the light through the optical fiber loop 11. Hence, the light filter may be improved in resolution based on the vernier effect, resulting in more intense wavelength selection, narrow line width and a longer coherence length. Meanwhile, the bidirectional optical amplifier 12 may be inserted at any desired position in the loop.

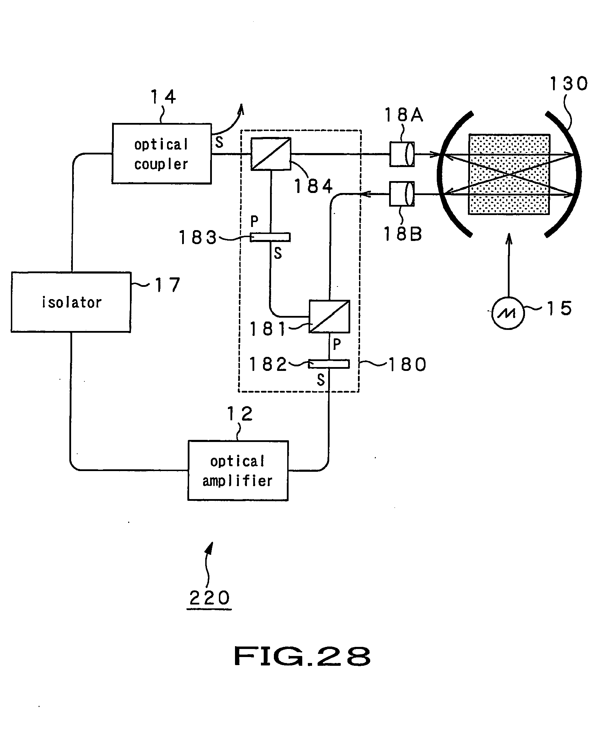

With the confocal ring resonation Fabry-Perot electro-optic modulator 130, the FSR differs with the direction of polarization, and the electro-optic modulation is applied strongly with respect to the light polarization along the C-axis direction. Thus, by providing an optical system 180 in which polarized components are separated and synthesized as in a wavelength scanning laser light source 220 shown in Fig.28, it is possible for the sole confocal ring resonation Fabry-Perot electro-optic modulator 130 to operate as the two Fabry-Perot resonators 130A, 130B having proximate values of FSR as set forth above.

That is, a wavelength scanning laser light source 220 shown in Fig.28 is remodeled from the wavelength scanning laser light source 210 shown in Fig.18. The wavelength scanning laser light source includes a confocal ring resonation Fabry-Perot electro-optic modulator 130 provided in the optical fiber loop, an optical amplifier 12 having a gain bandwidth at the oscillation wavelength, an optical coupler 14, a resonator length controller 15 and an optical system 180. The optical coupler 14 is fiber-connected to the optical amplifier 12 via an optical isolator 17. The resonator length controller 15 causes the relative resonator lengths of the P-polarized light and the S-polarized light of the confocal ring resonation Fabry-Perot electro-optic modulator 130 to be periodically changed within a preset range. The optical system 180 is provided between the optical amplifier 12 and the optical coupler 14 on one hand and the light incident optical system 18A and the light outgoing optical system 18B on the other and it separates and synthesizes the polarized light component. The optical system 180 includes a first PBS coupler 181, a first polarized light converter 182, a second polarized light converter183 and a second PBS coupler 184. The outgoing light from the confocal ring resonation Fabry-Perot electro-optic modulator 130 is incident via the light outgoing optical system 18B on the first PBS coupler 181. The light of the P-polarized component separated by the first PBS coupler 181 from the outgoing light falls on the first polarized light converter 182. The light of the S-polarized light separated by the PBS coupler 181 from the outgoing light is incident on the second polarized light converter 183, and the light of the S-polarized component is incident via the optical coupler 14 on the second PBS coupler 184.

In this optical system 180, the first PBS coupler 181 separates the light incident via the outgoing side optical system 16B into the P-polarized light and the S-polarized light from the ring resonation Fabry-Perot electro-optic modulator 130. The light of the P-polarized component separated from the outgoing light by the first PBS coupler 181 falls on the first polarized light converter 182, while the light of the S-polarized component falls on the second polarized light converter183. The first polarized light converter 182 rotates the light of the P-polarized light component separated by the first PBS coupler 181 from the outgoing light through 90° to transform it into light of the S-polarized component. The light of the S-polarized component transformed by the first polarized light converter 182 falls on the optical amplifier 12.

With the present wavelength scanning laser light source 220, the light of the S-polarized component, which is incident via the first polarized light converter 182, is amplified by the optical amplifier 12 that has a gain bandwidth at the oscillation wavelength, and falls via the optical isolator 17 and the optical coupler 14 on the second PBS coupler 184.

The second polarized light converter183 rotates the direction of polarization of the light of the S-polarized component separated from the outgoing light by the first PBS coupler 181 through 90° to transform it into light of the P-polarized component. The light of the P-polarized component transformed by the second polarized light converter 183 falls on the second PBS coupler 184.

The first and second polarized light converters 182, 183 may be formed by Faraday devices or wave plates. The direction of polarization may be rotated through 90° by properly selecting the PM axis at a point of connection of the PM fiber to the optical component.

The second PBS coupler 184 synthesizes the light of the S-polarized light, incident thereon via the optical coupler 14, and the light of the P-polarized light, incident thereon via the second polarized light converter 183.

The light obtained by synthesis by the second PBS coupler 184 is incident via the light incident optical system 18A on the confocal ring resonation Fabry-Perot electro-optic modulator 130 as incident light.

The first and second PBS couplers 181, 184 may be replaced by circulators.

In the present wavelength scanning laser light source 220, the confocal ring resonation Fabry-Perot electro-optic modulator 130 differs in FSR depending on the direction of light polarization. Moreover, the polarized light along the C-axis is subjected strongly to electro-optic modulation. The confocal ring resonation Fabry-Perot electro-optic modulator thus operates as two Fabry-Perot resonators having different degrees of modulation for proximate values of FSRp and FSRs with respect to the light of the P-polarized component and the light of the S-polarized component that make up the synthesized light.

In the present wavelength scanning laser light source 220, the confocal ring resonation Fabry-Perot electro-optic modulator 130 operating as two Fabry-Perot resonators having different modulation degrees for proximate FSRp and FSRs operates as a wavelength selection filter. Specifically, by varying the relative resonator lengths for P-polarized light and S-polarized light, the confocal ring resonation Fabry-Perot electro-optic modulator operates as a band-pass filter having narrow band wavelength selection characteristic and which is capable of varying the selection wavelength by the vernier effect.

With the present wavelength scanning laser light source 220, the light propagated through a bandpass filter by the sole confocal ring resonation Fabry-Perot electro-optic modulator 130 provided within the optical fiber loop and having the proximate FSRp and FSRs, that is, the outgoing light of the ring resonation Fabry-Perot electro-optic modulator 130 is separated by the first PBS coupler 181 into light of the P-polarized component and light of the S-polarized component. The light of the P-polarized component separated from the outgoing light by the first PBS coupler 181 is transformed by the first polarized light converter 182 into light of the S-polarized component and amplified by the optical amplifier 12 so as to be fed back by the fiber loop. The light of the S-polarized component separated from the outgoing light by the first PBS coupler 181 is transformed into light of the P-polarized component by the second polarized light converter 183 and fed back, thus causing oscillation. With the present wavelength scanning laser light source 220, the oscillation wavelength is periodically varied by periodically changing the relative resonator lengths for the P-polarized light and the S-polarized light of the sole confocal ring resonation Fabry-Perot electro-optic modulator 130 by the resonator length controller 15. The wavelength of the laser light taken outside via the optical coupler 14, may thus be periodically changed.

The wavelength scanning laser light source 220 uses a sole confocal ring resonation Fabry-Perot electro-optic modulator 11 in which the direct reflected light Lr of the incident light Lin is not returned to the light incident side. With the wavelength scanning laser light source, the wavelength of the light source having a spectrum of a narrow bandwidth capable of generating monochromatic light of extremely low noise may be scanned over a wide range continuously at a high speed.

In the case of two Fabry-Perot resonators,

$$FSR1 = c / (4ng \cdot L1)$$

$$FSR2 = c / (4ng \cdot L2)$$

so that, approximately,

(FSR1 - FSR2) / FSR = (L2 - L1) / L. Hence, to provide for a difference of 1/1000, the difference between L1 and L2 needs to be 1/1000. It is thus necessary to set the lengths to an accuracy of 1/1000 of L and to maintain the difference of FSR based on the temperature difference. In the case of a sole Fabry-Perot resonator, since

$$FSR1 = c / (4nog \cdot L1 + 4neg \cdot L2)$$

$$FSR2 = c / (4neg \cdot L1 + 4nog \cdot L2)$$

so that, approximately,

$$(FSR1 - FSR2)/FSR = (L2 - L1)/L\ (nog - neg)/ng$$

Since (nog - neg) / ng is approximately 0.04, it is sufficient to set the difference in length to 1/40 of L, even if the polishing accuracy remains the same. Since the polishing accuracy is of the order of 1/1000, the difference in FSR may be realized to the accuracy of 1/1000 ± 1/40000 solely by polishing. Two crystals are proximate to each other and hence can be thermally bonded together. Precision in the FSR difference obtained on polishing may be maintained in the absence of temperature control, and hence the temperature control for maintaining the FSR difference constant, such as is used in a waveguide modulator, may be dispensed with. As a result, two temperature controllers may be dispensed with, resulting in corresponding cost reduction.

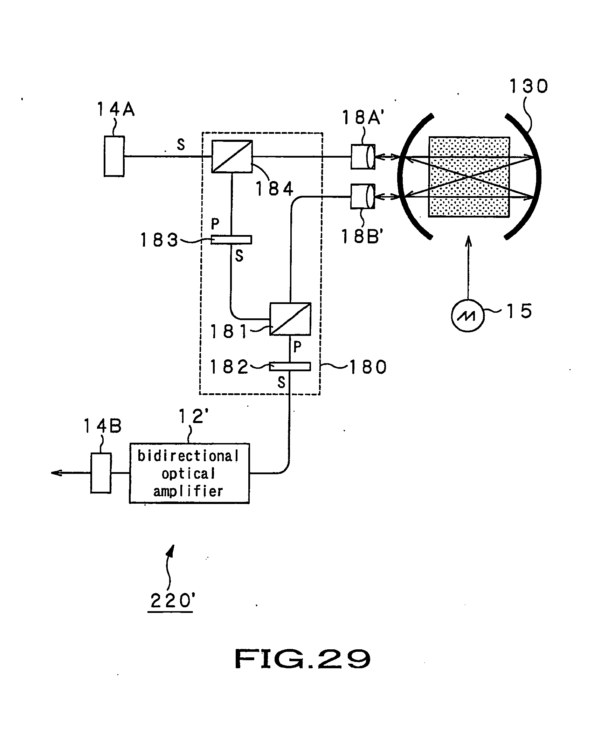

Moreover, the confocal ring resonation Fabry-Perot electro-optic modulator 130 may be used as a bidirectional electro-optic modulator as set forth above. Hence, a bidirectional optical amplifier 12' such as SOA, capable of amplifying light regardless of which of the polarized components or which direction polarized component is to be amplified, may be used as the optical amplifier 12 in the wavelength scanning laser light source 220 shown in Fig.28, as exemplified by the wavelength scanning laser light source 220' shown in Fig.29. Moreover, a mirror of total reflection 14A and a half mirror 14B may be provided in place of the optical coupler 14, and a sole confocal ring resonation Fabry-Perot electro-optic modulator 11 operating as two Fabry-Perot resonators different in the modulation degrees for the proximate FSRp and FSRs as set forth above, may be used for bidirectional operations.

With the present wavelength scanning laser light source 220', laser oscillations occur as follows: The light taken outside via the light incident/outgoing optical system 18A' from the confocal ring resonation Fabry-Perot electro-optic modulator 130 is propagated through the second PBS coupler 184 to fall on the mirror of total reflection 14A. The light reflected by the mirror of total reflection 14A is passed through the second PBS coupler 184 to fall on the confocal ring resonation Fabry-Perot electro-optic modulator 11 via the light incident/outgoing optical system 18A'. The light taken outside via the light incident/outgoing optical system 18B' from the confocal ring resonation Fabry-Perot electro-optic modulator 11 falls on the bidirectional optical amplifier 12' via the first PBS coupler 181 and the first polarized light converter 182. The light amplified by the bidirectional optical amplifier 12' falls on the half mirror 14B. The light reflected by the half mirror 14B falls on the bidirectional optical amplifier 12'. The light amplified by the bidirectional optical amplifier 12' falls from the light incident/outgoing optical system 18B' via the first polarized light converter 182 and first PBS coupler 181 on the confocal ring resonation Fabry-Perot electro-optic modulator 130. This sets light into laser oscillations so that the laser light is taken outside via the half mirror 14B.

With the wavelength scanning laser light source 220', the sole confocal ring resonation Fabry-Perot electro-optic modulator 130 operating as two Fabry-Perot resonators having different modulation degrees for the proximate FSRp and FSRs is bidirectionally used as set forth above. Hence, as the light performs its round trip through the fiber loop, it may have two passes through a Fabry-Perot resonator of FSR1 and two passes through a Fabry-Perot resonator of FSR2. These two Fabri-Perot resonators are formed by a confocal ring resonation Fabry-Perot electro-optic modulator 130. It is thus possible to improve the resolution of the optical filter by a vernier effect and to provide for sharper wavelength selection, narrower line width and longer coherence length. It is observed that the bidirectional optical amplifier 12 may be provided at any desired position on the loop. If the bidirectional optical amplifier 12 is provided ahead or in rear or the second polarized light converter 183, light may be amplified twice during the time the light performs round trip through the fiber loop, that is, during the time the light has two passes through each of the Fabry-Perot resonator of FRS1 and the Fabry-Perot resonator of FSR2 of the confocal ring resonation Fabry-Perot electro-optic modulator 130, thus improving the SN ratio.

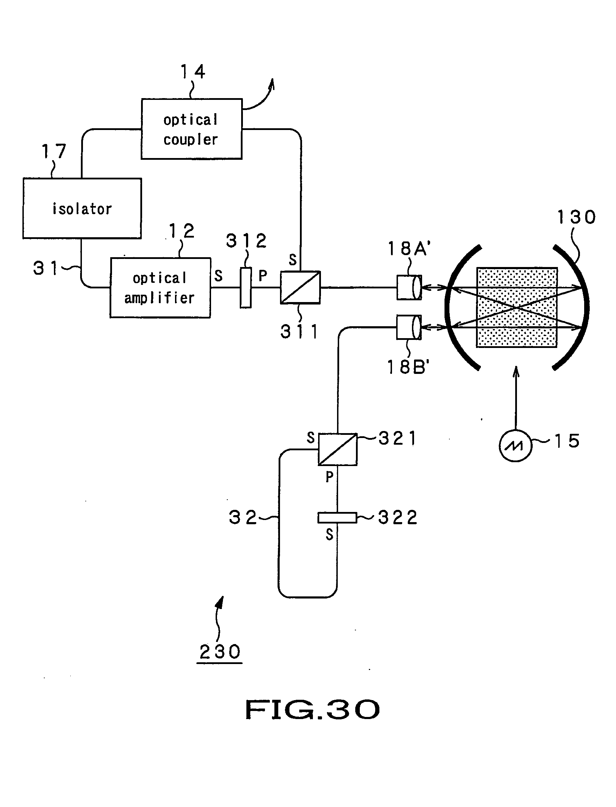

The confocal ring resonation Fabry-Perot electro-optic modulator 130 may be used as a bidirectional electro-optic modulator as set forth above. Thus, as in a wavelength scanning laser light source 230 shown for example in Fig.30, light may be caused to be incident on or radiated from the sole confocal ring resonation Fabry-Perot electro-optic modulator 130 having proximate values of FSRp and FSRs via the light incident/outgoing optical system 18A' and a light incident/outgoing optical system 36B. The light radiated via the light incident/outgoing optical system 18A' may be made to be incident via a first optical fiber loop 331 on the light incident/outgoing optical system 18A', while the light radiated via the light incident/outgoing optical system 18B' may be made to be incident on the light incident/outgoing optical system 18B' via a second optical fiber loop 332.

With the wavelength scanning laser light source 230, the first optical fiber loop 331 is formulated as follows: The light radiated via the light incident/outgoing optical system 18A' from the confocal ring resonation Fabry-Perot electro-optic modulator 11 is separated by the first PBS coupler 311 into light of the P-polarized component and light of the S-polarized component. The light of the P-polarized component thus separated has its direction of polarization rotated through 90° and is thereby transformed by a first polarized light converter 312 into light of the S-polarized component. The so transformed light falls on the optical amplifier 12. The light of the S-polarized light amplified by the optical amplifier 12 is returned via the optical isolator 17 and the optical coupler 14 to a first PBS coupler 311. The light from the first PBS coupler 311 is incident on the confocal ring resonation Fabry-Perot electro-optic modulator 130 via the light incident/outgoing optical system 18A'. The second optical fiber loop 332 is formulated as follows: The light radiated from the confocal ring resonation Fabry-Perot electro-optic modulator 130 via the incident/outgoing optical system 18B' is separated by a second PBS coupler 321 into light of the P-polarized light and light of the S-polarized light. The direction of light polarization of the light of the P-polarized component and that of the light of the S-polarized component are rotated through 90° by a second polarized light converter 322 so that the two are synthesized by the second PBS coupler 321. The light synthesized by the second PBS coupler 321 is incident via the light incident/outgoing optical system 18B' on the confocal ring resonation Fabry-Perot electro-optic modulator 130.

Thus, with the present wavelength scanning laser light source 230, the optical fiber loop is formulated as follows: The light radiated via the light incident/outgoing optical system 18A' of the confocal ring resonation Fabry-Perot electro-optic modulator 130 is amplified by the optical amplifier 12 in the first optical fiber loop 331 to fall via the light incident/outgoing optical system 18A' on the confocal ring resonation Fabry-Perot electro-optic modulator 130. The light radiated from the confocal ring resonation Fabry-Perot electro-optic modulator 130 via the light incident/outgoing optical system 18B' has its direction of light polarization rotated through 90° by the second optical fiber loop 332 so as to be incident via the light incident/outgoing optical system 18B' on the confocal ring resonation Fabry-Perot electro-optic modulator 130.

With the present wavelength scanning laser light source 230, the sole confocal ring resonation Fabry-Perot electro-optic modulator 130, in which the direct reflected light Lr of the incident light Lin is not returned to the incident side, is used as a bidirectional electro-optic modulator. The wavelength of a light source having a spectrum of a narrow bandwidth and which is capable of generating monochromatic light of extremely low noise may be scanned over a wide range continuously at a high speed.

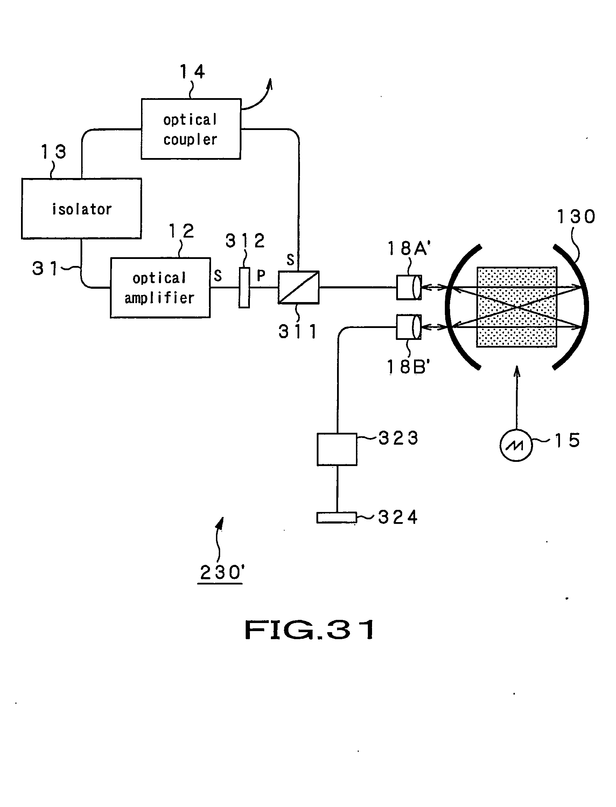

It is observed that the above-described first optical fiber loop, in which the light radiated from the confocal ring resonation Fabry-Perot electro-optic modulator 130 via the light incident/outgoing optical system 18B' has its direction of polarization rotated through 90° so as to be returned to the confocal ring resonation Fabry-Perot electro-optic modulator 130 via the light incident/outgoing optical system 18B', may be replaced by a Faraday rotator 323 and a mirror of total reflection 324 as in a wavelength scanning laser light source 230' shown in Fig.31.

This wavelength scanning laser light source 230' is remodeled from the wavelength scanning laser light source 230 shown in Fig.30. The wavelength scanning laser light source 230' is similar to the wavelength scanning laser light source 230 except that the second optical fiber loop 332 has been replaced by the Faraday rotator 323 and the mirror of total reflection 324. Hence, the same reference numerals are used to depict the same components and detailed description therefor is dispensed with.

With the wavelength scanning laser light source 230', the light radiated from the confocal ring resonation Fabry-Perot electro-optic modulator 130 via the light incident/outgoing optical system 18B' has its direction of polarization rotated through 45° by the Faraday rotator 323 so as to be incident on the mirror of total reflection 324. The light totally reflected by the mirror of total reflection 324 has its direction of polarization further rotated through 45° by the Faraday rotator 323 so as to be incident via the light incident/outgoing optical system 18B' on the confocal ring resonation Fabry-Perot electro-optic modulator 130.

With this wavelength scanning laser light source 230', the sole confocal ring resonation Fabry-Perot electro-optic modulator 130, in which the direct reflected light Lr of the incident light Lin is not returned to the incident side, is used as a bidirectional electro-optic modulator. The wavelength of a light source having a spectrum of a narrow bandwidth and which is capable of generating monochromatic light of extremely low noise may continuously be scanned over a wide range at a high speed.

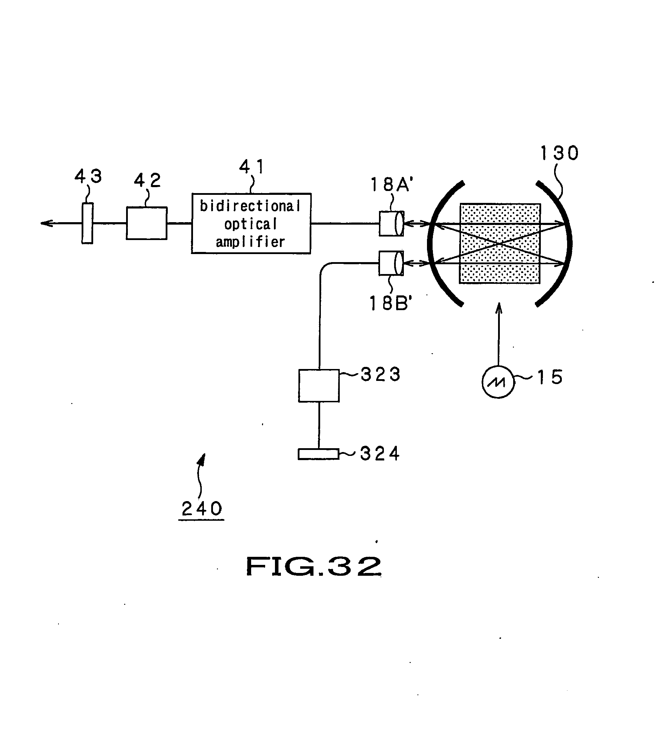

It is observed that an optical amplifier exemplified by SOA is capable of amplifying polarized light regardless of which polarized light component or which direction polarized light component is to be amplified. Thus, by providing a bidirectional optical amplifier 41 such as SOA, as in a wavelength scanning laser light source 240 shown in Fig.32, it is possible to effect light amplification twice during one round trip through the optical fiber loop.

This wavelength scanning laser light source 240 shown in Fig.32 is remodeled from the wavelength scanning laser light source 230' shown in Fig.31. The wavelength scanning laser light source 240 is similar to the wavelength scanning laser light source 230' except that the first optical fiber loop 331 has been replaced by a bidirectional optical amplifier 41, a Faraday rotator 42 and a half mirror 43. Hence, the same reference numerals are used to depict the same components and detailed description therefor is dispensed with.

With the wavelength scanning laser light source 240, the light radiated from the confocal ring resonation Fabry-Perot electro-optic modulator 130 via the light incident/outgoing optical system 18A' is amplified by the bidirectional optical amplifier 41, and has its direction of polarization rotated through 45° by the Faraday rotator 42 so as to be incident on the half mirror 43. The light reflected by the half mirror 43 has its direction of polarization further rotated through 45° by the Faraday rotator 42 so as to be incident on the bidirectional optical amplifier 41. The light amplified by the bidirectional optical amplifier 41 is incident from the light incident/outgoing optical system 18A' on the confocal ring resonation Fabry-Perot electro-optic modulator 130 so as to be thereby set into oscillations. The laser light is taken outside via the half mirror 43.

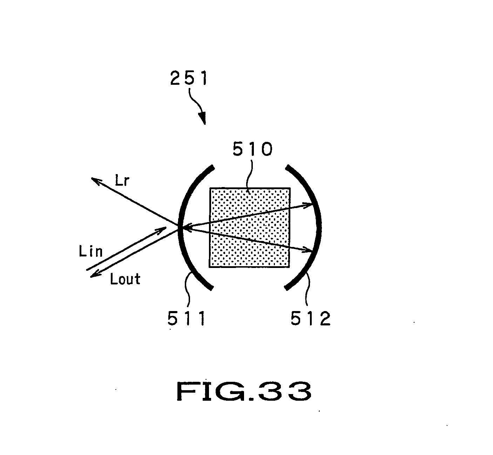

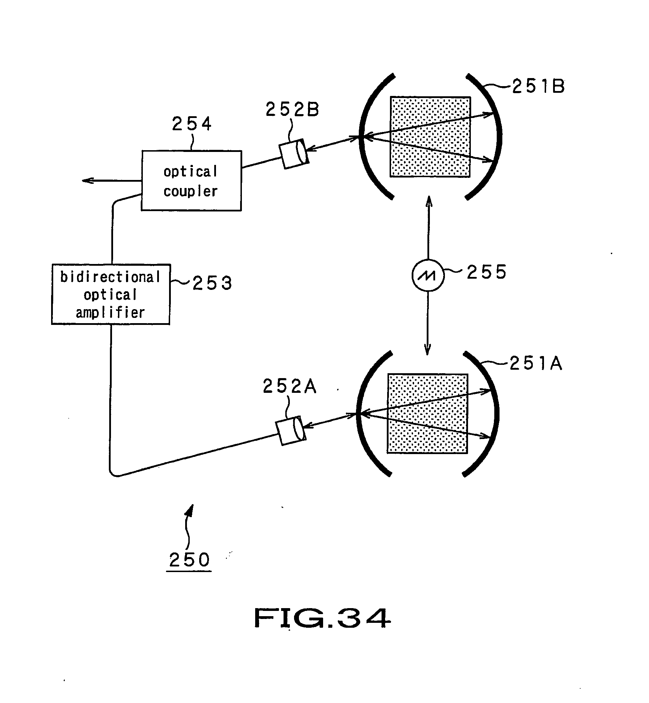

Also, with the confocal resonation Fabry-Perot electro-optic modulator, V-shaped resonation may be achieved by adjusting the direction of light incidence of the incident light. As in a confocal V-shaped resonation Fabry-Perot electro-optic modulator 251 shown in Fig.33, V-shaped resonation in which the direct reflected light Lr of the incident light Lin is not returned to the light incident side and the outgoing light Lout is returned in the direction of light incidence may be achieved by setting the curvatures of concave mirrors 511, 512 so that the Fabry-Perot resonator having an electro-optic crystal 510 will provide a confocal system, and by adjusting the direction of light incidence of the incident light Lin. Thus, by using the confocal V-shaped resonation Fabry-Perot electro-optic modulator 251, capable of providing V-shaped resonation, in which the direct reflected light of the incident light is not returned to the light incident side and the outgoing light will be returned in the direction of light incidence, it is possible to construct a linear resonation wavelength scanning laser light source 250 shown for example in Fig.34.

This wavelength scanning laser light source 250 includes two confocal V-shaped resonation Fabry-Perot electro-optic modulators 251A, 251B, a bidirectional optical amplifier 253, an optical coupler 254 and a resonator length controller 255. The bidirectional optical amplifier 253 is optical-fiber-connected via a light incident/outgoing side optical system 252A to the V-shaped resonation Fabry-Perot electro-optic modulator 251A. The optical coupler 254 is optical-fiber-connected via a light incident/outgoing side optical system 252B to the V-shaped resonation Fabry-Perot electro-optic modulator 251B. The resonator length controller 255 causes the resonator length of one of the two confocal V-shaped resonation Fabry-Perot electro-optic modulators 251A, 251B, here the V-shaped resonation Fabry-Perot electro-optic modulator 251A, to be changed periodically within a preset range. The bidirectional optical amplifier 253 is optical-fiber-connected to the optical coupler 254.

The two confocal V-shaped resonation Fabry-Perot electro-optic modulators 251A, 251B have proximate values of FSR (free spectral range) and operate as wavelength selection filters. At least one of the two confocal V-shaped resonation Fabry-Perot electro-optic modulators 251A, 251B, here the V-shaped resonation Fabry-Perot electro-optic modulator 251A, may have its selection wavelength changed by varying its resonator length.

The two confocal V-shaped resonation Fabry-Perot electro-optic modulators 251A, 251B having proximate values of FSR operate as a band-pass filter having a narrow-band wavelength selection characteristic, capable of varying the selection wavelength by the vernier effect by varying the resonator length of the V-shaped resonation Fabry-Perot electro-optic modulator 251A.

With the wavelength scanning laser light source 250, the light propagated through the band-pass filter of the two V-shaped Fabry-Perot electro-optic modulators 251A, 251B of the proximate Values of FSR is amplified by the bidirectional optical amplifier 253 having the gain bandwidth at the oscillation wavelength, as the light travels back and forth between the two V-shaped Fabry-Perot electro-optic modulators 251A, 251B. With this wavelength scanning laser light source 250, the resonator length of the V-shaped resonation Fabry-Perot electro-optic modulator 251A is periodically changed within a preset range by the resonation length controller 255 to cause periodic changes in the oscillation wavelength. The wavelength of the laser light taken outside via the optical coupler 254 may thus be changed periodically.

With the above-described wavelength scanning laser light source 250, the resonator length may be increased by elongating the optical-fiber-connected light path to narrow down the longitudinal mode spacing of the entire laser. The effect of mode hop may be removed without changing the resonator length. Even though the oscillation is not strictly a single mode oscillation, pseudo-continuous wavelength variation may be achieved solely by changing the selection wavelength of the band-pass filter.

With this wavelength scanning laser light source 250, the wavelength of the light source having a narrow-band spectrum may be continuously scanned over a broad bandwidth at a high speed. Moreover, since the confocal V-shaped resonation Fabry-Perot electro-optic modulators 251A, 251B in which the direct reflected light Lr of the incident light Lin is not returned to the light incident side are used, the optical isolator may be omitted, thus suppressing the noise due to light loss in the optical isolator from increasing.

With the above-described confocal V-shaped resonation Fabry-Perot electro-optic modulator 251, as in the confocal ring resonation Fabry-Perot electro-optic modulator 130, the difference in FSR may be finely set based on the difference in length by arranging two electro-optic crystals between the two concave mirrors so that their C-axes will be at right angles to each other.

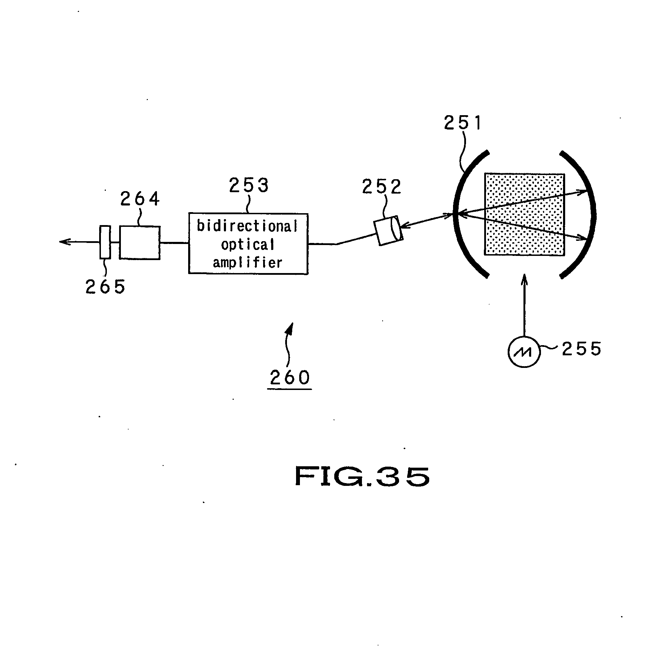

With the above-described confocal V-shaped resonation Fabry-Perot electro-optic modulator 251, the FSR differs depending on the direction of light polarization, and the electro-optic modulation acts strongly with respect to the polarized light along the C-axis. Hence, a linear resonation wavelength scanning laser light source 260 may be formulated by a sole confocal V-shaped resonation Fabry-Perot electro-optic modulator 251 as shown for example in Fig.35.

This wavelength scanning laser light source 260 is remodeled from the wavelength scanning laser light source 250 shown in Fig.34, and includes a sole confocal V-shaped resonation Fabry-Perot electro-optic modulator 251, a bidirectional optical amplifier 253, a Faraday rotator 264 and a half mirror 265. The bidirectional optical amplifier 253 is optical-fiber-connected to this V-shaped resonation Fabry-Perot electro-optic modulator 251 via a light incident/outgoing optical system 252. The Faraday rotator 264 is optical-fiber-connected to the bidirectional optical amplifier 253, and the half mirror 265 is optical-fiber-connected to the Faraday rotator 264.

With this wavelength scanning laser light source 260, the light radiated from the confocal V-shaped resonation Fabry-Perot electro-optic modulator 251 via the light incident/outgoing optical system 252 is amplified by the bidirectional optical amplifier 253 and has its direction of light polarization rotated through 45° by the Faraday rotator 264 to fall on the half mirror 265. The light reflected by the half mirror 265 has its direction of light polarization further rotated through 45° by the Faraday rotator 264 to fall on the bidirectional optical amplifier 253. The light amplified by this bidirectional optical amplifier 253 falls on the confocal V-shaped resonation Fabry-Perot electro-optic modulator 251 via the light incident/outgoing optical system 252.

With the wavelength scanning laser light source 250, the FSR of the confocal V-resonation Fabry-Perot electro-optic modulator 251 differs in dependence upon the direction of light polarization. Moreover, electro-optic modulation acts strongly on the polarized light along the C-axis. The confocal V-resonation Fabry-Perot electro-optic modulator 251 acts as two Fabri-Perot resonators of different modulation degrees of the proximate values of FSRp, FSRs with respect to the light of the P-polarized component and light of the S-polarized component of the incident light, that is, the synthesized light.

The sole confocal V-shaped resonation Fabry-Perot electro-optic modulator 251 operating as two Fabry-Perot resonators of differing modulation degrees of proximate values of FSRp, FSRs operates as a wavelength selection filter. By varying the relative resonator lengths for the P-polarized light and for the S-polarized light, the sole confocal V-shaped resonation Fabry-Perot electro-optic modulator also operates as a band-pass filter having wavelength selection characteristics of narrow bandwidth capable of varying the selection wavelength by the vernier effect.

With the wavelength scanning laser light source 260, the light radiated from the confocal V-shaped resonation Fabry-Perot electro-optic modulator 251 is reflected by the half mirror 265 and returned to the confocal V-shaped resonation Fabry-Perot electro-optic modulator 251 so as to be amplified by the bidirectional optical amplifier 253 to cause laser oscillation. The bidirectional optical amplifier has a gain wavelength at the oscillation wavelength. Moreover, with the wavelength scanning laser light source 260, the relative resonator lengths of the V-shaped resonation Fabry-Perot electro-optic modulator 251 for the P-polarized component and the S-polarized component are periodically changed within a preset range by the resonator length controller 55 to cause periodic changes in the oscillation wavelength and in the wavelength of the laser light taken outside via the half mirror.

With the above-described wavelength scanning laser light source 260, the resonator length may be increased by elongating the optical-fiber-connected light path to narrow down the longitudinal mode spacing of the entire laser. The effect of mode hop may be removed without changing the resonator length. Even though the oscillation is not strictly a single mode oscillation, pseudo-continuous wavelength variation may be achieved solely by changing the selection wavelength of the band-pass filter.

With this wavelength scanning laser light source 260, the sole confocal V-shaped resonation Fabry-Perot electro-optic modulator 251 in which the direct reflected light Lr of the incident light Lin is not returned to the incident side is used to scan the wavelength of a light source having a spectrum of a narrow bandwidth and which is capable of generating monochromatic light of extremely low noise over a wide range continuously at a high speed.

In the above-described embodiments of the present invention, the wavelength scanning laser light source is formulated by using the confocal ring resonation Fabry-Perot electro-optic modulators 130 (130A, 130B) or the use of the confocal V-shaped resonation Fabry-Perot electro-optic modulators 251 (251A, 251B). It is not necessary for the ring resonation Fabry-Perot electro-optic modulators 130 (130A, 130B) or the V-shaped resonation Fabry-Perot electro-optic modulators 251 (251A, 251B) to be confocal as a principle, provided that light can be coupled to the single transverse mode.

That is, spatial Fabry-Perot electro-optic modulators may be used as the ring resonation Fabry-Perot electro-optic modulators 130 (130A, 130B) or as the V-shaped resonation Fabry-Perot electro-optic modulators 251 (251A, 251B). The spatial Fabry-Perot electro-optic modulators yield a merit that the loss is decreased and the finesse increased. On an optical waveguide, loss amounts to ca. 7 dB and the finesse amounts to ca. 50 at the maximum. With the spatial type, the loss may be on the order of 1 dB and the finesse on the order of 400. In this case, the SN ratio may be improved by reducing the loss. Since improving the finesse may increase the resolution as the wavelength filter, it is possible to increase the coherence length of the variable wavelength laser.

By using the 'ring-shaped' or 'V-shaped' spatial Fabry-Perot electro-optic modulator, it is possible to inhibit direct reflection to reduce the number of the optical isolators.

As a principle, the 'ring-shaped' or 'V-shaped' Fabry-Perot electro-optic modulator need not be confocal provided that the light can be coupled to a single transverse mode. However, if the modulator is not confocal, unneeded transverse modes may be presented provided that the light cannot be coupled to the single transverse mode due to only minor adjustment errors. Since these modes differ from the frequencies of the desired mode, the vernier effect is disturbed. With the confocal 'ring-shaped' or 'V-shaped' Fabry-Perot electro-optic modulator, the resonation frequency of the transverse mode is degenerated. Hence, the modulator may be used without unneeded modes being presented even if adjustment is not optimum.

With the wavelength scanning laser light source employing the confocal 'ring-shaped' or 'V-shaped' Fabry-Perot electro-optic modulator, as with the wavelength scanning laser light source 10 shown in Fig.17, the laser light whose wavenumber is linear with respect to time may be obtained by the configuration in which a resonator length controller reads out from a ROM calibration data for obtaining the laser light whose wavenumber is linear with respect to time in order to generate a waveform for the scanning signal.

Thus, with the optical coherence tomography device 100 described above, in which the two Fabry-Perot resonators are run in operation as a narrow bandwidth for wavelength variable filter capable of varying the selection wavelength by the vernier effect and in which a wavelength scanning laser light source for outputting laser light of scanning wavelength temporally is used, it is possible to get a high-speed OCT signal. By adjusting the waveform of the periodic scanning signal to be afforded to the Fabry-Perot resonator, and by adjusting the wavenumber ($2\pi$ / wavelength) of laser light taken outside via the optical device so as to be linear with respect to time, it is possible to re-array the resulting interference signals so that the interference signals will be equally spaced apart with respect to the wavenumber ($2\pi$ / wavelength) of laser light without the necessity of signal processing. It is thus possible to continuously scan the wavelength of the light source having the spectrum of a narrow bandwidth at a high speed over a broad bandwidth without the necessity of performing signal processing for calibration of an OCT signal.

As an embodiment for reducing the number of the optical isolators, such a case has been described above in which a spatial Fabry-Perot electro-optic modulator is composed of a spatial Fabry-Perot resonator that is of the 'ring' or 'V' shape to inhibit direct reflection and that contains an electro-optic crystal. Alternatively, a wavelength scanning laser light source may be formulated by using a Fabry-Perot electro-optic modulator composed of a bulk Fabry-Perot resonator other than the spatial Fabry-Perot resonator such as waveguide Fabry-Perot electro-optic modulator having an electro-optic crystal arranged therein.

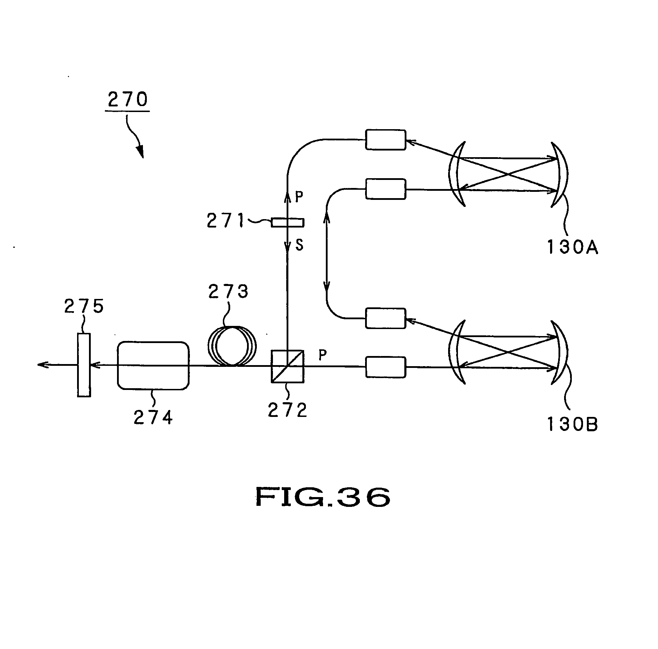

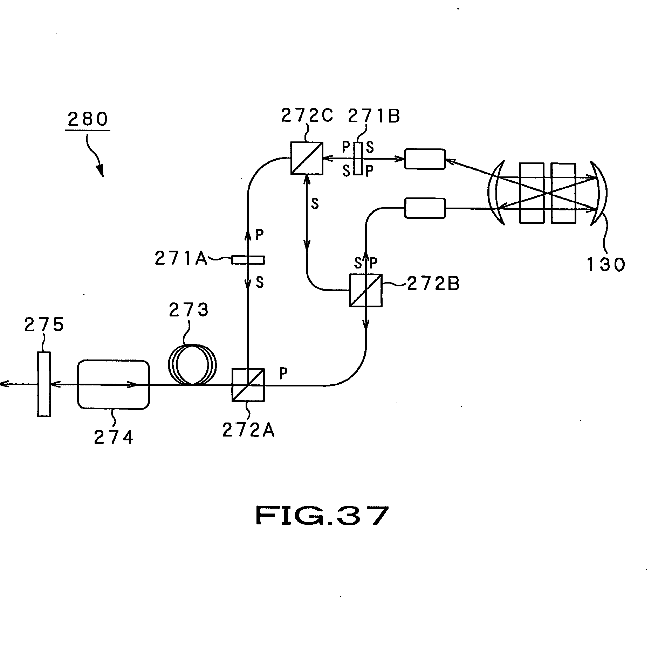

With the ring Fabry-Perot electro-optic modulator, the resonator characteristic is not dependent on the direction of rotation of the laser light within the resonator. Thus, as in wavelength scanning laser light sources 270, 280 shown for example in Figs.36 and 37, two ring Fabry-Perot electro-optic modulators may be made non-dependent on the polarized wave and an optical amplifier non-dependent on the polarized wave and having a gain bandwidth corresponding to the oscillation wavelength may be provided on the light path of laser oscillation.

The polarized wave dependent optical amplifier is expensive and its characteristics such as extinction ratio are not optimum. This inconvenience may, however, be overcome by employing an optical amplifier non-dependent on the polarized wave.

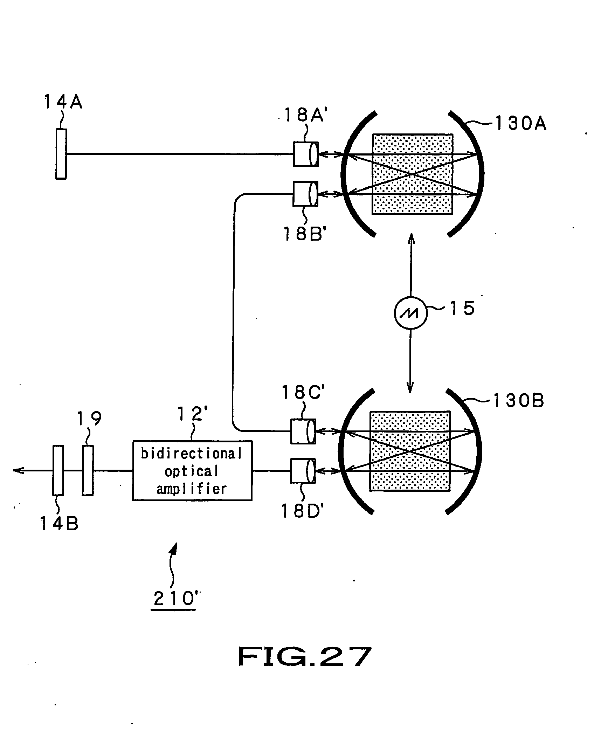

The wavelength scanning laser light source 270 shown in Fig.36, is remodeled from the wavelength scanning laser light source 210' shown in Fig.27, and is made non-dependent on the polarized wave. The wavelength scanning laser light source includes two ring Fabry-Perot electro-optic modulators 130A, 130B having proximate values of FSRp and FSR, and an oscillation light path. On this light path, there are provided a polarized light converter 271, a PBS coupler 272, an SM fiber 273, a bidirectional optical amplifier non-dependent on the polarized wave 274 and a half mirror 275.

The optical amplifier non-dependent on the polarized wave 274 such as SOA is capable of amplifying polarized light without regard to which polarized light component or which direction polarized light component is to be amplified. It amplifies light incident thereon from the PBS coupler 272 via the SM fiber 273 and outputs part of the amplified outgoing light via the half mirror 275 to outside, while amplifying return light reflected back from the half mirror 275 to cause the so amplified light to fall via the SM fiber 273 on the PBS coupler 272.

The PBS coupler 272 transmits the P-polarized component of the incident light and reflects its S-polarized light to split the incident light into its polarized components. It also combines the P-polarized light and the S-polarized light of the incident light.

Here, the PBS coupler 272 separates the light incident thereon via the SM fiber 273 into the P-polarized component and the S-polarized component.

The incident light falls on the PBS coupler 272 via the SM fiber 273. The P-polarized light transmitted through the PBS coupler 272 falls via a light incident/outgoing optical system on the ring resonation Fabry-Perot electro-optic modulator 130B.

The P-polarized component of the incident light, incident on the ring resonation Fabry-Perot electro-optic modulator 130B, is taken outside from the ring resonation Fabry-Perot electro-optic modulator 130B via the light incident/outgoing optical system to fall on the ring resonation Fabry-Perot electro-optic modulator 130A via the light incident/outgoing optical system. The P-polarized light component of the light incident on the ring resonation Fabry-Perot electro-optic modulator 130A is taken outside via the light incident/outgoing optical system from the ring resonation Fabry-Perot electro-optic modulator 130A.

The P-polarized component of the incident light taken outside via the light incident/outgoing optical system from the ring resonation Fabry-Perot electro-optic modulator 130A has its direction of polarization rotated through 90° by the polarized light converter 271 so as to be thereby converted into the S-polarized component which is incident on the PBS coupler 272.

The PBS coupler 272 reflects the light converted into the S-polarized light by rotation of the direction of polarization by the polarized light converter 271 through 90°, and causes it to be incident via the SM fiber 273 on the polarized wave non-dependent bidirectional optical amplifier 274.

Of the incident light falling on the PBS coupler 272 via the SM fiber 273, the S-polarized light reflected by the PBS coupler 272 has its direction of polarization rotated through 90° by the polarized light converter 271 so as to be thereby converted into the P-polarized light. This P-polarized light falls on the ring resonation Fabry-Perot electro-optic modulator 130A via the light incident/outgoing optical system.

The P-polarized light incident via the light incident/outgoing optical system on the ring resonation Fabry-Perot electro-optic modulator 130A, that is, the P-polarized light converted from the S-polarized light of the incident light by the polarized light converter 271, is taken outside from the ring resonation Fabry-Perot electro-optic modulator 130A via the light incident/outgoing optical system. The P-polarized light is then incident via the light incident/outgoing optical system on the ring resonation Fabry-Perot electro-optic modulator 130B. The P-polarized light incident on the ring resonation Fabry-Perot electro-optic modulator 130B is taken out via the light incident/outgoing optical system from the ring resonation Fabry-Perot electro-optic modulator 130A so as to fall on the PBS coupler 272.

The PBS coupler 272 transmits the P-polarized light taken outside via the light incident/outgoing optical system from the ring resonation Fabry-Perot electro-optic modulator 130A. The light so taken out is incident via the SM fiber 273 on the polarized wave non-dependent bidirectional optical amplifier 274.

With the wavelength scanning laser light source 270, the light reflected by the half mirror is amplified by the polarized wave non-dependent optical amplifier 274 and falls via the SM fiber 273 on the PBS coupler 272 where it is separated into the P-polarized component and the S-polarized component. These P-polarized and S-polarized components are returned via the ring resonation Fabry-Perot electro-optic modulators 130A, 130B having proximate values of FSRp and FSRs to the PBS coupler 272 so as to be combined together. The resulting combined light is incident via the SM fiber 273 on the polarized wave non-dependent optical amplifier 274 where it is amplified. The so amplified light is incident on the half mirror 14B. The light reflected by the half mirror 14B falls on the polarized wave non-dependent optical amplifier 274 where it is set into oscillations. The resulting laser light is taken out via the half mirror 14B.

The wavelength scanning laser light source 280 shown in Fig.37 is remodeled from the wavelength scanning laser light source 270 shown in Fig.36. That is, the sole ring resonation Fabry-Perot electro-optic modulator 130 is used as a bidirectional electro-optic modulator whereby the ring resonation Fabry-Perot electro-optic modulator 130 operates as two ring resonation Fabry-Perot electro-optic modulators 130A, 130B having proximate values of FSRp and FSRs.

The wavelength scanning laser light source 280 includes a sole ring resonation Fabry-Perot electro-optic modulator 130 and a light path for oscillation, on which there are provided polarized light converters 271A, 271B, PBS couplers 272A, 272B, 272C, an SM fiber 273, a polarized wave non-dependent optical amplifier 274 and a half mirror 275.

The polarized wave non-dependent optical amplifier 274 such as SOA is capable of amplifying polarized light without regard to which polarized light or which direction polarized light is to be amplified. It amplifies light incident thereon from the PBS coupler 272A via the SM fiber 273 and outputs part of the amplified outgoing light via the half mirror 275 to outside, while amplifying return light reflected back from the half mirror 275 to cause the so amplified light to fall via the SM fiber 273 on the PBS coupler 272A.

The PBS coupler 272A separates the incident light incident thereon via the SM fiber 273 into the P-polarized component and the S-polarized component.

Of the light incident via the SM fiber 273 on the PBS coupler 272A, the P-polarized component transmitted through the PBS coupler 272 is incident on the PBS coupler 272B, and the P-polarized component transmitted through the PBS coupler 272B falls via the light incident/outgoing optical system on the ring resonation Fabry-Perot electro-optic modulator 130.

Of the light incident via the SM fiber 273 on the PBS coupler 272A, the S-polarized component reflected by the PBS coupler 272A has its direction of polarization rotated through 90° by the polarized light converter 271A and is thereby converted into a P-polarized component which is then incident on the PBS coupler 272C. The P-polarized component transmitted through the PBS coupler 272C has its direction of polarization rotated through 90°C and is thereby converted into an S-polarized component by the polarized light converter 271B which is then incident via the light incident/outgoing optical system on the ring resonation Fabry-Perot electro-optic modulator 130.

The P-polarized component of the light incident via the light incident/outgoing optical system on the ring resonation Fabry-Perot electro-optic modulator 130 is taken outside via the light incident/outgoing optical system from the ring resonation Fabry-Perot electro-optic modulator 130. The P-polarized component has its direction of polarization rotated through 90° by the polarized light converter 271B so as to be converted into an S-polarized component which then falls on the PBS coupler 272C. The S-polarized component is reflected by the PBS coupler 272C so as to be incident via the light incident/outgoing optical system on the ring resonation Fabry-Perot electro-optic modulator 130.

The light of the S-polarized component taken outside via the light incident/outgoing optical system from the ring resonation Fabry-Perot electro-optic modulator 130 has its direction of polarization rotated through 90° by the polarized light converter 271B so as to be converted into the P-polarized component, which is then incident on the PBS coupler 272C. The P-polarized component transmitted through the PBS coupler 272C has its direction of polarization rotated through 90° by the polarized light converter 271A so as to be converted into the S-polarized component, which is then incident on the PBS coupler 272A.

The PBS coupler 272 reflects the light which has its direction of polarization rotated through 90° by the polarized light converter 271 and which thereby is converted into the S-polarized component. The reflected light is incident via the SM fiber 273 on the polarized wave non-dependent optical amplifier 274.

The S-polarized component of light incident via the light incident/outgoing optical system on the ring resonation Fabry-Perot electro-optic modulator 130 is taken out via the light incident/outgoing optical system from the ring resonation Fabry-Perot electro-optic modulator 130 so as to fall on the PBS coupler 272B. The light is then reflected by the PBS coupler 272B to fall on the PBS coupler 272C and has its direction of rotation rotated through 90° by the polarized light converter 271B through 90° so as to be converted into a P-polarized component, which then falls on the ring resonation Fabry-Perot electro-optic modulator 130 via the light incident/outgoing optical system.

The light of the P-polarized component taken outside via the light incident/outgoing optical system from the ring resonation Fabry-Perot electro-optic modulator 130, falls on the PBS coupler 272B, and is transmitted through the PBS coupler 272 to fall on the PBS coupler 272A.

The PBS coupler 272A transmits the light of the P-polarized component which is then incident via the SM fiber 273 on the polarized wave non-dependent optical amplifier 274.

With the wavelength scanning laser light source 270, the light reflected by the half mirror is amplified by the polarized wave non-dependent optical amplifier 274. The amplified light is then incident via the SM fiber 273 on the PBS coupler 272A so as to be separated into a P-polarized component and an S-polarized component. The light separated into the P-polarized component and the S-polarized component is returned via the ring resonation Fabry-Perot electro-optic modulator 130 to the PBS coupler 272A so as to be combined together. The resulting light is incident via the SM fiber 273 on the polarized wave non-dependent optical amplifier 274 where it is amplified. The light thus amplified by the polarized wave non-dependent optical amplifier 274 is incident on the half mirror 14B. The light reflected by the half mirror 14B is incident on the polarized wave non-dependent optical amplifier 274 so as to be set into oscillations. The laser light is taken outside via the half mirror 14B.

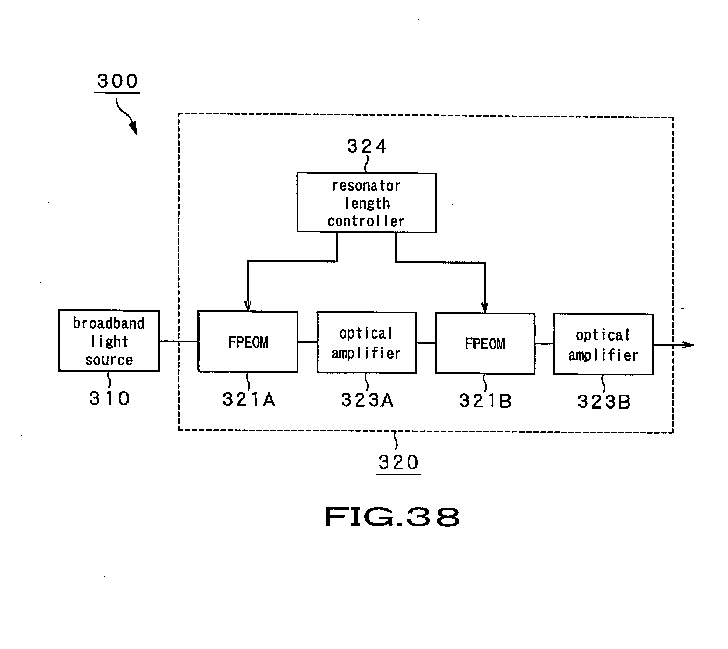

The foregoing description is directed to an optical coherence tomography device in which the two Fabry-Perot resonators are in operation as a narrow-band wavelength variable filter capable of varying the selection wavelength under the vernier effect. The optical coherence tomography device makes use of a wavelength scanning laser light source provided with a filter means for wavelength variation that outputs the light whose wavelength has temporally been scanned. It is observed that the light source for the optical coherence tomography device does not have to be a laser light source and a non-laser wavelength scanning light source 300 shown in Fig.38 may, for example, be used.

The non-laser wavelength scanning light source 300 shown in Fig.38 includes a broadband light source 310 and a filter unit 320 for wavelength variation. The broadband light source radiates light of a broad bandwidth that covers the wavelength scanning range needed by the optical coherence tomography device, and the filter unit 320 takes out light of a desired wavelength range from the broadband light radiated from the broadband light source 310.

The filter unit 320 includes two Fabry-Perot resonators 321A, 321B having values of FSR (free spectral range) proximate to each other, optical amplifiers 323A, 323B that amplify output light of each of the Fabry-Perot resonators 321A, 321B, and a resonator length controller 324. The Fabry-Perot resonators 321A, 321B are provided on an outgoing light path for broadband light radiated from the broadband light source 310. The resonator length controller 324 causes the resonator length of one of the two Fabry-Perot resonators 321A, 321B, having values of FSR proximate to each other, here the Fabry-Perot resonator 321A, to be periodically varied within a preset range.

With the non-laser wavelength scanning light source 300, the two Fabry-Perot resonators 321A, 321B having values of FSR proximate to each other operate as wavelength selection filters. At least one of the two Fabry-Perot resonators 321A, 321B, here the Fabry-Perot resonator 321A, is adapted to vary its resonator length to vary the selection wavelength. The two Fabry-Perot resonators 321A, 321B having values of FSR proximate to each other operate as a band-pass filter having narrow-band wavelength selection characteristic capable of varying the selection wavelength by the vernier effect. This is made possible by varying the resonator length of one of the two Fabry-Perot resonators 321A, 321B having proximate FSR values, here the Fabry-Perot resonator 321A.

With the non-laser wavelength scanning light source 300, the light propagated through the band-pass filter formed by two Fabry-Perot resonators 321A, 321B having values of FSR proximate to each other, is amplified by the optical amplifiers 323A, 323B such as SOAs or fiber amplifiers, and the so amplified light is output.

Wavelengths can be varied under electro-optic effects by using a modulator made up of a Fabry-Perot resonator, termed a Fabry-Perot electro-optic modulator or light COM generator, and a pair electrode. The Fabry-Perot optical resonator is formed of a material whose refractive index can be varied electrically, such as LN ($LiNbO_3$). Wavelength variation is superior in linearity and reproducibility because modulation is electrical modulation.

Thus, the Fabry-Perot electro-optic modulator is used for the Fabry-Perot resonator 321A in which the resonator length in the non-laser wavelength scanning light source 300 is varied. A periodic signal such as a serrated signal is afforded by the resonator length controller 324 to the Fabry-Perot electro-optic modulator for light modulation. In this manner, the two Fabry-Perot resonators 321A, 321B having proximate values of FSR may be in operation as a band-pass filter of narrow band wavelength selection characteristic in which the selection wavelength can be varied under the vernier effect. The center frequency can be scanned at a high speed to high accuracy within a preset range. Thus, by periodically varying the resonator length of the Fabry-Perot resonator 321A by the resonator length controller 324 within a preset range, the wavelength of light that is taken outside may be varied periodically to effect high-speed scanning.

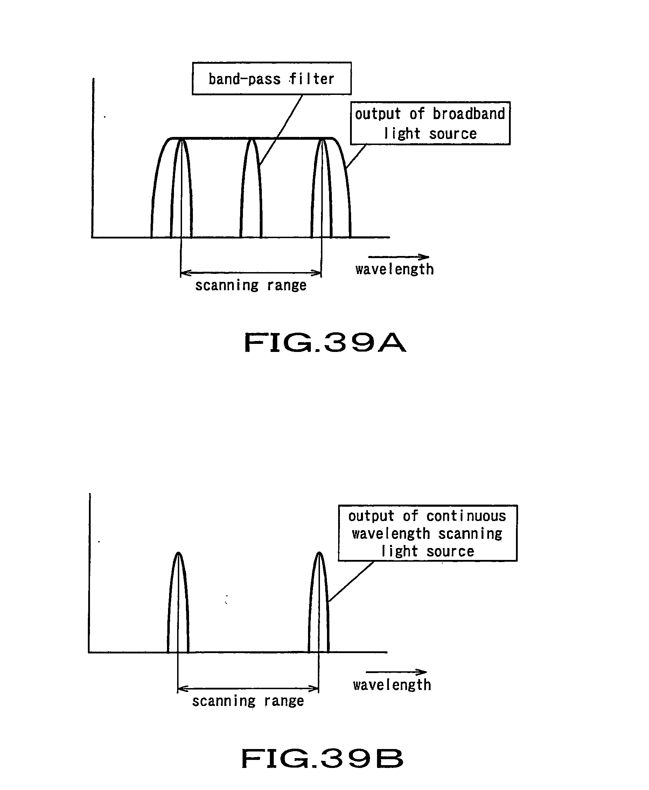

That is, with this non-laser wavelength scanning light source 300, the two Fabry-Perot resonators 321A, 321B having proximate values of FSR are operated as a band-pass filter of a narrow bandwidth wavelength selection characteristic capable of varying the selection wavelength under the vernier effect as shown in Fig.39A. The narrow bandwidth light related with the wavelength selection characteristic of the band-pass filter is extracted from the broadband light radiated from the broadband light source 310, and the center frequency of the band-pass filter is scanned within a preset range. By so doing, the narrow bandwidth light which has scanned the wavelength continuously within a preset scanning range may be obtained as shown in Fig.39B.

If a linear, that is, waveguide, Fabry-Perot electro-optic modulator is used as two Fabry-Perot resonators 321A, 321B, an optical isolator needs to be enclosed within the optical amplifiers 323A, 323B in order to prevent laser oscillations by reflected light from the Fabry-Perot electro-optic modulator.

It is presumed that the ring resonation Fabry-Perot electro-optic modulator 130, composed of a Fabry-Perot resonator with an electro-optic crystal 110 arranged in its inside and two concave mirrors 111, 112, whose curvatures are set so as to yield a confocal resonation system as shown in Fig.19, is used as the above two Fabry-Perot resonators 321A, 321B. With the ring resonation Fabry-Perot electro-optic modulator 130, in which the curvatures of the concave mirrors are set so as to yield a confocal resonation system, direct reflected light Lr to the incident light Lin is not returned to the light incident side. Hence, no optical isolator is needed. It is thus possible to dispense with the optical isolator at input and output parts of the Fabry-Perot resonators 321A, 321B. It is sufficient to provide an optical isolator at the output side of the optical amplifier 323B.

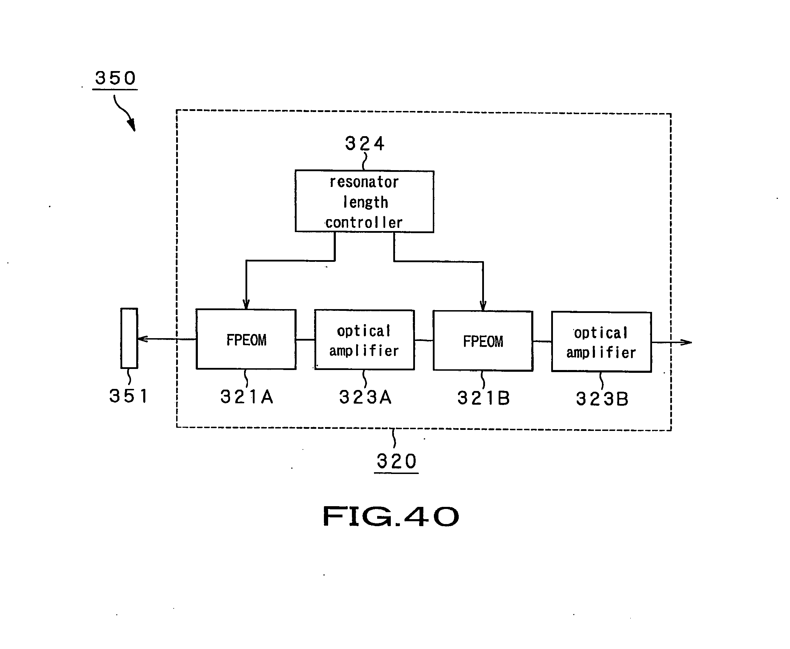

Moreover, if the Fabry-Perot electro-optic modulator is of the ring type, the amplified spontaneous emission (ASE) generated from the optical amplifiers 323A, 323B may be regarded a broadband light source. For example, by providing a reflection mirror 351 that reflects the amplified spontaneous emission (ASE) radiated from the optical amplifiers 323A, 323B in place of the broadband light source 310, as in a wavelength scanning laser light source 350 shown in Fig.40, the optical amplifiers 323A, 323B radiating the amplified spontaneous emission (ASE), may be used as the broadband light source 310.

With the wavelength scanning laser light source 350, the amplified spontaneous emission (ASE) generated by the optical amplifier 323A is propagated in a reverse direction, that is, towards the Fabry-Perot resonator 321B, optical amplifier 323A, Fabry-Perot resonator 321A and a reflecting mirror 351. The light is reflected by the reflection mirror 351 and propagated in a reverse direction via the Fabry-Perot resonator 321A, optical amplifier 323A and the Fabry-Perot resonator 321B so as to be output. The number of the optical isolators or the broadband light sources 310, for example, may be reduced with reduction in cost. Since the light has two passes through a filter, the light may be improved in S/N ratio.

If, with the use of the wavelength scanning laser light source 10 in the optical coherence tomography device, the coherence length needed is $\delta L$ and the speed of light is c, the maximum FSR (FSRmax) of the Fabry-Perot electro-optic modulator is given from the sampling theorem, by the following equation (1):

$$FSRmax = c \, / \, \delta L \tag{1}$$

Since the two Fabry-Perot electro-optic modulators each suffer from the loss of 7 dB, the overall loss is not less than 14 dB. The output power is short of the saturation limit, such that it is sensitive to the optical response characteristic of each Fabry-Perot electro-optic modulator.

Thus, it may be presumed that the cut-off (Srate-cut) of the frequency scanning rate (Srate), which is the frequency scanned by the laser light per unit time, is given, as the function of the maximum scanning range (FSRv), by the following equation (2):

$$Srate\text{-}cut = FSRv \, / \, F\delta t \tag{2}$$

where $\delta t$ (= F / FSR) is the life of a photon in the Fabry-Perot electro-optic modulator.

The cut-off (Srate-cut) of the frequency scanning rate (Srate) obtained as the result of the experiment, was 12 THz/ $\mu$s. The limit ($\delta$Llimit) of the coherence length ($\delta$L) was presumed to be given by the equation (3):

$$\delta L limit = c/ \, Srate \, \delta t$$

$$(3)$$

From the above result, it may be presumed that the coherence length ($\delta L$) and the frequency scanning rate (Srate) are in a relationship of trade-off to each other. The limit ($\delta L limit$-cutoff) of the coherence length ($\delta L$) in the cut-off (Srate-cut) of the frequency scanning rate (Srate) is given by the following equation (4):

$$\delta L limit\text{-}cutoff = cF / FSRv$$

$$(4)$$

Thus, if the coherence length ($\delta L$), frequency scanning rate (Srate) and the maximum scanning range (FSRv) are given by system needs, the condition of the resonator, that is, the condition of the finesse (F) and the free spectral range (FSR), may be determined as follows:

That is, the range that can be assumed by the finesse (F) may generally be found from the above equation (3) by:

$$c/ \, FSR/ \, Srate \, \delta L > F$$

while the range that can be assumed by the free spectral range (FSR) is

$$c/ \, \delta L > FSR.$$

On actual devices, an optimum operation could be confirmed for the range that can be assumed by the finesse (F) of

$$c/\delta L > FSR > SrateF^2 / FSRv/2$$

that is, for the range of the frequency scanning rate (Srate) of from 1/2 to two times the cut-off (Srate-cut).

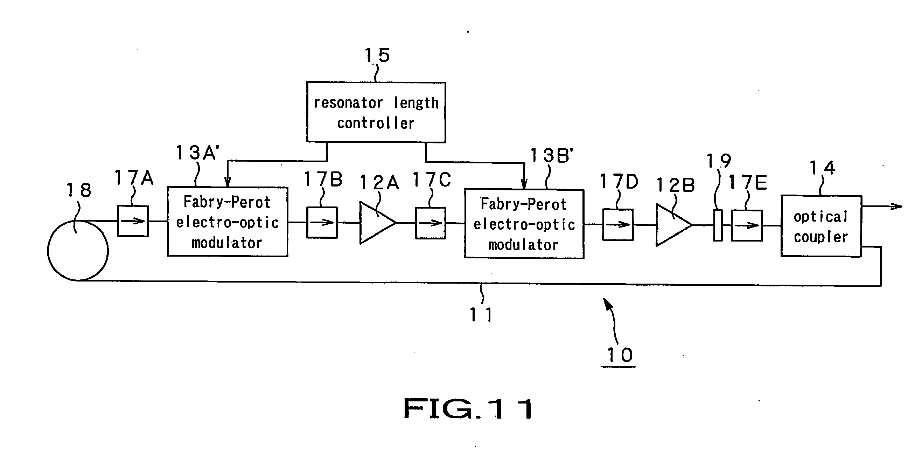

If, with the wavelength scanning laser light source 10, the scanning rate is low, in particular, if the frequency scanning rate (Srate) is lower than the cut-off (Srate-cut)/ 2, intensity noise was produced. This noise is thought to be the mode competition noise. The mode competition noise may be decreased by applying high frequency superposition on the SOA, whereby the characteristic in case of the low scanning rate may be improved. If the frequency scanning rate (Srate) is faster than cut-off (Srate-cut) / 2, the characteristic may be improved by increasing the loop gain by a method shown in Figs.11 to 13.

**Claims**

1.  A wavelength scanning light source comprising:

    a broadband light source; and
    a variable wavelength filter unit that takes out light of a desired wavelength range from broadband light outgoing from said broadband light source;
    said variable wavelength filter unit including two Fabry-Perot electro-optic modulators, each including a spatial Fabry-Perot resonator and a resonator length controller;
    said Fabry-Perot electro-optic modulators being arranged in an outgoing path of light outgoing from said broadband light source and each being provided with an electro-optic crystal arranged therein; said Fabry-Perot electro-optic modulators having values of FSR (free spectral range) proximate to each other; said resonator length controller causing the resonator length of at least one of said two Fabry-Perot resonators having the FSR values proximate to each other to be periodically varied within a preset range; wherein

light is optically modulated in at least one of said two Fabry-Perot electro-optic modulators by a periodic scanning signal afforded by said resonator length controller.

2. The wavelength scanning light source according to claim 1 wherein each of said two Fabry-Perot electro-optic modulators is a ring resonation Fabry-Perot electro-optic modulator in which ring resonation is produced by a Fabry-Perot resonator having said electro-optic crystal arranged therein.

3. The wavelength scanning light source according to claim 2 comprising:

an optical amplifier provided on said outgoing light path of said variable wavelength filter unit, and a reflection mirror that reflects amplified spontaneous emission (ASE) radiated from said optical amplifier to cause the resulting light to be incident on said variable wavelength filter unit; said optical amplifier radiating said amplified spontaneous emission (ASE) being used as said broadband light source.

4. A wavelength scanning light source comprising:

an optical amplifier provided in a light path of laser oscillation and having a gain bandwidth at a wavelength of oscillation;
two Fabry-Perot resonators arranged in said light path of laser oscillation and having values of FSR (free spectral range) proximate to each other;
an optical device that takes part of light propagated through said light path of laser oscillation; and
a resonator length controller that periodically varies the resonator length of one of said two Fabry-Perot resonators having FSR values proximate to each other within a preset range.

5. The wavelength scanning light source according to claim 4, wherein
said Fabry-Perot resonator whose resonator length is periodically changed by said resonator length controller within a preset range is a Fabry-Perot electro-optic modulator that includes a pair electrode, and wherein
light propagated is optically modulated by a periodic scanning signal afforded by said resonator length controller.

6. The wavelength scanning light source according to claim 5, wherein said two Fabry-Perot resonators having FSR values proximate to each other are each formed by a Fabry-Perot electro-optic modulator, and are adjusted to said proximate FSR values by temperature control.

7. The wavelength scanning light source according to claim 6, wherein said resonator length controller affords reciprocally reversed scanning signals to said two Fabry-Perot resonators having FSR values proximate to each other, thereby causing the resonator lengths of said Fabry-Perot resonators in respective opposite directions.

8. The wavelength scanning light source according to claim 7, wherein said resonator length controller superposes a control voltage on a scanning signal to control the center wavelength of light propagated through said two Fabry-Perot resonators having proximate FSR values.

9. The wavelength scanning light source according to claim 4, wherein said optical amplifiers are arranged on the trailing sides of said two Fabry-Perot resonators.

10. The wavelength scanning light source according to claim 4, wherein
said light is separated by polarized light or the direction of light propagated through said light path of laser oscillation, and wherein
light is amplified by said sole optical amplifier on the trailing sides of said two Fabry-Perot resonators.

11. The wavelength scanning light source according to claim 4, wherein said resonator length controller adjusts the waveform of said periodic scanning signal afforded to said Fabry-Perot resonator to calibrate the wavelength so that the wavenumber of laser light taken outside by said optical device will be linear with respect to time.

12. The wavelength scanning light source according to claim 4, wherein
said Fabry-Perot resonator has a finesse (F) with a range shown by c FSR/ Srate $\delta$L > F
where $\delta$L denotes a coherence length needed and Srate denotes a frequency scanning rate; and wherein
said Fabry-Perot resonator has a value of FSR shown by c/ $\delta$L > FSR.

**13.** An optical coherence tomography device comprising:

a wavelength scanning light source including two Fabry-Perot resonators provided in a light path for laser oscillation; said Fabry-Perot resonators having proximate values of FSR (free spectral range);
the resonator length of at least one of said two Fabry-Perot resonators being periodically varied within a preset range to cause said two Fabry-Perot resonators to operate as a wavelength length varying filter of a narrow bandwidth capable of varying the selection wavelength by the vernier effect to output temporally wavelength scanned laser light;
an interference optical system that causes the laser light output from said wavelength scanning light source to be branched into light for reference and light for observation and that generates interference light of reflected light of said light for observation illuminated on an object for observation and said light for reference; and
signal processing means for receiving said interference light obtained from said interference optical system for transforming said received interference light into an electrical signal to calculate the optical tomographic image information of said object for observation.

**14.** The optical coherence tomography device according to claim 13, wherein said wavelength scanning light source is calibrated so that the wavenumber of laser light being scanned will be linear with respect to time.

**15.** The optical coherence tomography device according to claim 13, wherein said wavelength scanning light source includes an optical fiber loop that is to be a light path for laser oscillation, an optical amplifier provided within said optical fiber loop and having a gain bandwidth at a wavelength of oscillation, two Fabry-Perot resonators provided within said optical fiber loop and having values of FSR proximate to each other, an optical device connected to said optical fiber loop to take out part of light propagated through said fiber loop, and a resonator length controller that periodically varies the resonator length of at least one of said two Fabry-Perot resonators having values of FSR proximate to each other within a preset range.

**16.** The optical coherence tomography device according to claim 15, wherein said Fabry-Perot resonator whose resonator length is periodically varied within a preset range by said resonator length controller is a Fabry-Perot electro-optic modulator provided with a pair electrode, and optically modulates the propagated light by a periodic scanning signal afforded by said resonator length controller.

**17.** The optical coherence tomography device according to claim 16, wherein said two Fabry-Perot resonators having proximate values of FSR are each a Fabry-Perot electro-optic modulator and are adjusted by temperature control to said proximate FSR values.

**18.** The optical coherence tomography device according to claim 17, wherein said resonator length controller affords reciprocally reversed scanning signals to said two Fabry-Perot resonators having proximate FSR values to vary the resonator lengths of said Fabry-Perot resonators in respective opposite directions.

**19.** The optical coherence tomography device according to claim 18, wherein said resonator length controller superposes a control voltage on a scanning signal to control the center wavelength of light propagated through said two Fabry-Perot resonators having values of FSR proximate to each other.

**20.** The optical coherence tomography device according to claim 15, wherein said optical amplifiers are arranged respectively on the trailing sides of said two Fabry-Perot resonators.

**21.** The optical coherence tomography device according to claim 15, wherein
said light is separated by polarized light or the direction of light propagated through said optical fiber loop, and wherein light is amplified by said sole optical amplifier on the trailing sides of said two Fabry-Perot resonators.

**22.** The optical coherence tomography device according to claim 15, wherein said resonator length controller adjusts the waveform of said periodic scanning signal afforded to said Fabry-Perot resonator to calibrate the wavelength scanning light source so that the wavenumber of laser light taken outside by said optical device will be linear with respect to time.

**23.** The optical coherence tomography device according to claim 13, wherein said light path for laser oscillation interconnects two Fabry-Perot electro-optic modulators in which spatial Fabry-Perot resonators having proximate values of FSR an electro-optic crystal arranged therein, an optical amplifier having a gain bandwidth at a wavelength of oscillation, and an optical device that takes output light to outside, over an optical fiber.

24. The optical coherence tomography device according to claim 23, wherein said two Fabry-Perot electro-optic modulators are each a ring resonation Fabry-Perot electro-optic modulator designed to produce ring resonation by a Fabry-Perot resonator having an electro-optic crystal arranged therein.

25. The optical coherence tomography device according to claim 23, wherein said two Fabry-Perot resonators are each a V-shaped resonation Fabry-Perot electro-optic modulator designed to produce V-shaped resonation by a Fabry-Perot resonator having an electro-optic crystal arranged therein.

26. The optical coherence tomography device according to claims 24 or 25, wherein said two Fabry-Perot electro-optic modulators are each a confocal Fabry-Perot electro-optic modulator in which curvatures of respective concave mirrors are set so that the Fabry-Perot resonator having an electro-optic crystal arranged therein will prove to be a confocal resonator.

27. The optical coherence tomography device according to claim 23, wherein said Fabry-Perot electro-optic modulator includes two electro-optic crystals arranged with the C-axes at right angles to each other between concave mirrors of said Fabry-Perot resonator.

28. The optical coherence tomography device according to claim 23, wherein
said wavelength scanning light source includes a resonator length controller that periodically varies the resonator length of at least one of said two Fabry-Perot resonators having proximate values of FSR within a preset range, and wherein
said resonator length controller adjusts the waveform of said periodic scanning signal afforded to said Fabry-Perot resonator to calibrate the wavelength scanning light source so that the wavenumber of laser light taken outside by said optical device will be linear with respect to time.

29. The optical coherence tomography device according to claim 13, wherein
said light path for laser oscillation interconnects a Fabry-Perot electro-optic modulator in which a spatial Fabry-Perot resonator having an electro-optic crystal arranged therein, an optical amplifier having a gain bandwidth at a wavelength of oscillation, a polarized light converter that causes 90° rotation of the direction of polarized light outgoing from said Fabry-Perot electro-optic modulator, and an optical device that takes output light to outside, via an optical fiber; and wherein
said Fabry-Perot electro-optic modulator operates as two Fabry-Perot resonators having proximate values of FSR with respect to polarized light components that are perpendicular to each other; said polarized light components perpendicular to each other being modulated by a periodic scanning signal afforded by said resonator length controller.

30. The optical coherence tomography device according to claim 29, wherein said Fabry-Perot electro-optic modulator is a ring resonation Fabry-Perot electro-optic modulator adapted to produce ring-shaped resonation by a Fabry-Perot resonator having said electro-optic crystal arranged therein.

31. The optical coherence tomography device according to claim 29, wherein said Fabry-Perot resonator is a V-shaped resonation Fabry-Perot electro-optic modulator adapted to produce V-shaped resonation by a Fabry-Perot resonator having said electro-optic crystal arranged therein.

32. The optical coherence tomography device according to claims 30 or 31, wherein said Fabry-Perot electro-optic modulators are each a confocal Fabry-Perot electro-optic modulator in which curvatures of respective concave mirrors are set so that the Fabry-Perot resonator having an electro-optic crystal arranged therein will prove to be a confocal resonator.

33. The optical coherence tomography device according to claim 29, wherein said Fabry-Perot electro-optic modulator includes two electro-optic crystals arranged between concave mirrors of said Fabry-Perot resonator with the C-axes at right angles to each other.

34. The optical coherence tomography device according to claim 29, wherein said resonator length controller in said wavelength scanning light source adjusts the waveform of said periodic scanning signal afforded to said Fabry-Perot resonator to calibrate the wavelength so that the wavenumber of laser light taken outside by said optical device will be linear with respect to time.

35. The optical coherence tomography device according to claim 13, wherein said two Fabry-Perot resonators are each a ring resonation Fabry-Perot electro-optic modulator designed to produce ring-shaped resonation by a Fabry-Perot resonator having an electro-optic crystal arranged therein.

36. The optical coherence tomography device according to claim 35, wherein
said two Fabry-Perot resonators are each a polarized wave non-dependent ring resonation Fabry-Perot electro-optic modulator;
said wavelength scanning light source including a polarized wave non-dependent optical amplifier having a gain bandwidth at a wavelength of oscillation in a light path for laser oscillation.

37. The optical coherence tomography device according to claim 13, wherein
said Fabry-Perot resonator has a finesse (F) with a range shown by $cFSR/$ Srate $\delta L > F$
where $\delta L$ denotes a coherence length needed and Srate denotes a frequency scanning rate; and wherein
said Fabry-Perot resonator has a value of FSR shown by $c/\delta L > FSR$.

38. An optical coherence tomography device comprising:

a broadband light source and a wavelength scanning light source; said wavelength scanning light source including two Fabry-Perot resonators provided in a light path for laser oscillation of light outgoing from said broadband light source; said Fabry-Perot resonators having proximate values of FSR (free spectral range);
the resonator length of at least one of said two Fabry-Perot resonators being periodically varied within a preset range to cause said two Fabry-Perot resonators to operate as a wavelength length varying filter capable of varying the selection wavelength by the vernier effect to output laser light that has wavelength temporally scanned;
an interference optical system that causes the laser light output from said wavelength scanning light source to be branched into light for reference and light for observation and that generates interference light of reflected light of said light for observation illuminated on an object for observation and said light for reference; and
signal processing means for receiving said interference light obtained from said interference optical system for transforming said received interference light into an electrical signal to calculate the optical tomographic image information of said object for observation.

39. The optical coherence tomography device according to claim 38, wherein each of said two Fabry-Perot electro-optic modulators is a ring resonation Fabry-Perot electro-optic modulator in which ring resonation is produced by a Fabry-Perot resonator having an electro-optic crystal arranged therein.

40. The optical coherence tomography device according to claim 39, wherein said wavelength scanning light source comprising an optical amplifier provided on said outgoing light path of said variable wavelength filter unit, and a reflection mirror that reflects amplified spontaneous emission (ASE) radiated from said optical amplifier to cause the resulting light to be incident on said variable wavelength filter unit; said optical amplifier radiating said amplified spontaneous emission (ASE) being used as said broadband light source.

2000

2015 optical coupler

2012

2014 band-pass filter

2011

**FIG.1**

FIG.2

FIG.3

FIG.4

FIG.5A

FIG.5B

FIG.6A

FIG.6B

FIG.6C

FIG.6D

FIG.6E

FIG.6F

FIG.6G

FIG.7

EP 2 136 443 A1

FIG.8

10% →

90%

optical
coupler

11

12 19

14

temperature
control

temperature
control

17A

17B

17C

Waveguide
Fabry-Perot
electro-optic
modulator

Waveguide
Fabry-Perot
electro-optic
modulator

13A'

13B'

15

10

# FIG.9

FIG.10

EP 2 136 443 A1

EP 2 136 443 A1

15
resonator length
controller

13A'
17A
18
17B 12A 17C
13B'
17D 12B 19 17E
14

| Fabry-Perot electro-optic modulator | | Fabry-Perot electro-optic modulator | | optical coupler |

11

10

# FIG.11

FIG.12

EP 2 136 443 A1

FIG.13

FIG.14

FIG.15A

FIG.15B

FIG.16A

FIG.16B

FIG.16C

FIG.17

FIG.18

**FIG.19**

**FIG.20**

FIG.21

**FIG.22**

**FIG.23**

**FIG.24A**

**FIG.24B**

**FIG.24C**

**FIG.24D**

FIG.24E

1001
120B
110B
112
110A
120A
111

FIG.24F

1001
110B 120B
125B
112
120A
110A
111
125A

18A

130

18D

C-axis
proximal direction

C-axis

111
120A

110A

110B 120B

112

18B

# FIG.25

EP 2 136 443 A1

FIG.26

14A

18A'          130A

18B'

19   12'        18C'          130B

bidirectional
optical
amplifier

14B            18D'

210'

FIG.27

FIG.28

FIG.29

**FIG.30**

FIG.31

43 42 41 18A' 130

bidirectional
optical
amplifier

18B'

323

324

15

240

# FIG.32

251

510

Lr

Lin

Lout

511        512

# FIG.33

254 optical coupler

252B

251B

bidirectional optical amplifier ~253

255

252A

251A

250

FIG.34

EP 2 136 443 A1

264
253
252
251

265 | bidirectional optical amplifier | 260 | 255

**FIG.35**

270

271

P
S

275    273
274    272    P

130A

130B

**FIG.36**

FIG.37

FIG.38

FIG.39A

FIG.39B

FIG.40

| INTERNATIONAL SEARCH REPORT | International application No. |
|---|---|
| | PCT/JP2007/067156 |

A.  CLASSIFICATION OF SUBJECT MATTER
*H01S3/106*(2006.01)i, *A61B10/00*(2006.01)i, *G01N21/17*(2006.01)i, *G02F2/02*
(2006.01)i, *H01S3/06*(2006.01)i

According to International Patent Classification (IPC) or to both national classification and IPC

B.  FIELDS SEARCHED

Minimum documentation searched (classification system followed by classification symbols)
H01S3/106, A61B10/00, G01N21/17, G02F2/02, H01S3/06

Documentation searched other than minimum documentation to the extent that such documents are included in the fields searched
| | | | |
|---|---|---|---|
| Jitsuyo Shinan Koho | 1922-1996 | Jitsuyo Shinan Toroku Koho | 1996-2007 |
| Kokai Jitsuyo Shinan Koho | 1971-2007 | Toroku Jitsuyo Shinan Koho | 1994-2007 |

Electronic data base consulted during the international search (name of data base and, where practicable, search terms used)

C.  DOCUMENTS CONSIDERED TO BE RELEVANT

| Category* | Citation of document, with indication, where appropriate, of the relevant passages | Relevant to claim No. |
|---|---|---|
| A | JP 2005-99503 A   (Nippon Telegraph And Telephone Corp., Kabushiki Kaisha Hikari Komu Kenkyusho), 14 April, 2005 (14.04.05), Full text; all drawings (Family: none) | 1-40 |
| A | JP 10-173275 A   (Central Research Institute of Electric Power Industry), 26 June, 1998 (26.06.98), Full text; all drawings (Family: none) | 1-40 |

| ☒ Further documents are listed in the continuation of Box C. | ☐ See patent family annex. |
|---|---|

| * | Special categories of cited documents: | "T" | later document published after the international filing date or priority date and not in conflict with the application but cited to understand the principle or theory underlying the invention |
|---|---|---|---|
| "A" | document defining the general state of the art which is not considered   to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
|---|---|
| 10 October, 2007 (10.10.07) | 23 October, 2007 (23.10.07) |

| Name and mailing address of the ISA/ | Authorized officer |
|---|---|
| Japanese Patent Office | |
| Facsimile No. | Telephone No. |

Form PCT/ISA/210 (second sheet) (April 2007)

**INTERNATIONAL SEARCH REPORT**

International application No.

PCT/JP2007/067156

C (Continuation). DOCUMENTS CONSIDERED TO BE RELEVANT

| Category* | Citation of document, with indication, where appropriate, of the relevant passages | Relevant to claim No. |
|---|---|---|
| A | JP 2002-538494 A (Marconi Caswell Ltd.), 12 November, 2002 (12.11.02), Full text; all drawings & US 6529646 B1 & GB 2347230 A & WO 2000/050952 A1 | 1-40 |
| A | JP 2005-55652 A (Hamamatsu Photonics Kabushiki Kaisha), 03 March, 2005 (03.03.05), Full text; all drawings & WO 2005/012983 A1 | 13-40 |

Form PCT/ISA/210 (continuation of second sheet) (April 2007)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2007018168 A
- JP 2007173347 A
- JP 2007173448 A
- JP 2006237359 A
- JP 2006278770 A
- JP 2007101365 A

**Non-patent literature cited in the description**

- **YAMASHITA et al.** *IEEE JOURNAL ON SELECTED TOPICS IN QUANTUM ELECTRONICS,* January 2001, vol. 7 (1), 41-43